# EUROPÄISCHE PATENTSCHRIFT

(11) **EP 3 565 816 B1**
(45) Veröffentlichungstag und Bekanntmachung des Hinweises auf die Patenterteilung: **16.03.2022**
(21) Anmeldenummer: 18701087.1
(22) Anmeldetag: 02.01.2018
(51) Int. Cl.: C07D 471/14, C07D 487/04, C07D 519/00, H01L 51/00, C09K 11/06

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 04.01.2017 EP 17150261
(43) Veröffentlichungstag der Anmeldung: 13.11.2019
(73) Patentinhaber: Merck Patent GmbH, 64293 Darmstadt (DE)
(72) Erfinder: PARHAM, Amir, 60486 Frankfurt Am Main (DE); KROEBER, Jonas, 60311 Frankfurt Am Main (DE); JOOSTEN, Dominik, 60487 Frankfurt Am Main (DE); LUDEMANN, Aurélie, 60322 Frankfurt Am Main (DE); GROSSMANN, Tobias, 64297 Darmstadt (DE)
(86) Internationale Anmeldenummer: PCT/EP2018/050004
(87) Internationale Veröffentlichungsnummer: WO 2018/127465

(56) Entgegenhaltungen:
- WO-A1-2015/171627
- DE-A1-102009 048 791
- DE-A1-102010 048 608
- M. Krzeszewski Et At: "Nonplanar Butterfly-Shaped [pi]-Expanded Pyrrolopyrroles", , 18 October 2016 (2016-10-18), XP055711241, Retrieved from the Internet: URL:https://chemistry-europe.onlinelibrary .wiley.com/doi/epdf/10.1002/chem.201603282 [retrieved on 2020-07-02]
- Maciej Krzeszewski ET AL: "Nonplanar Butterfly-Shaped [pi]-Expanded Pyrrolopyrroles", Chemistry - A European Journal, vol. 22, no. 46, 23 September 2016 (2016-09-23), pages 16478-16488, XP055711252, ISSN: 0947-6539, DOI: 10.1002/chem.201603282

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig metallorganische Komplexe eingesetzt, die Phosphoreszenz statt Fluoreszenz zeigen. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Elektronentransportmaterialien bzw. Lochblockiermaterialien. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine gute Lebensdauer, eine hohe Effizienz und eine geringe Betriebsspannung auf. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung. Aus Krzeszewski et al. (Chem. Eur. J. 2016, 22, 16478-16488) sind hochkondensierte heteroaromatische Verbindungen und deren mögliche Anwendung in organischen optoelektronischen Vorrichtungen bekannt. Verbindungen mit einem weniger ausgedehnten π-Elektronensystem werden nicht offenbart.

Gegenstand der vorliegenden Erfindung ist eine Verbindung gemäß Formel (1), 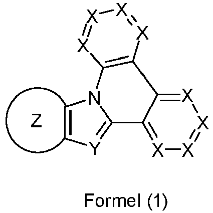 wobei für die verwendeten Symbole gilt:
- Z: ist eine Gruppe der folgenden Formel (2), 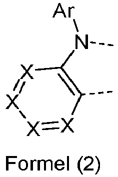 wobei die gestrichelten Bindungen die Verknüpfung dieser Gruppe mit den beiden explizit einzeichneten Kohlenstoffatomen in Formel (1) kennzeichnen;
- X: ist bei jedem Auftreten gleich oder verschieden CR oder N;
- Y: ist CR oder N;
- Ar: ist ein aromatisches oder heteroaromatisches Ringsystem mit 5 bis 40 aromatischen Ringatomen, welches mit einem oder mehreren Resten R substituiert sein kann;
- R: ist bei jedem Auftreten gleich oder verschieden H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann;
- Ar': ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
- R²: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome, bevorzugt 6 bis 40 C-Atome im Ringsystem. Ein heteroaromatisches Ringsystem im Sinne dieser Erfindung enthält 2 bis 60 C-Atome, bevorzugt 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylgruppen sowie Gruppen, in denen zwei oder mehr Aryl- bzw. Heteroarylgruppen direkt miteinander verknüpft sind, sowie Fluoren oder Spirobifluoren.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Wenn zwei Reste R bzw. R¹ miteinander ein Ringsystem bilden, so kann dieses mono- oder polycyclisch, aliphatisch, heteroaliphatisch, aromatisch oder heteroaromatisch sein. Dabei sind die Reste, die miteinander ein Ringsystem bilden, bevorzugt benachbart, d. h. dass diese Reste an dasselbe Kohlenstoffatom oder an Kohlenstoffatome, die direkt aneinander gebunden sind, gebunden sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

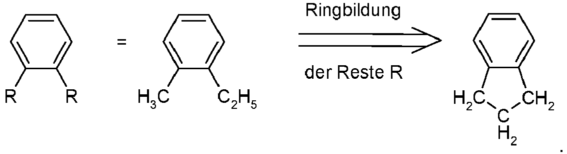

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

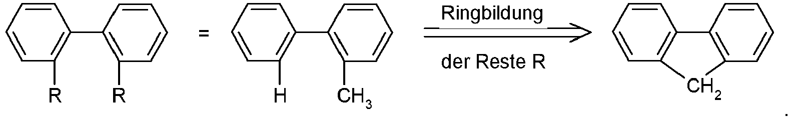

Die Bildung eines aromatischen Ringsystems soll durch das folgende Schema verdeutlicht werden:

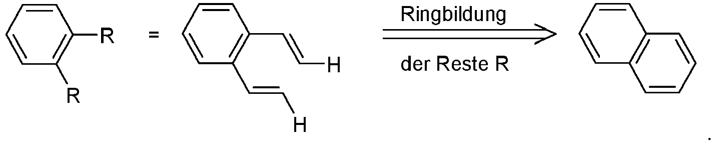

Je nach Ausrichtung der Gruppe Z ergeben sich zwei unterschiedliche Isomere. Diese sind nachfolgend durch die Formeln (3) und (4) dargestellt, 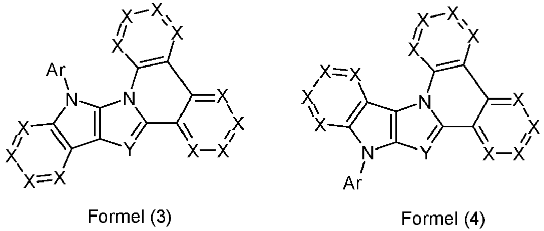 wobei die verwendeten Symbole die oben genannten Bedeutungen aufweisen.

In einer bevorzugten Ausführungsform der Erfindung stehen alle Symbole X in Formel (2) für CR, so dass es sich bei der Gruppe Z in Formel (1) um eine Gruppe der folgenden Formel (2a) handelt, 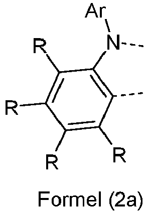 wobei die verwendeten Symbole die oben genannten Bedeutungen aufweisen.

In einer weiteren bevorzugten Ausführungsform der Erfindung steht in den Formeln (1), (3) und (4) maximal ein Symbol X pro Cyclus für N und die anderen Symbole X stehen für CR. In einer besonders bevorzugten Ausführungsform der Erfindung steht maximal ein Symbol X in den Formeln (1), (3) und (4) für N und die anderen Symbole X stehen für CR. Dabei ist es besonders bevorzugt, wenn Z für eine Gruppe der oben aufgeführten Formel (2a) steht.

Eine bevorzugte Ausführungsform der Formeln (3) und (4) sind somit die Verbindungen der folgenden Formeln (3a) und (4a), 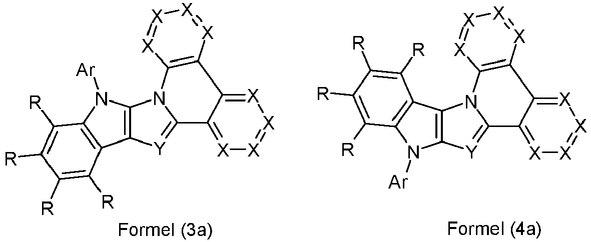 wobei die verwendeten Symbole die oben genannten Bedeutungen aufweisen und maximal ein X für N steht.

In einer besonders bevorzugten Ausführungsform der Erfindung stehen alle X für CR, so dass die Verbindungen der Formel (1) durch die Formeln (3b) und (4b) dargestellt werden können, 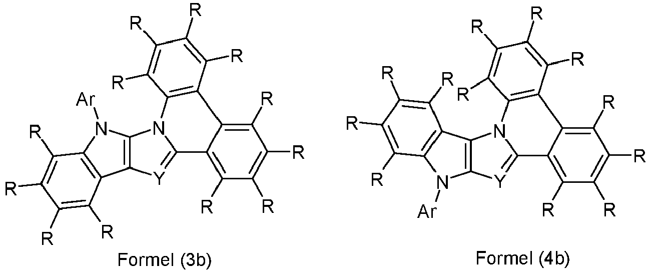 wobei die verwendeten Symbole die oben genannten Bedeutungen aufweisen.

In einer Ausführungsform der Erfindung steht Y in den Formeln (3a) und (4a) für CR, so dass die erfindungsgemäßen Verbindungen die Strukturen der folgenden Formeln (3a-1) und (4a-1) aufweisen, 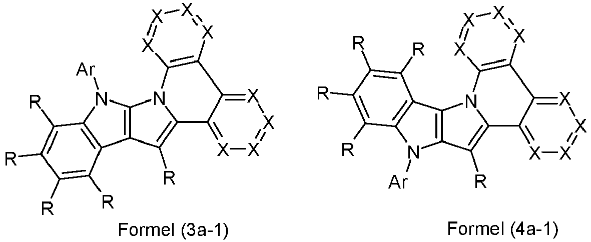 wobei die verwendeten Symbole die oben genannten Bedeutungen aufweisen.

In einer weiteren Ausführungsform der Erfindung steht Y in den Formeln (3a) und (4a) für N, so dass die erfindungsgemäßen Verbindungen die Strukturen der folgenden Formeln (3a-2) und (4a-2) aufweisen, 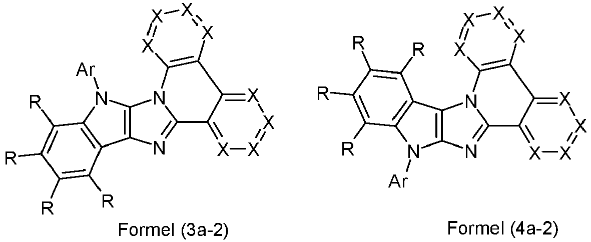 wobei die verwendeten Symbole die oben genannten Bedeutungen aufweisen.

In einer bevorzugten Ausführungsform der Erfindung steht Y für CR, und alle X stehen für CR, so dass die erfindungsgemäßen Verbindungen die Strukturen der Formeln (3b-1) und (4b-1) aufweisen, 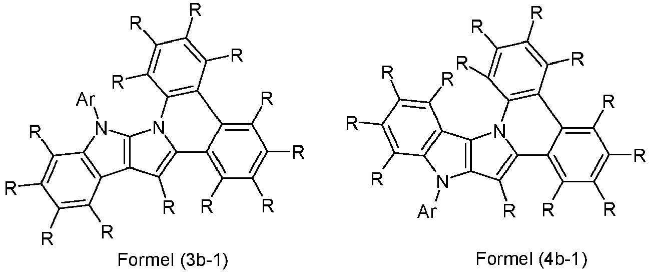 wobei die verwendeten Symbole die oben genannten Bedeutungen aufweisen.

In einer weiteren bevorzugten Ausführungsform der Erfindung steht Y für N, und alle X stehen für CR, so dass die erfindungsgemäßen Verbindungen die Strukturen der Formeln (3b-2) und (4b-2) aufweisen, 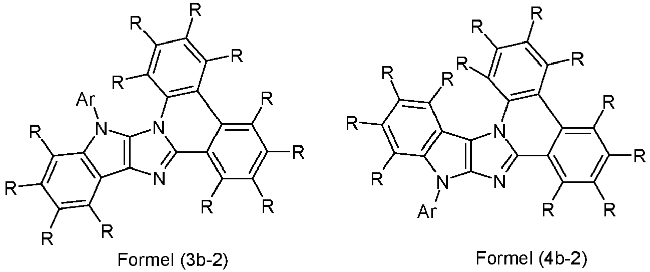 wobei die verwendeten Symbole die oben genannten Bedeutungen aufweisen.

In einer bevorzugten Ausführungsform der Erfindung stehen insgesamt maximal zwei Reste R, besonders bevorzugt maximal ein Rest R in der Verbindung der Formel (1) bzw. den oben aufgeführten bevorzugten Strukturen für eine Gruppe ungleich Wasserstoff. In einer weiteren bevorzugten Ausführungsform der Erfindung steht Y für CR, und der Rest an diesem Kohlenstoffatom, für das Y steht, sowie maximal zwei weitere Reste R, besonders bevorzugt maximal ein weiterer Rest R in der Verbindung der Formel (1) bzw. den oben aufgeführten bevorzugten Strukturen sind ungleich Wasserstoff.

In einer besonders bevorzugten Ausführungsform der Erfindung ist die Verbindung der Formel (1) ausgewählt aus den Verbindungen der folgenden Formeln (3c-1), (4c-1), (3c-2) und (4c-2), 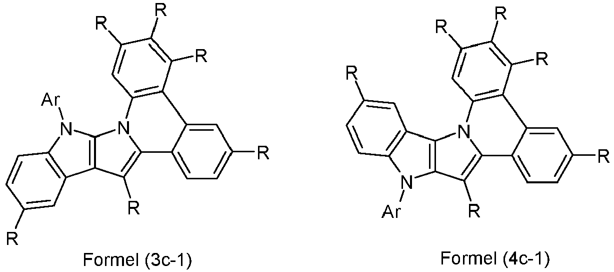 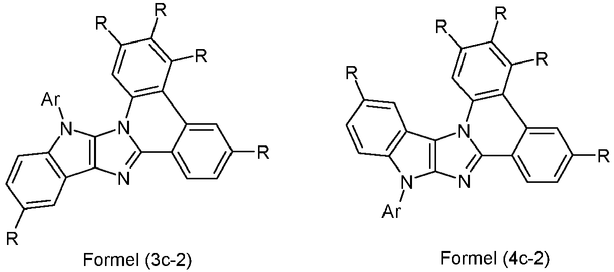 wobei die verwendeten Symbole die oben genannten Bedeutungen aufweisen und bevorzugt maximal zwei Gruppen R, besonders bevorzugt maximal eine Gruppe R, ungleich Wasserstoff sind.

Ganz besonders bevorzugt sind Verbindungen, in denen Y = N ist, also die Verbindungen der Formeln (3a-2), (4a-2), (3b-2), (4b-2), (3c-2) und (4c-2).

Im Folgenden werden bevorzugte Substituenten Ar, R, Ar', R¹ und R² an den erfindungsgemäßen Verbindungen beschrieben. In einer besonders bevorzugten Ausführungsform der Erfindung treten die nachfolgend genannten Bevorzugungen für Ar, R, Ar', R¹ und R² gleichzeitig auf und gelten für alle Strukturen der Formeln (1), (3), (3a), (3b), (3a-1), (3a-2), (3b-1), (3b-2), (3c-1), (3c-2), (4), (4a), (4b), (4a-1), (4a-2), (4b-1), (4b-2), (4c-1) und (4c-2).

In einer bevorzugten Ausführungsform der Erfindung ist Ar ein aromatisches oder heteroaromatisches Ringsystem mit 6 bis 30 aromatischen Ringatomen, das durch einen oder mehrere Reste R substituiert sein kann. Besonders bevorzugt ist Ar ein aromatisches oder heteroaromatisches Ringsystem mit 6 bis 24 aromatischen Ringatomen, insbesondere mit 6 bis 12 aromatischen Ringatomen, das durch einen oder mehrere, bevorzugt nicht-aromatische, Reste R substituiert sein kann. Wenn Ar für eine Heteroarylgruppe, insbesondere für Triazin, Pyrimidin oder Chinazolin steht, können auch aromatische oder heteroaromatische Substituenten R an dieser Heteroarylgruppe bevorzugt sein. Weiterhin kann es bevorzugt sein, wenn Ar mit einer Gruppe N(Ar')₂ substituiert ist, so dass der Substituent Ar am Stickstoffatom in Formel (1) bzw. den bevorzugten Ausführungsformen insgesamt eine Triarylamin- bzw. Triheteroarylamin-Gruppe darstellt.

Geeignete aromatische bzw. heteroaromatische Ringsystem Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3-oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R, bevorzugt nichtaromatischen Resten R, substituiert sein können. Wenn Ar für eine Heteroarylgruppe, insbesondere für Triazin, Pyrimidin oder Chinazolin steht, können auch aromatische oder heteroaromatische Reste R an dieser Heteroarylgruppe bevorzugt sein.

Dabei ist Ar bevorzugt gewählt aus den Gruppen der folgenden Formeln Ar-1 bis Ar-76, 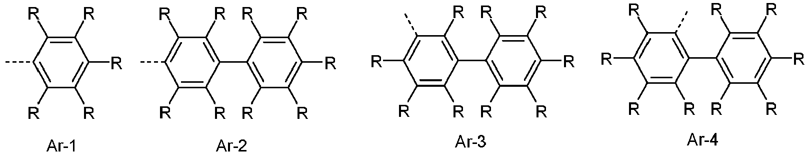 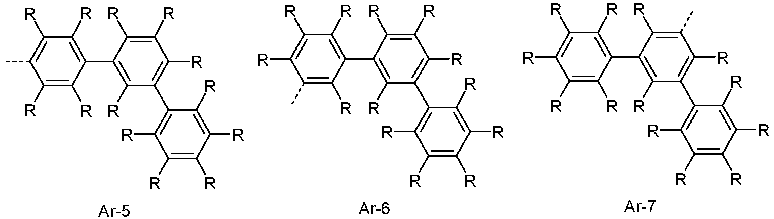 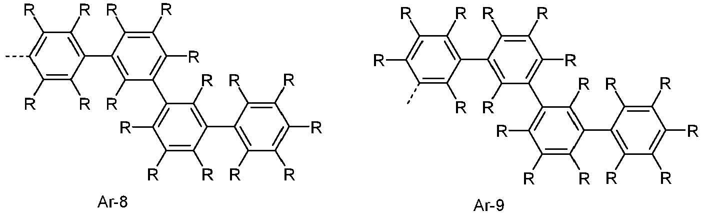 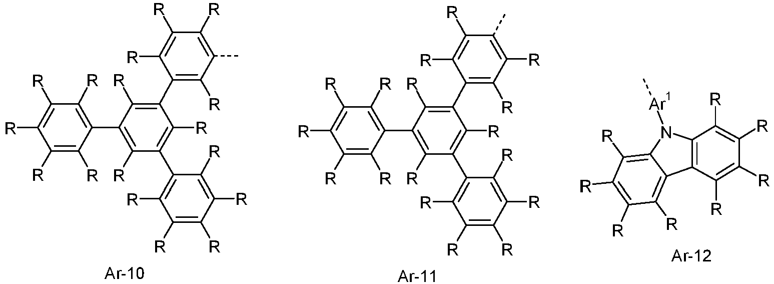 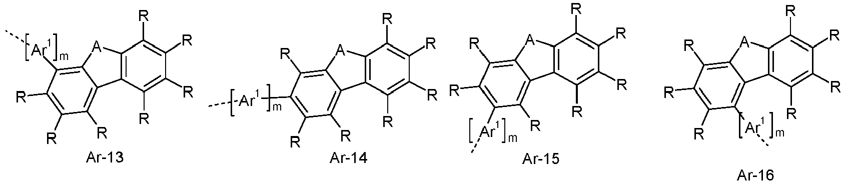 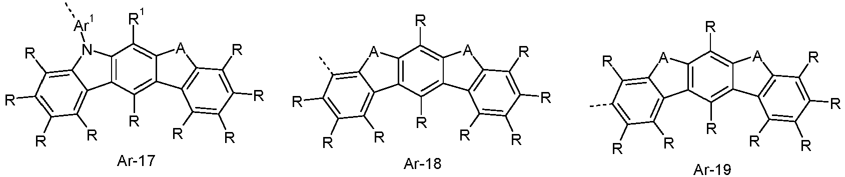 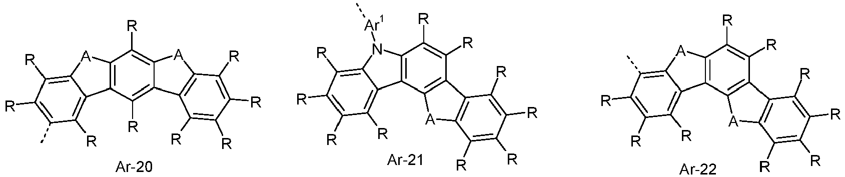 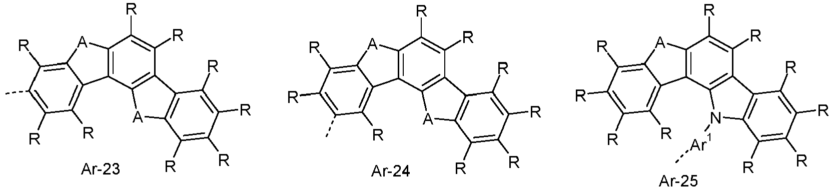 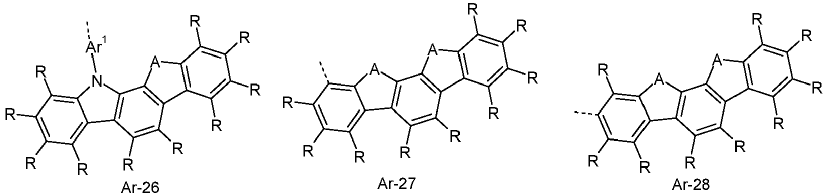 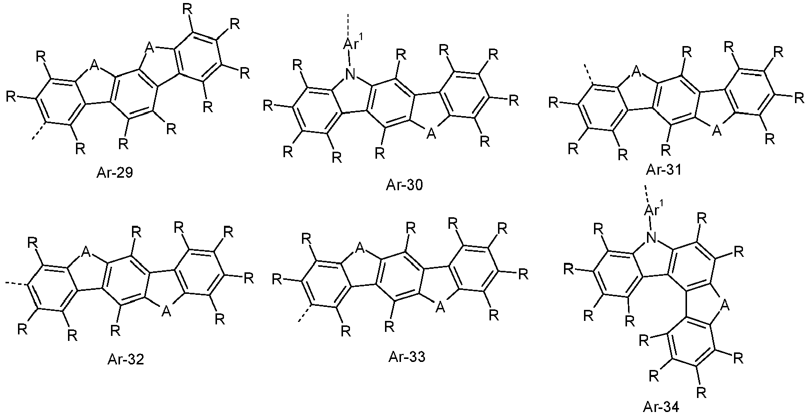 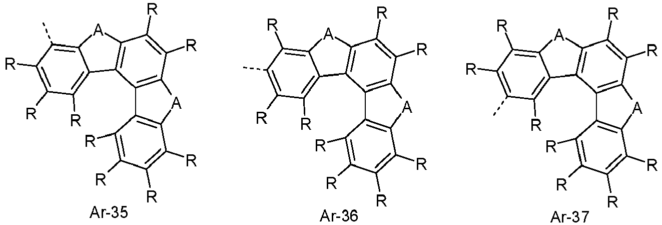 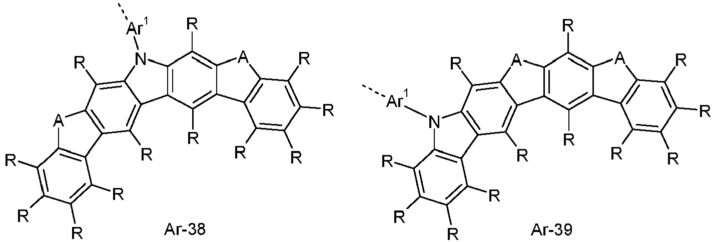 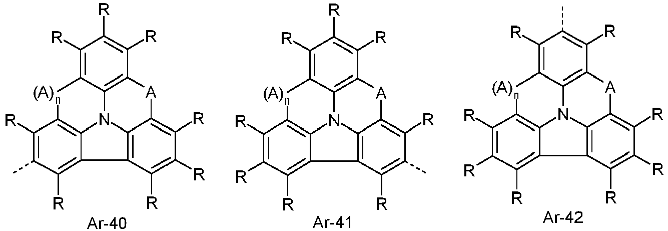 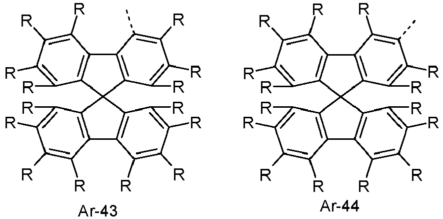 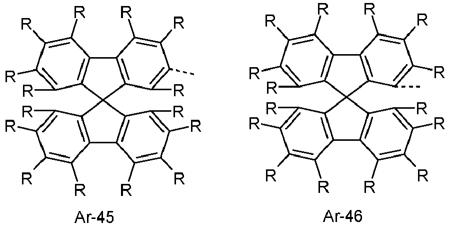 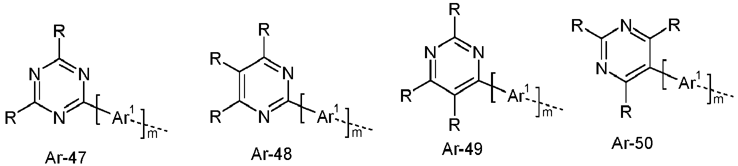 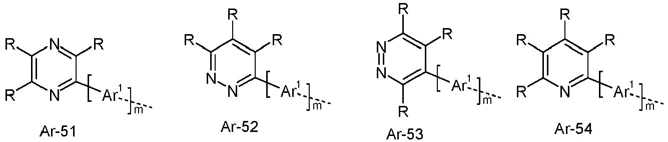 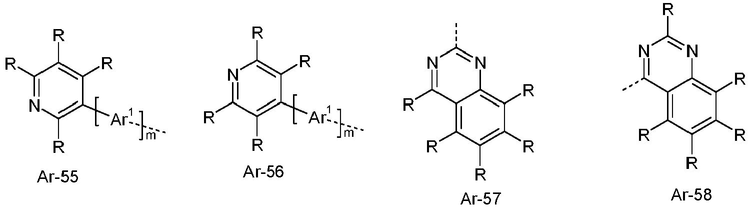 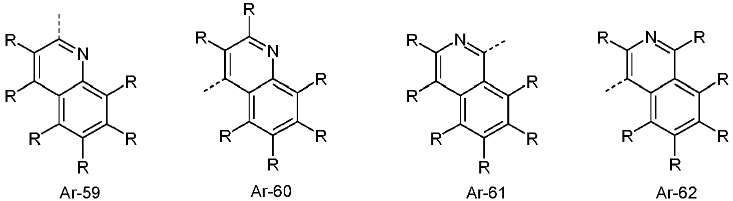 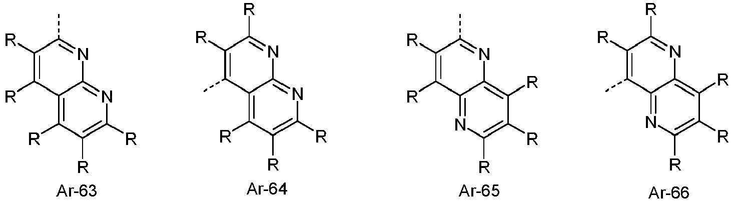 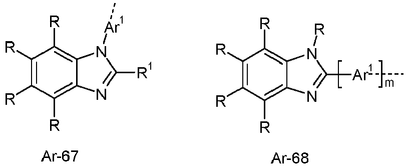 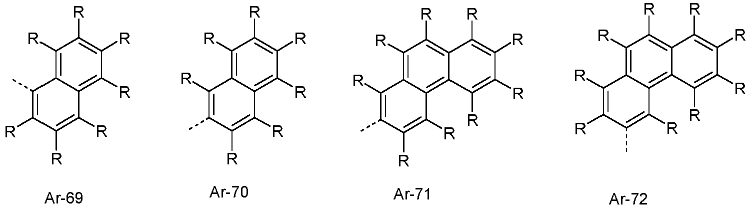 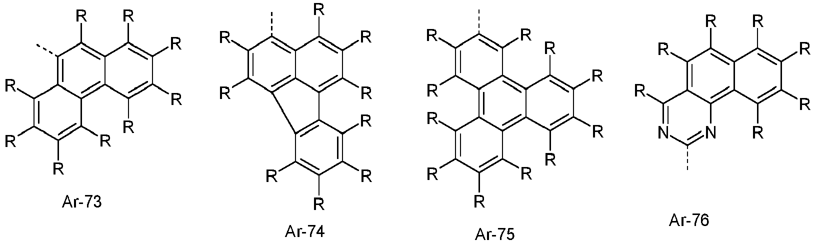 wobei R die oben genannten Bedeutungen aufweist, die gestrichelte Bindung die Bindung an das Stickstoffatom darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- A: ist bei jedem Auftreten gleich oder verschieden C(R)₂, NR, O oder S;
- n: ist 0 oder 1, wobei n = 0 bedeutet, dass an dieser Position keine Gruppe A gebunden ist und an die entsprechenden Kohlenstoffatome statt dessen Reste R gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an das Stickstoffatom gebunden ist.

In einer bevorzugten Ausführungsform der Erfindung ist R bei jedem Auftreten gleich oder verschieden ausgewählt aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann. Besonders bevorzugt ist R bei jedem Auftreten gleich oder verschieden ausgewählt aus der Gruppe bestehend aus H, N(Ar')₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt ist R bei jedem Auftreten gleich oder verschieden ausgewählt aus der Gruppe bestehend aus H oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Weiterhin kann es bevorzugt sein, wenn R für eine Triaryl- bzw. -heteroarylamingruppe steht, welche durch einen oder mehrere Reste R¹ substituiert sein kann. Diese Gruppe ist eine Ausführungsform eines aromatischen oder heteroaromatischen Ringsystems, wobei dann mehrere Aryl- bzw. Heteroarylgruppen durch ein Stickstoffatom miteinander verknüpft sind. Wenn R für eine Triaryl- bzw. -heteroarylamingruppe steht, weist diese Gruppe bevorzugt 18 bis 30 aromatische Ringatome auf und kann durch einen oder mehrere Reste R¹, bevorzugt nicht-aromatische Reste R¹, substituiert sein.

In einer weiteren bevorzugten Ausführungsform der Erfindung ist Ar' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist Ar' ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R¹ substituiert sein kann.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Geeignete aromatische bzw. heteroaromatische Ringsysteme R bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3-oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R¹ substituiert sein können. Wenn R bzw. Ar' für eine Heteroarylgruppe, insbesondere für Triazin, Pyrimidin oder Chinazolin steht, können auch aromatische oder heteroaromatische Reste R¹ an dieser Heteroarylgruppe bevorzugt sein.

Dabei sind die Gruppen R bzw. Ar' bevorzugt gewählt aus den Gruppen der folgenden Formeln R-1 bis R-76, 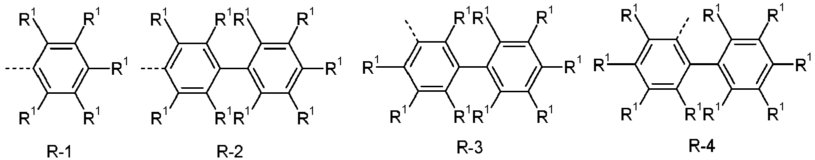 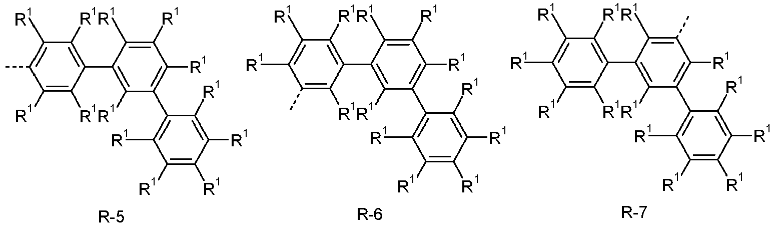 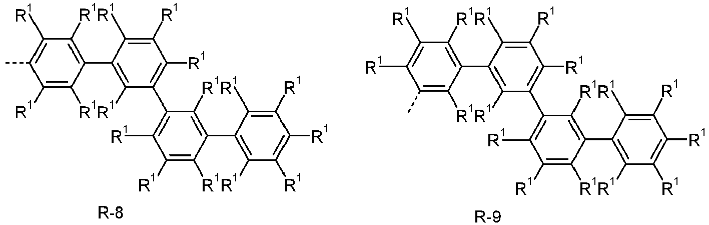 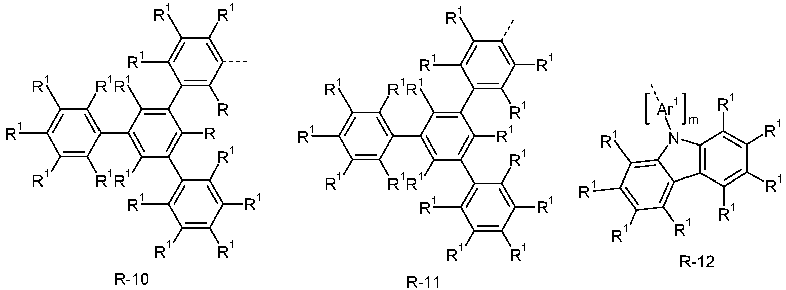 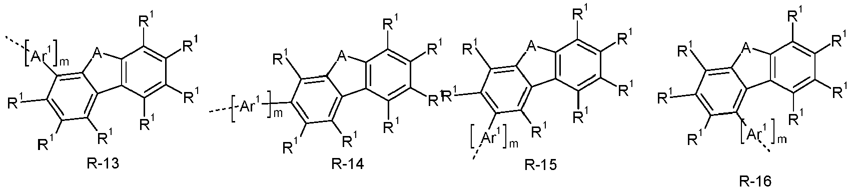 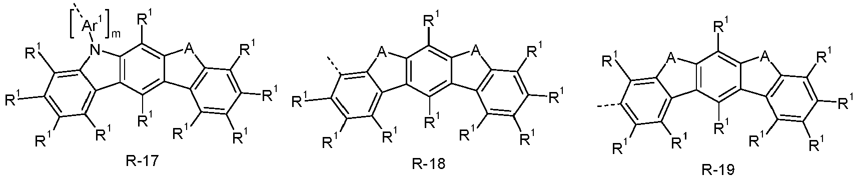 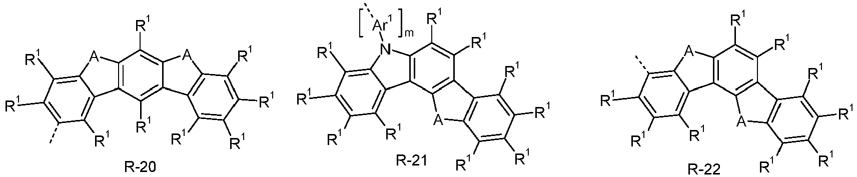 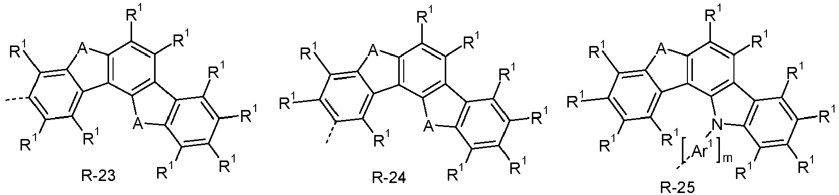 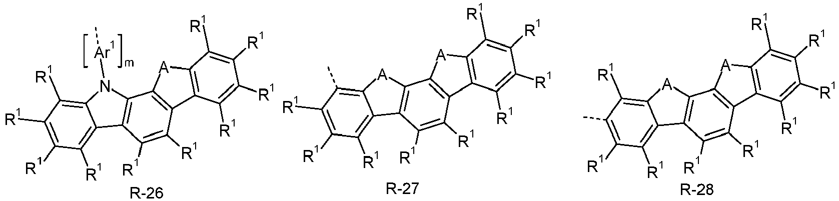 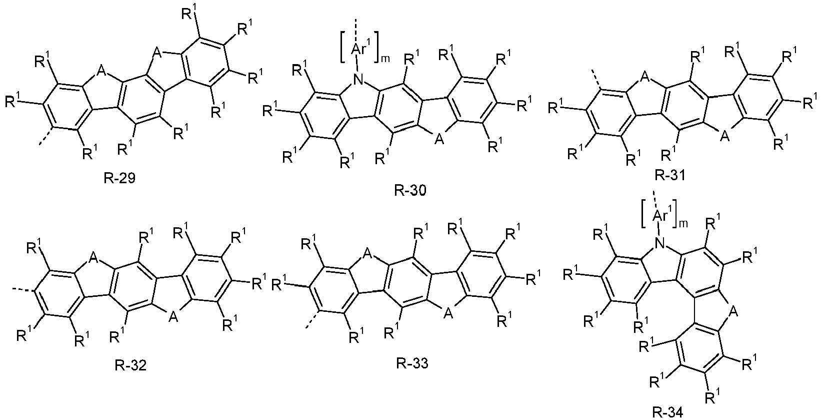 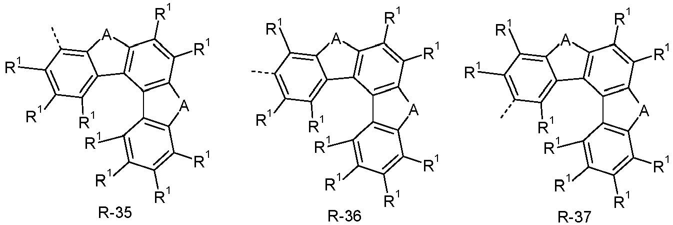 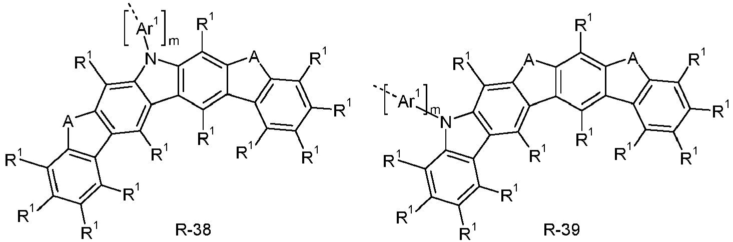 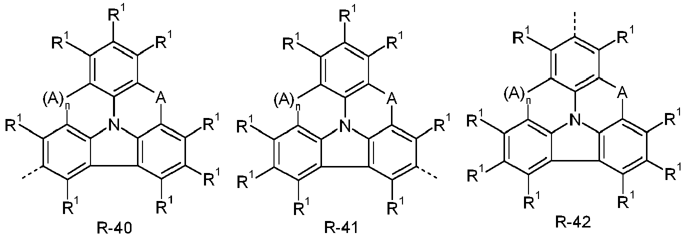 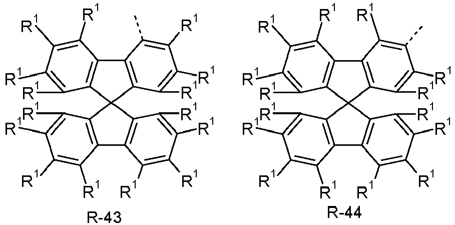 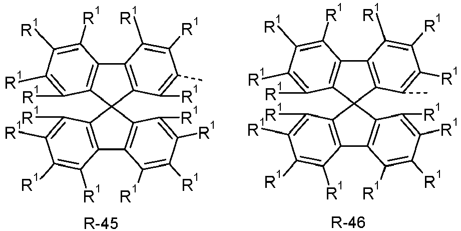 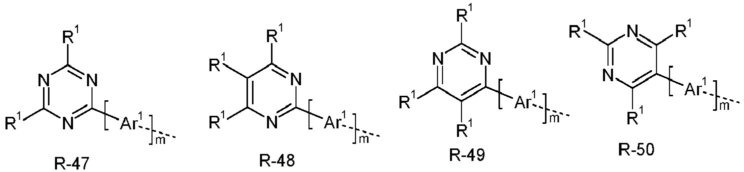 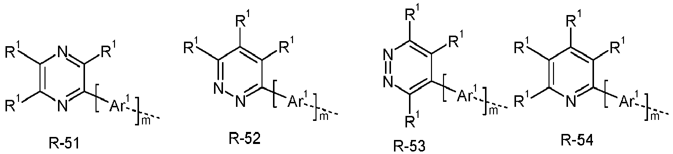 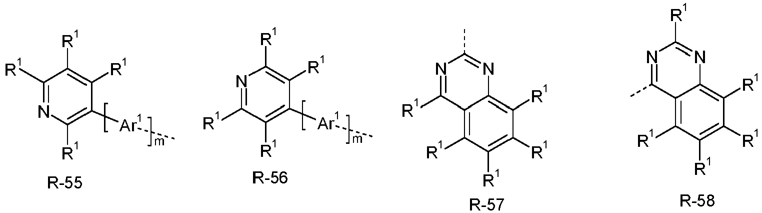 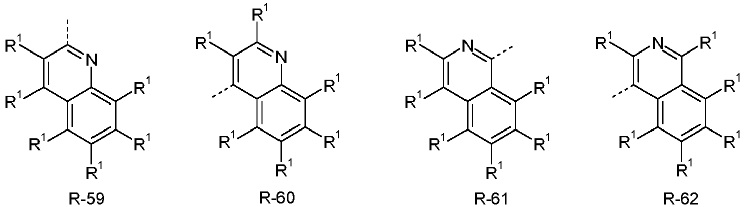 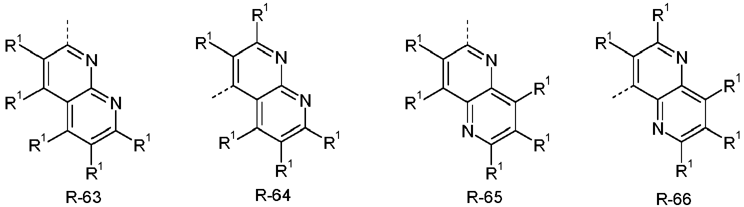 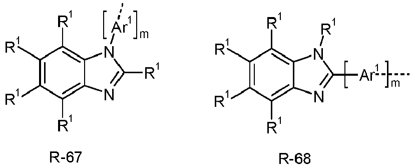 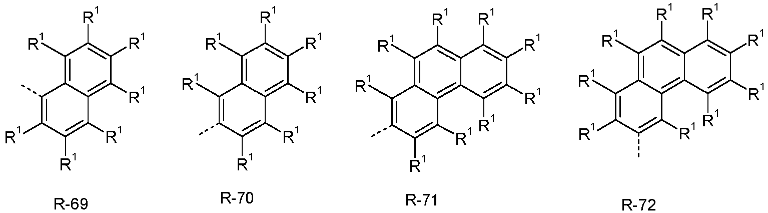 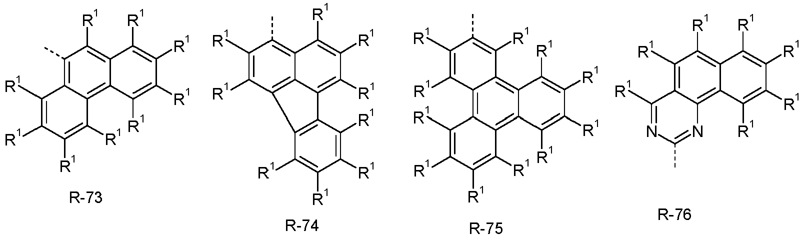 wobei R¹ die oben genannten Bedeutungen aufweist, die gestrichelte Bindung die Bindung an ein Kohlenstoffatom des Grundgerüsts in Formel (1) bzw. in den bevorzugten Ausführungsformen bzw. an das Stickstoffatom in der Gruppe N(Ar')₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an ein Kohlenstoffatom des Grundgerüsts in Formel (1) bzw. in den bevorzugten Ausführungsformen bzw. an das Stickstoffatom in der Gruppe N(Ar')₂ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar' handelt.

Wenn die oben genannten Gruppen Ar-1 bis Ar-76 für Ar bzw. R-1 bis R-76 für R bzw. Ar' mehrere Gruppen A aufweisen, so kommen hierfür alle Kombinationen aus der Definition von A in Frage. Bevorzugte Ausführungsformen sind dann solche, in denen eine Gruppe A für NR bzw. NR¹ und die andere Gruppe A für C(R)₂ bzw. C(R¹)₂ steht oder in denen beide Gruppen A für NR bzw. NR¹ stehen oder in denen beide Gruppen A für O stehen. In einer besonders bevorzugten Ausführungsform der Erfindung steht in Gruppen Ar, R bzw. Ar', die mehrere Gruppen A aufweisen, mindestens eine Gruppe A für C(R)₂ bzw. C(R¹)₂ oder für NR bzw. NR¹.

Wenn A für NR bzw. NR¹ steht, steht der Substituent R bzw. R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹ bzw. R² substituiert sein kann. In einer besonders bevorzugten Ausführungsform steht dieser Substituent R bzw. R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R¹ bzw. R² substituiert sein kann. Besonders bevorzugt sind Phenyl, Biphenyl, Terphenyl und Quaterphenyl mit Verknüpfungsmustern, wie vorne für Ar-1 bis Ar-11 bzw. R-1 bis R-11 aufgeführt, wobei diese Strukturen durch einen oder mehrere Reste R¹ bzw. R² substituiert sein können, bevorzugt aber unsubstituiert sind.

Wenn A für C(R)₂ bzw. C(R¹)₂ steht, stehen die Substituenten R bzw. R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ bzw. R² substituiert sein kann. Ganz besonders bevorzugt steht R bzw. R¹ für eine Methylgruppe oder für eine Phenylgruppe. Dabei können die Reste R bzw. R¹ auch miteinander ein Ringsystem bilden, was zu einem Spirosystem führt.

Weitere geeignete Gruppen Ar, R bzw. Ar' sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Für Ar ergibt sich eine solche Gruppe, indem die Gruppe Ar mit einer Gruppe N(Ar')₂ substituiert ist. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe, ausgewählt aus C(R¹)₂, NR¹, O oder S, verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, insbesondere mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe. Dies gilt insbesondere, wenn Ar⁴ mit Ar² durch einen Einfachbindung verbunden ist.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben die Alkylgruppen in erfindungsgemäßen Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste Ar, R, Ar', R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

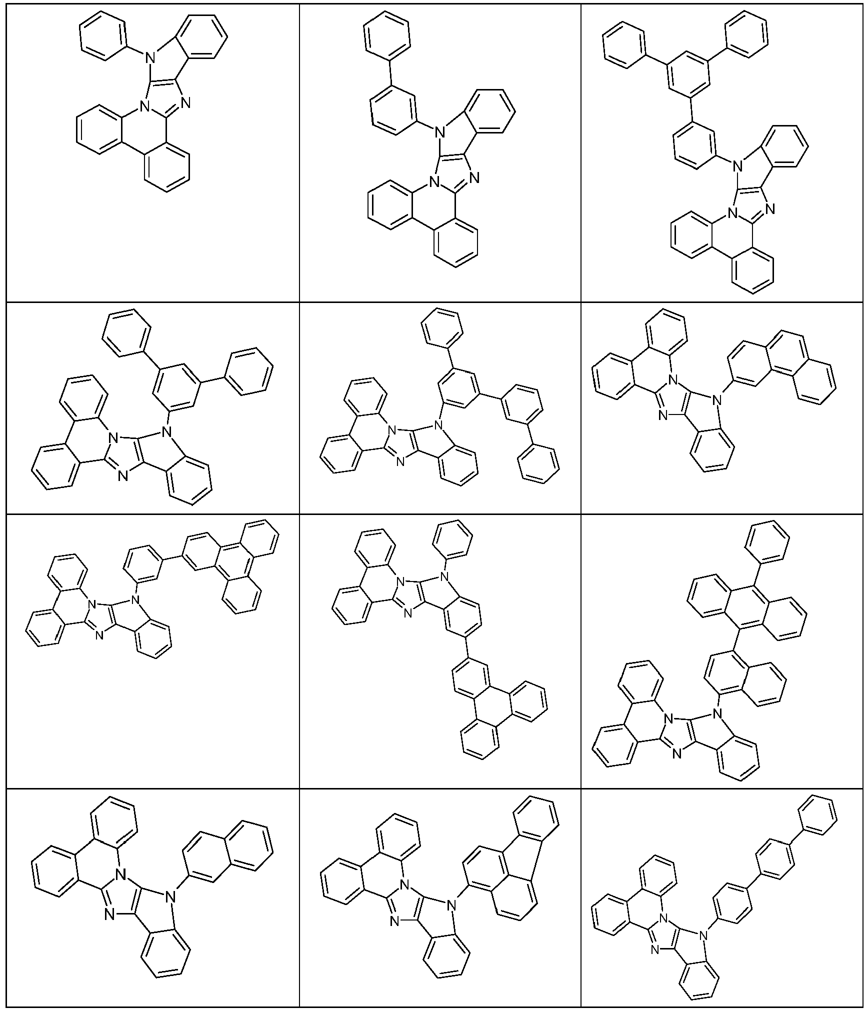

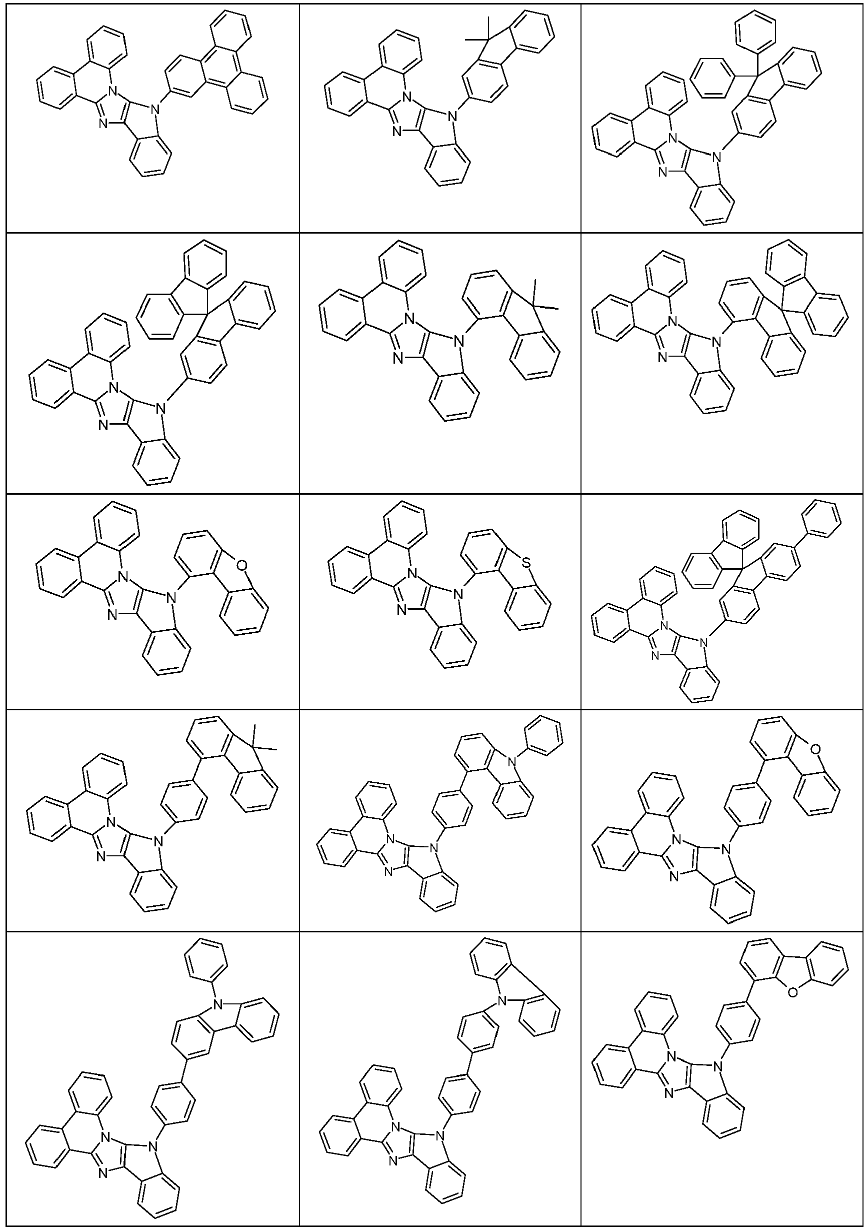

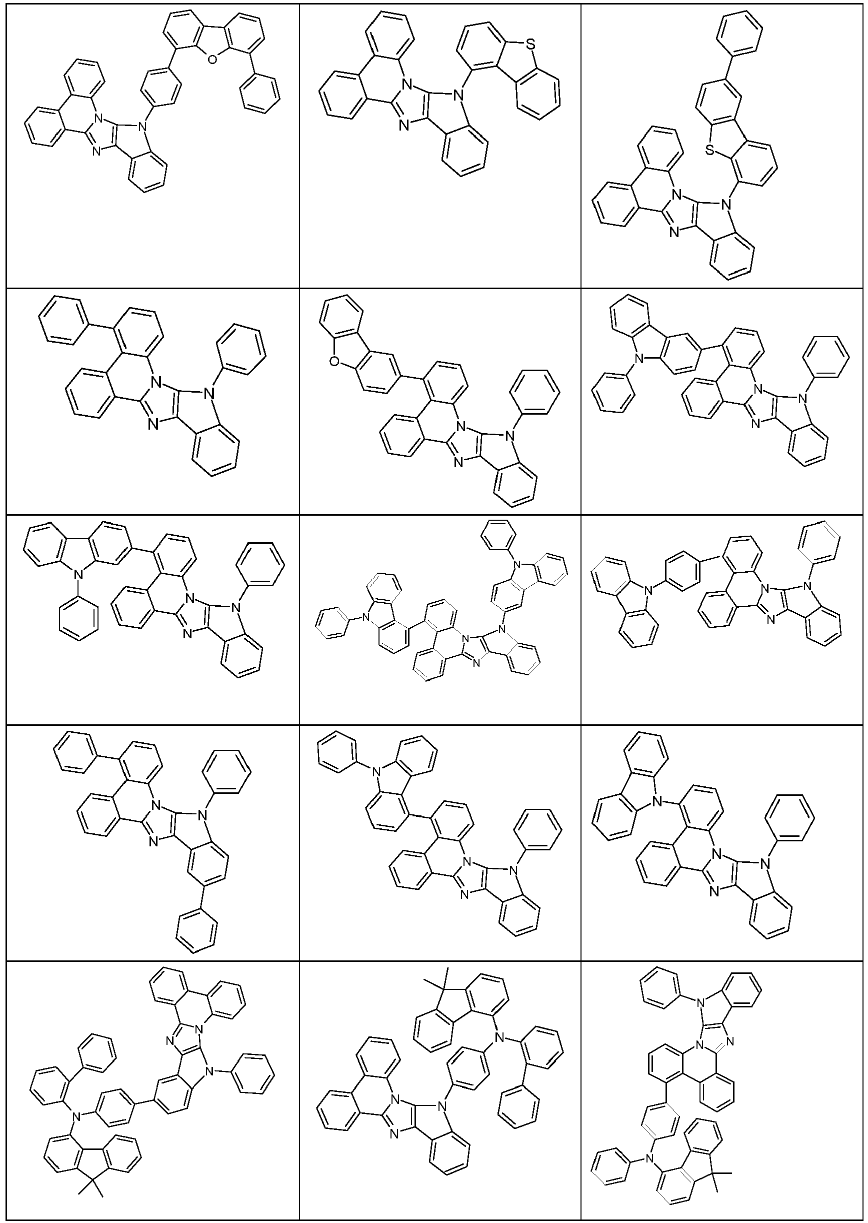

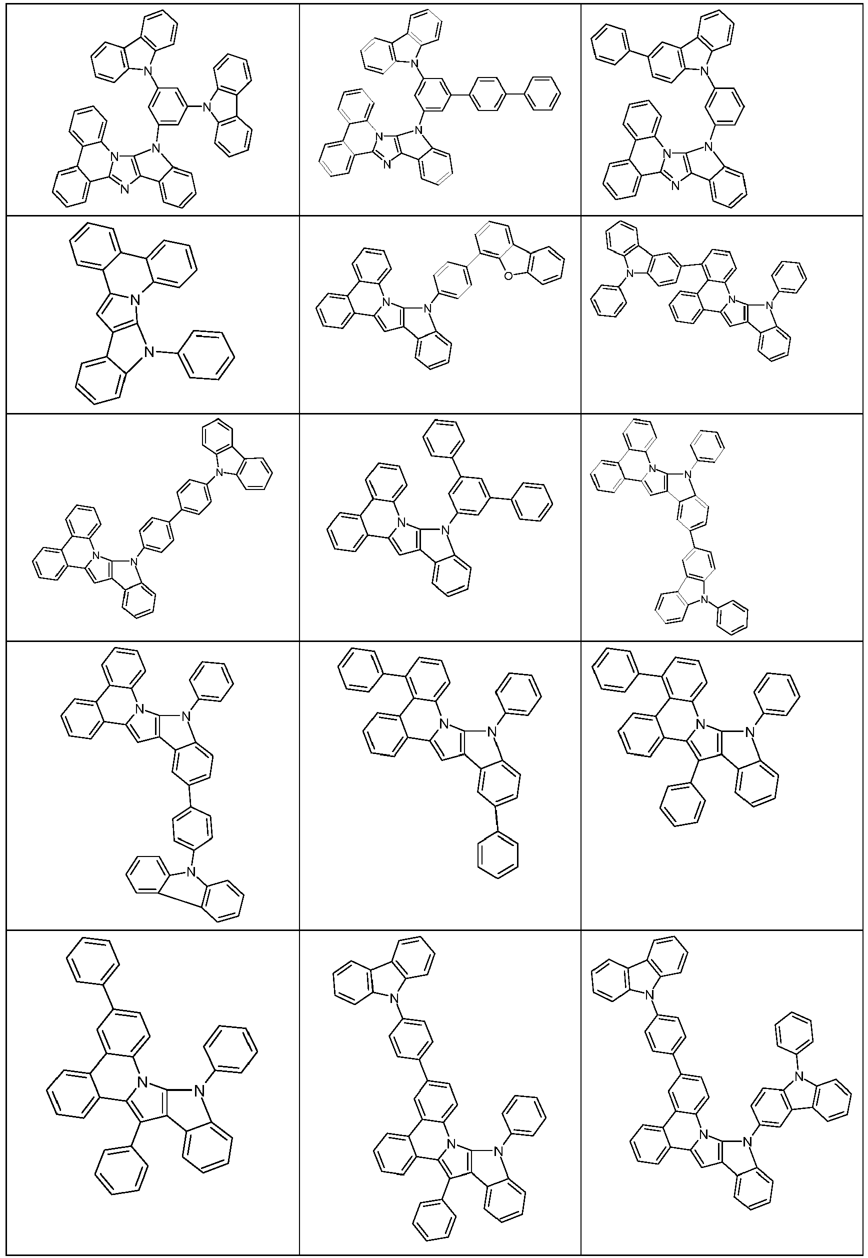

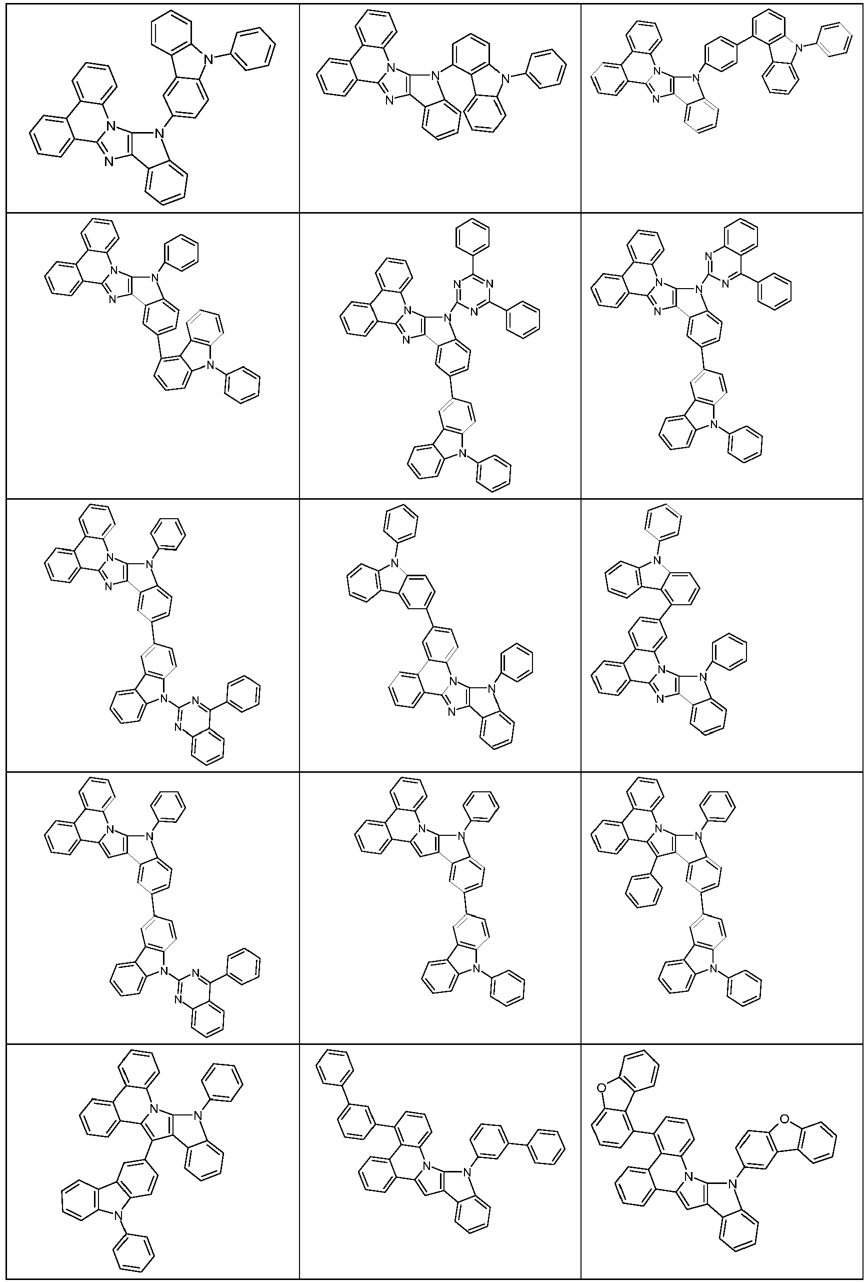

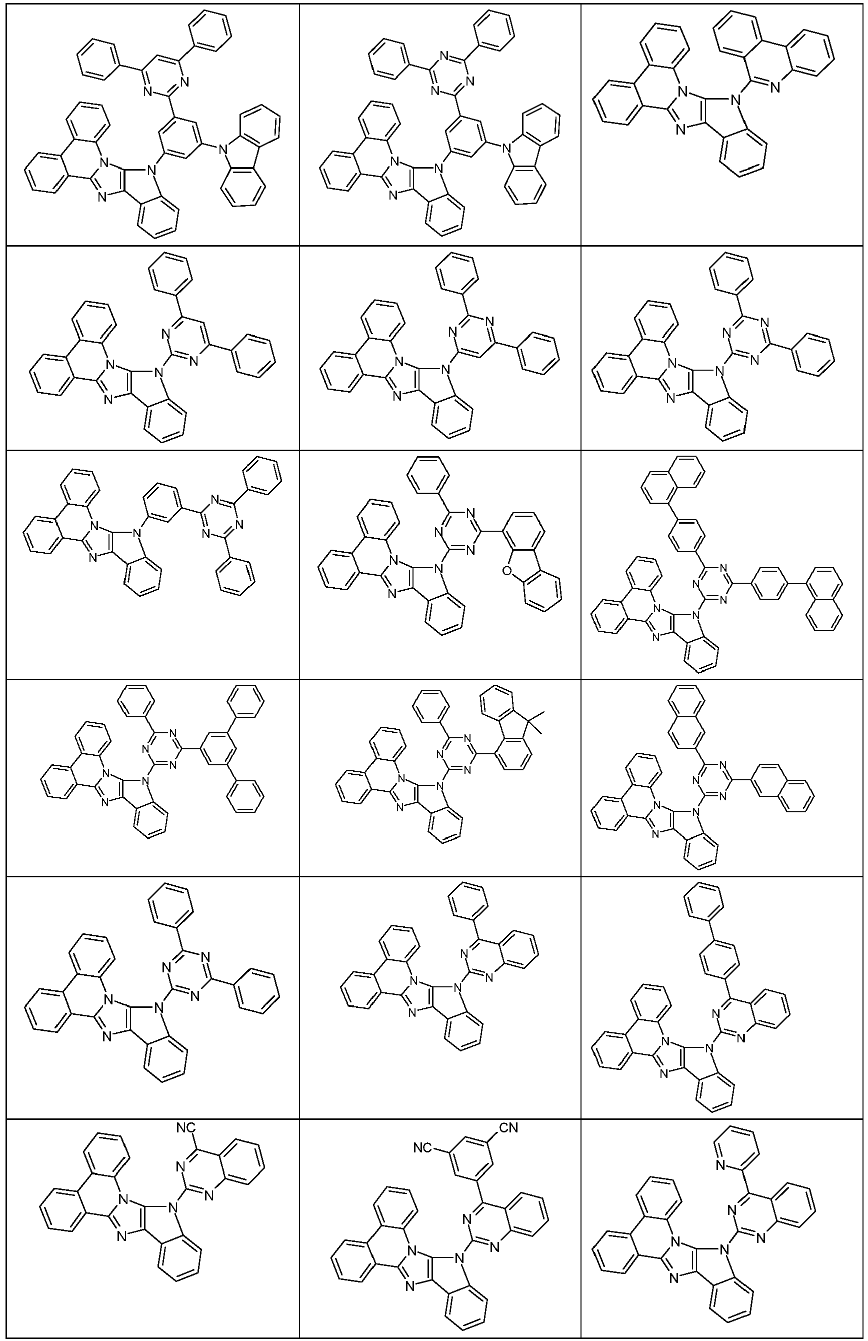

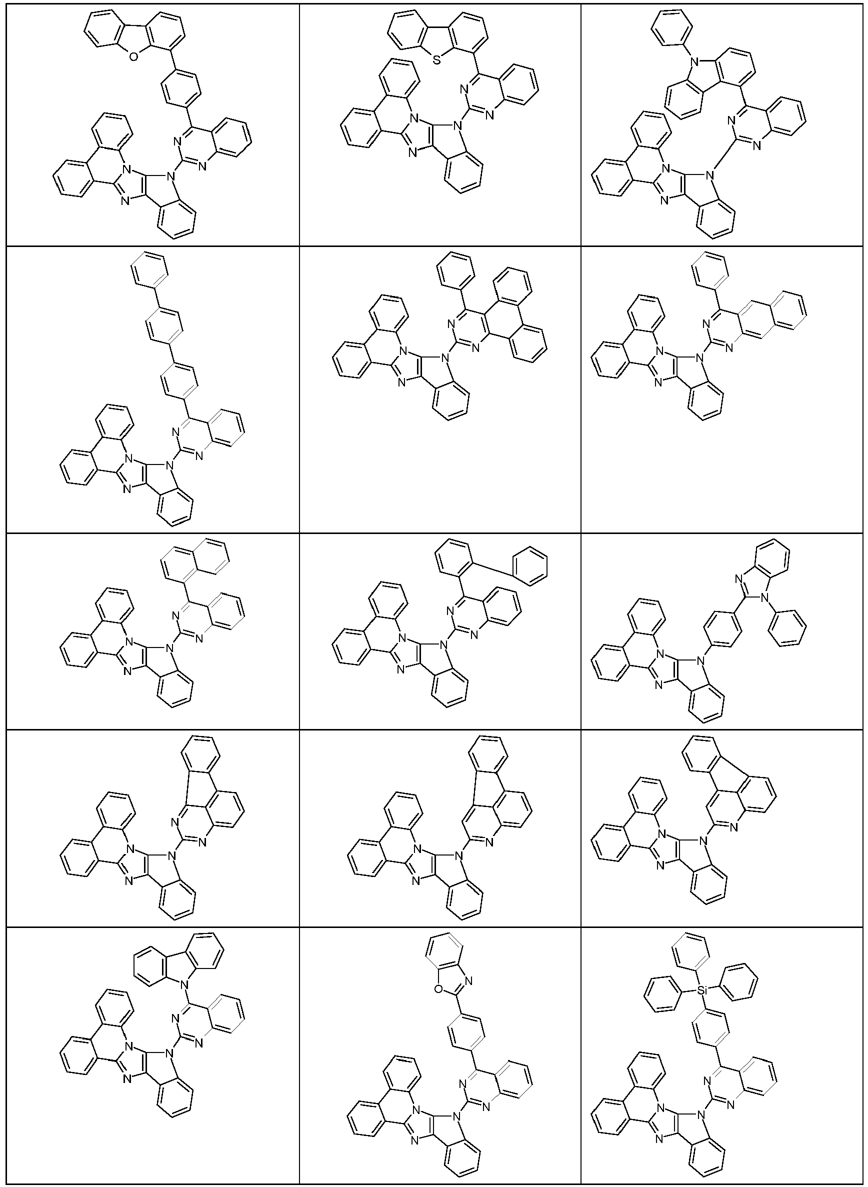

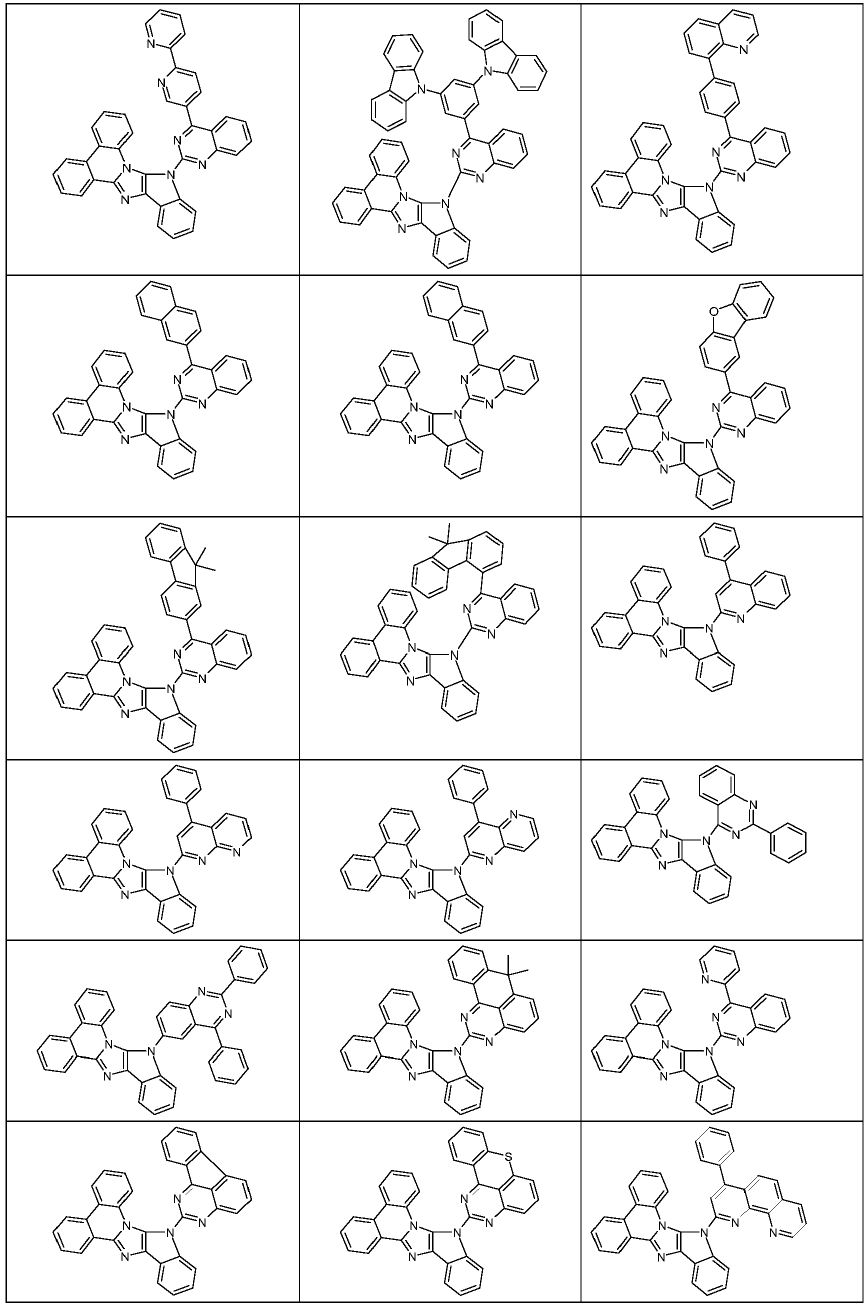

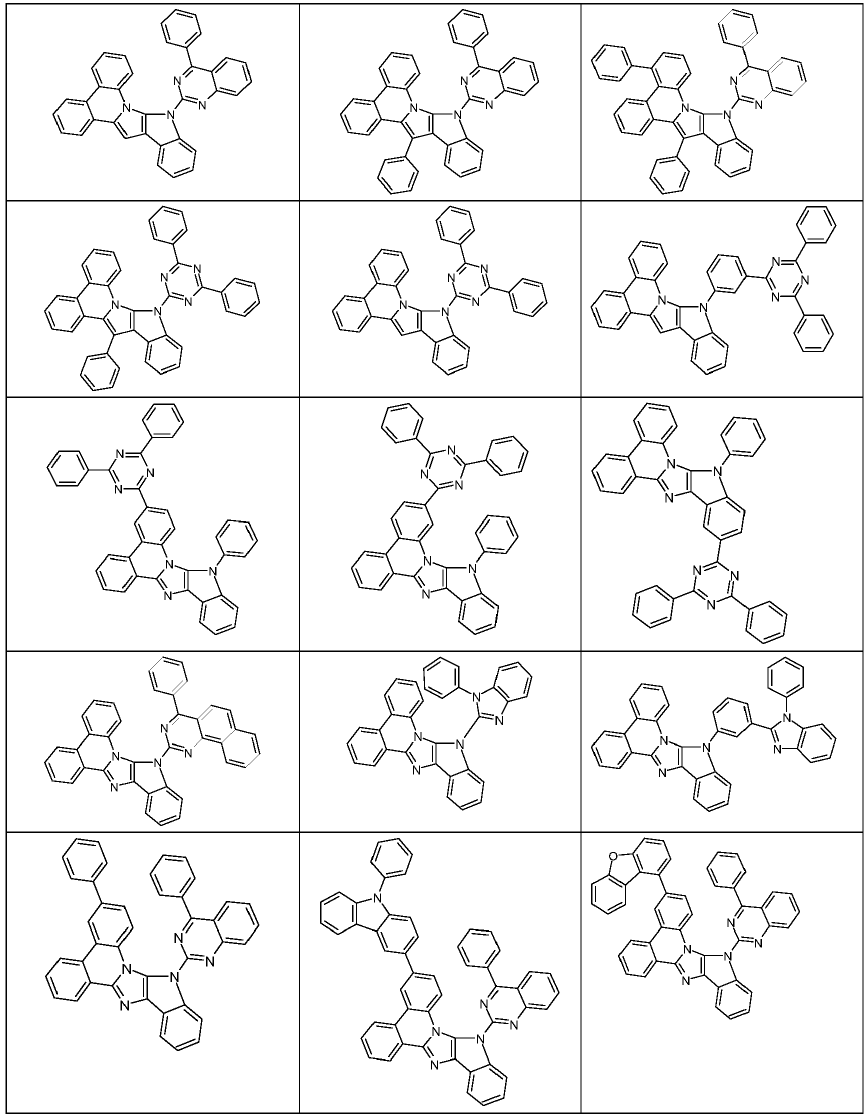

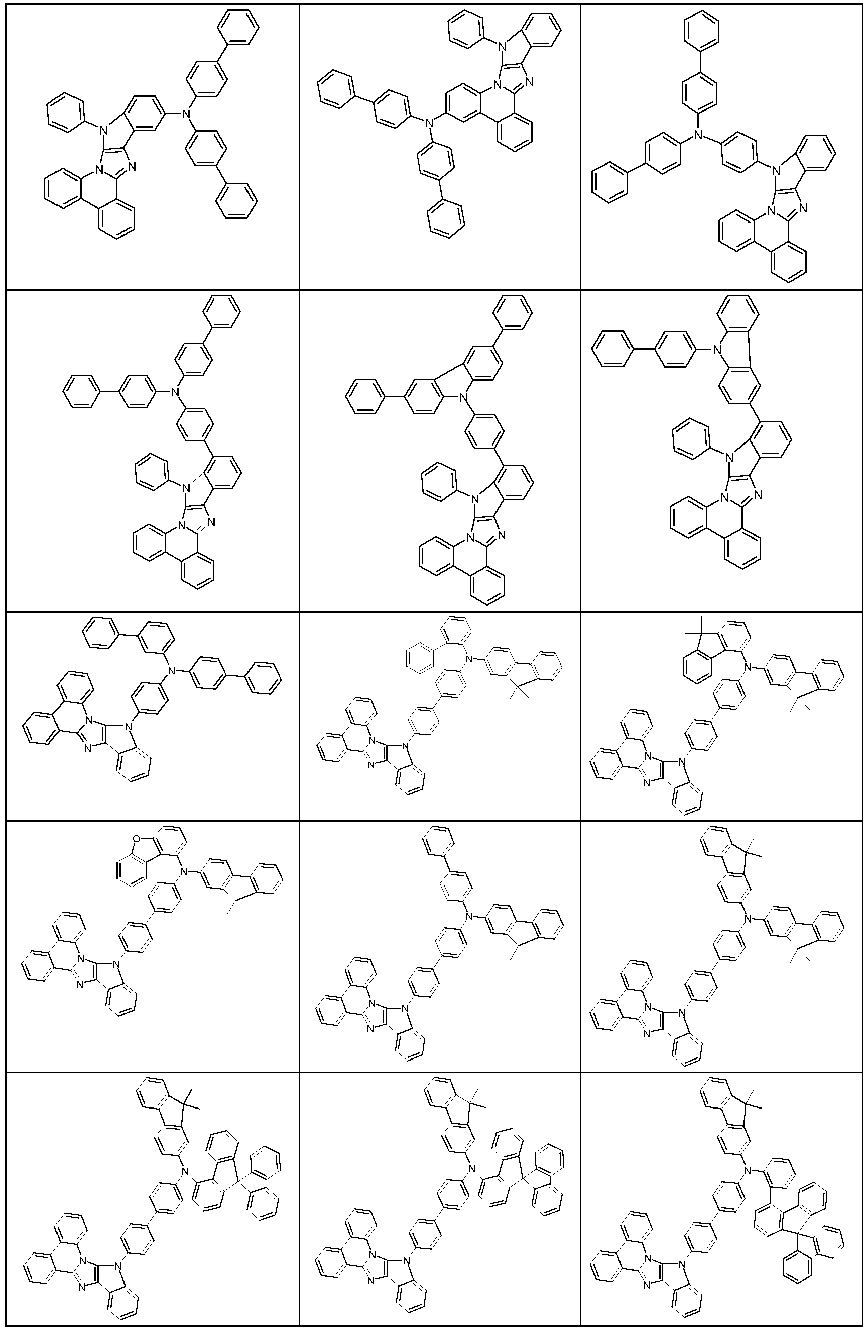

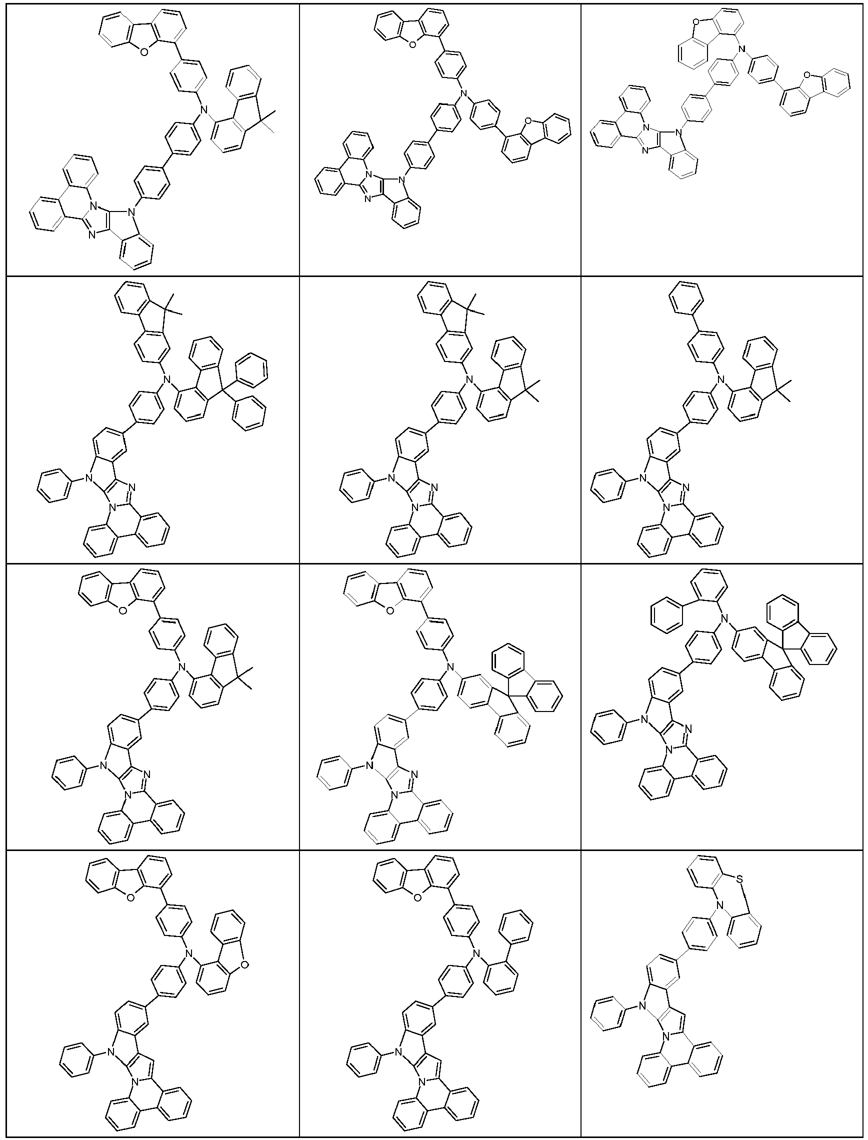

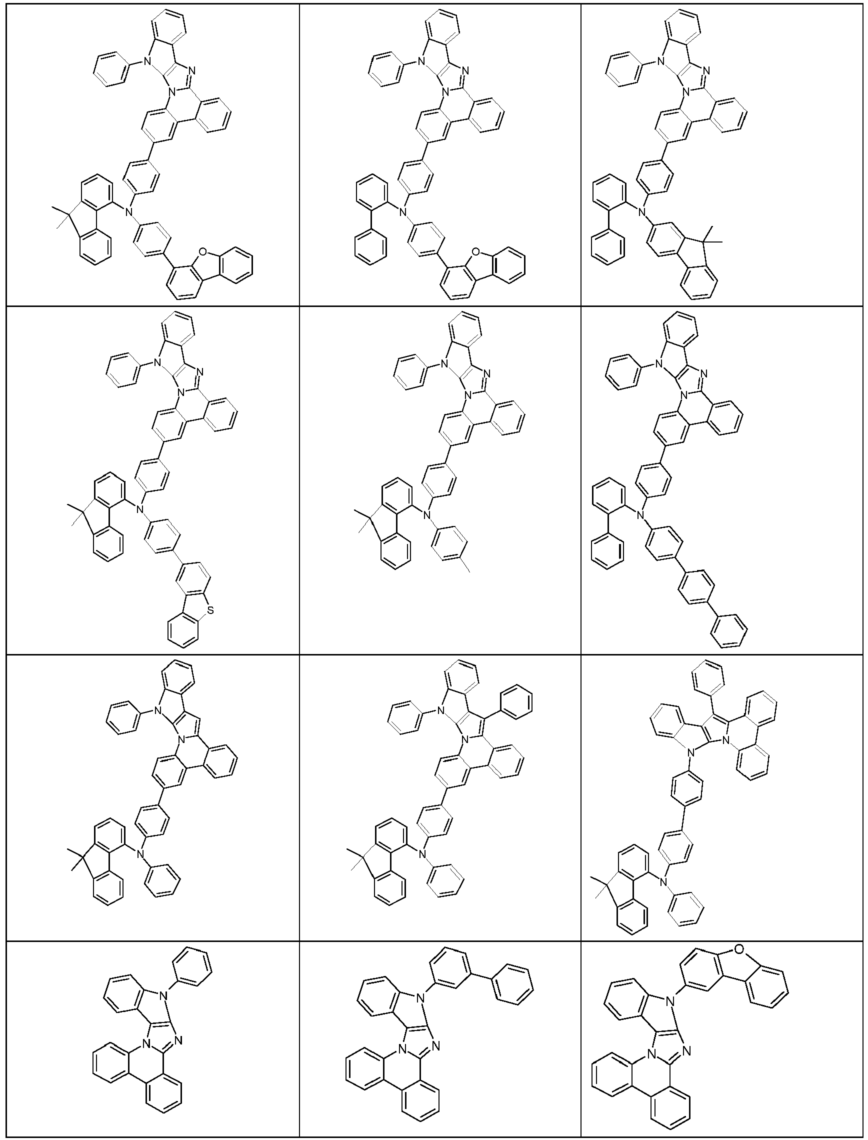

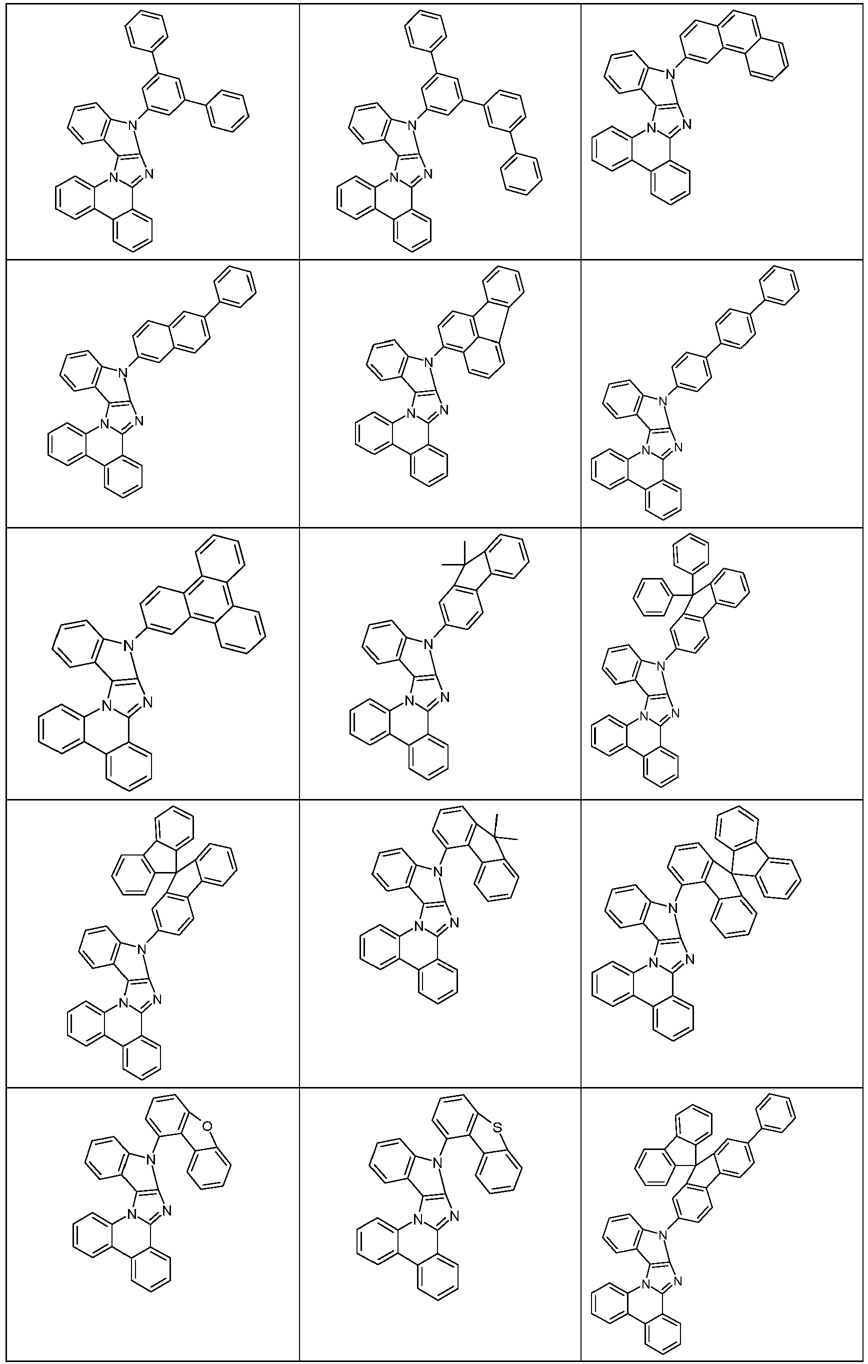

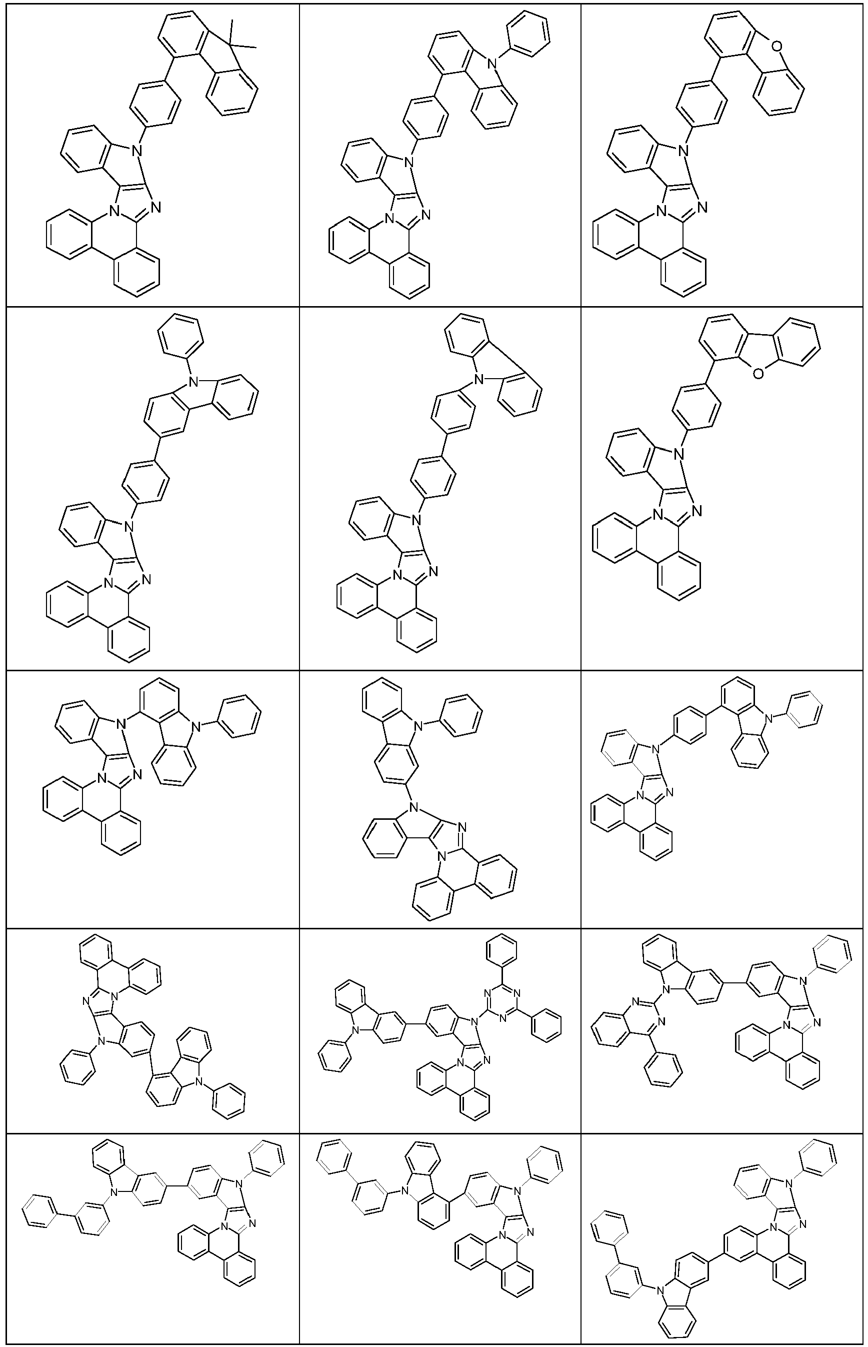

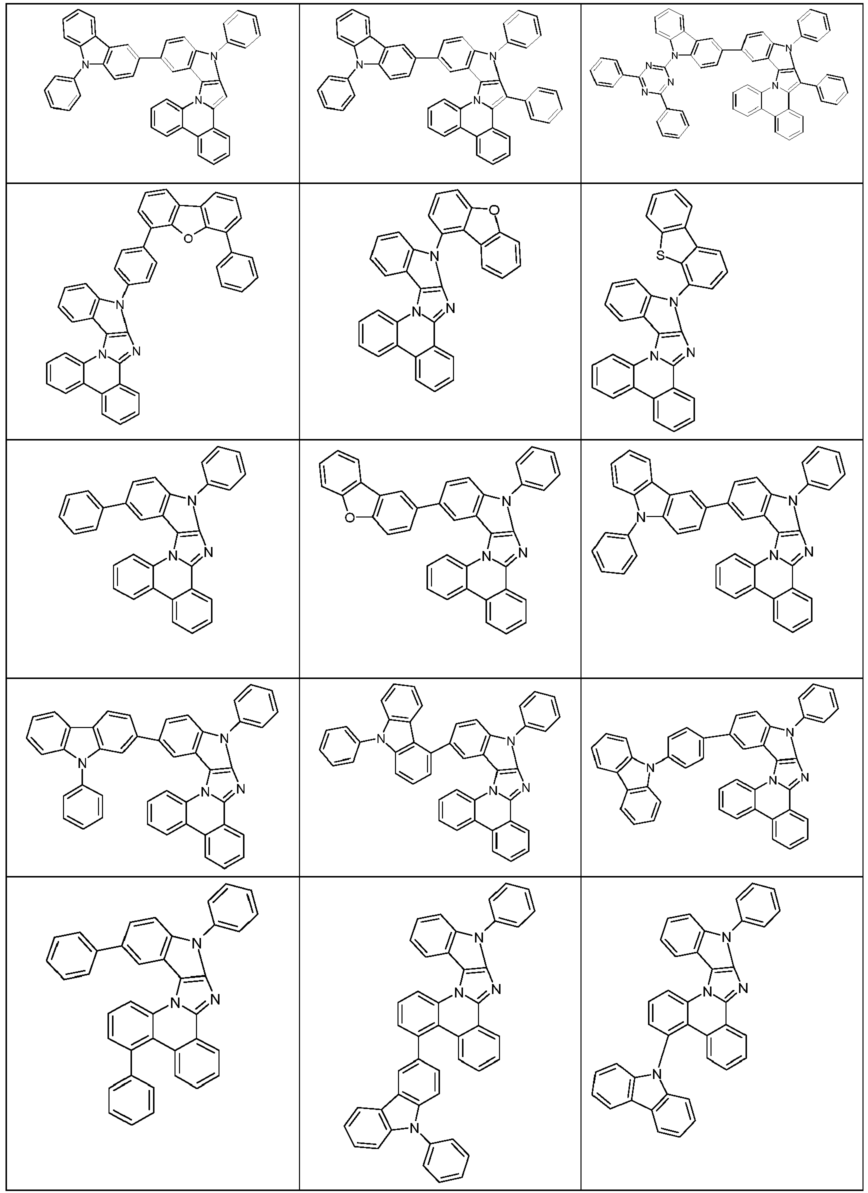

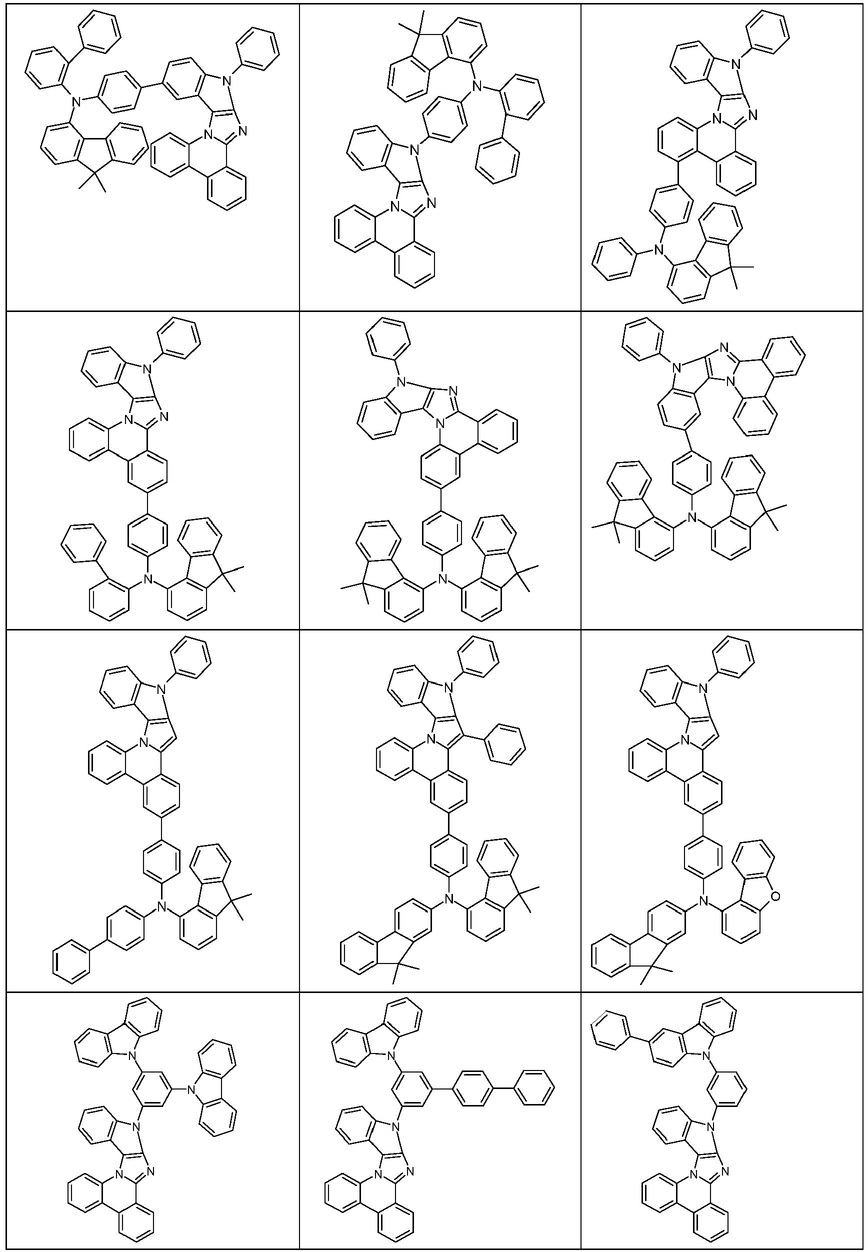

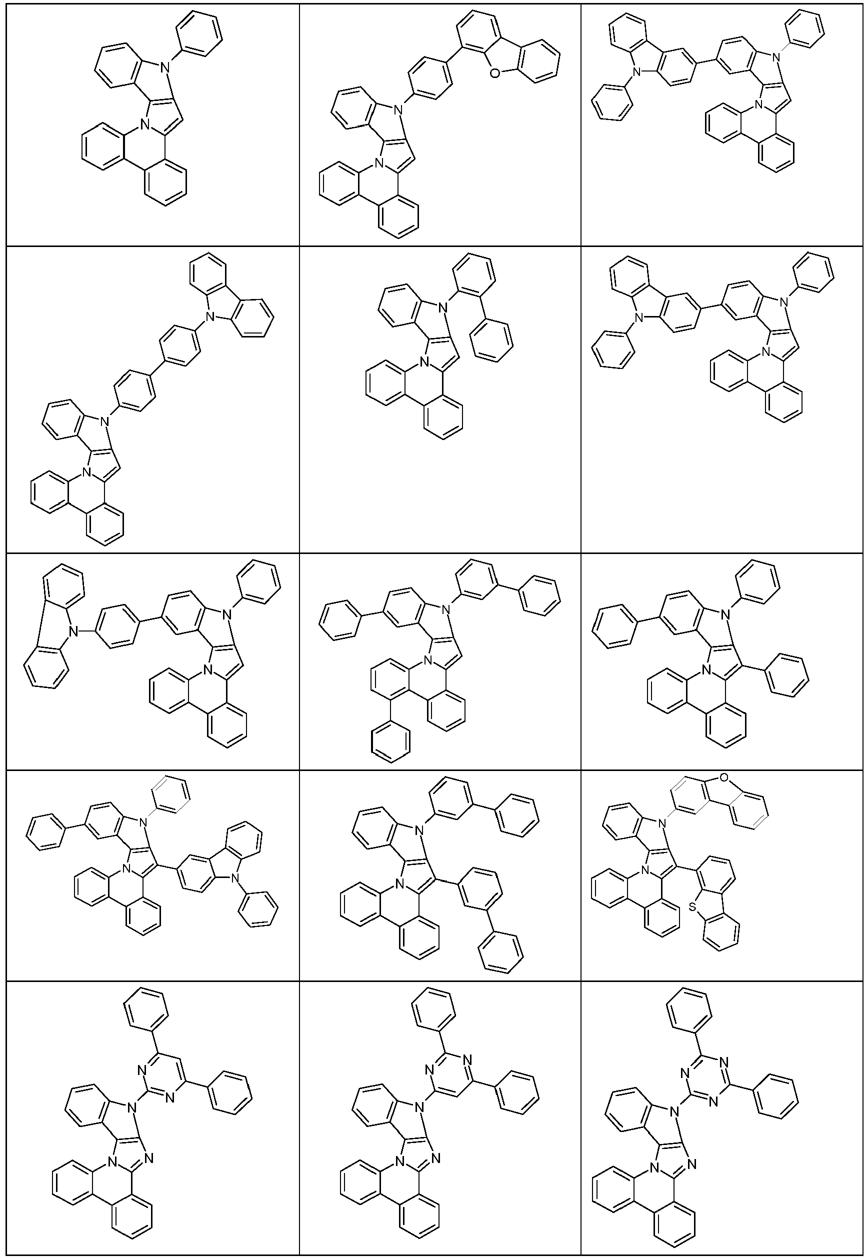

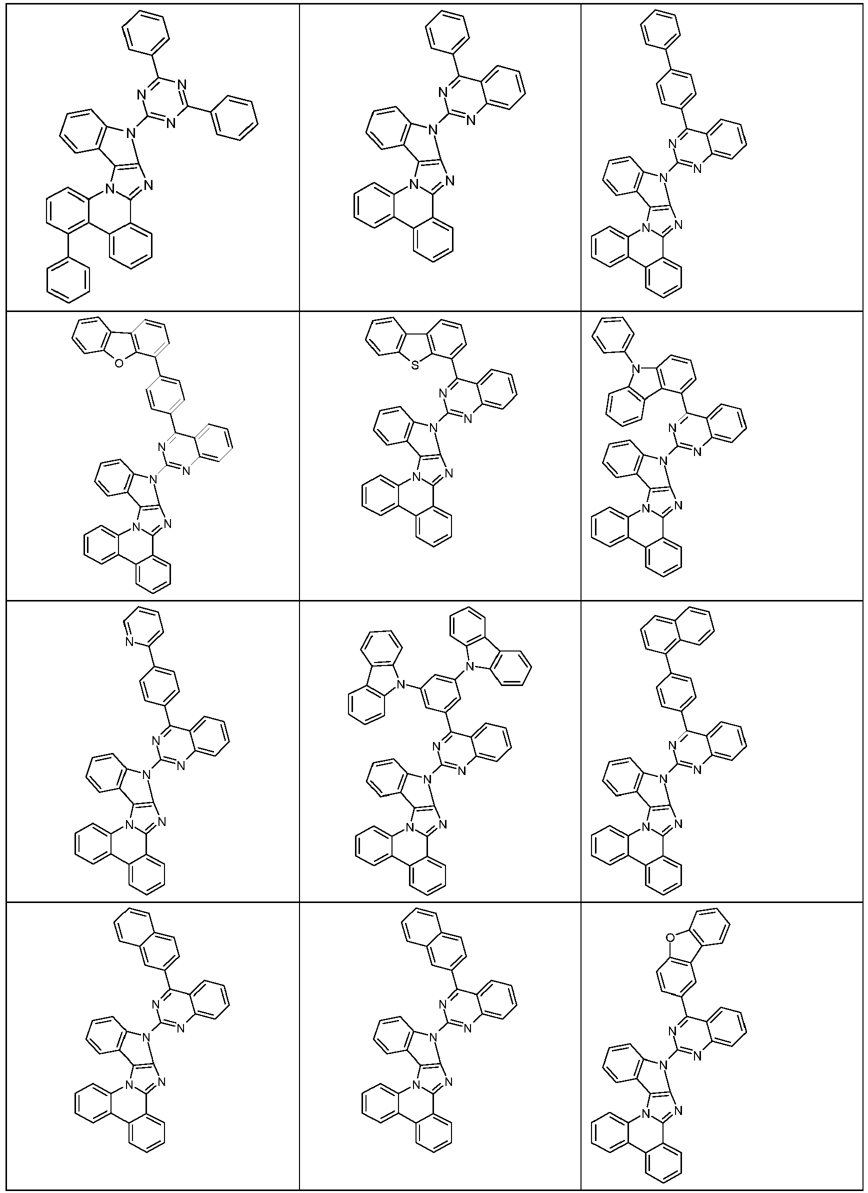

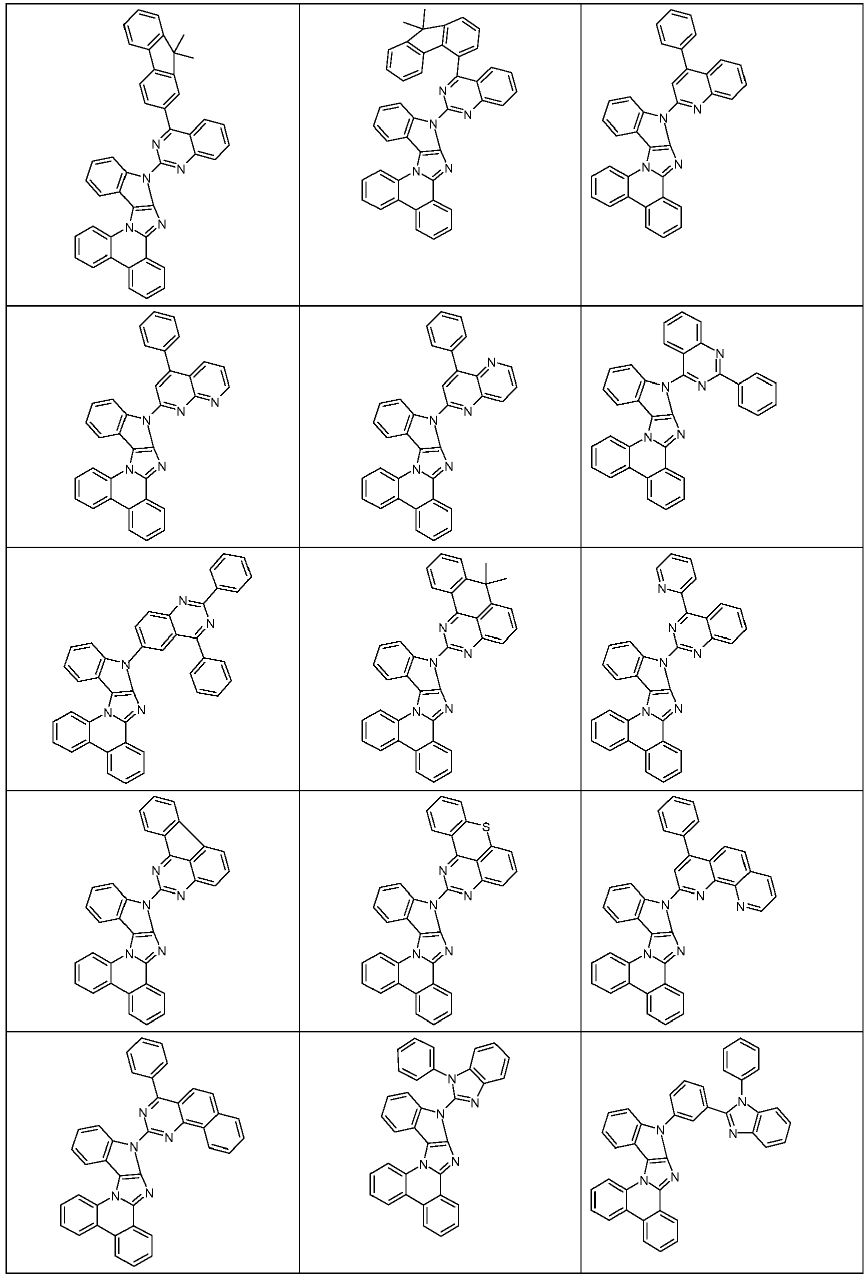

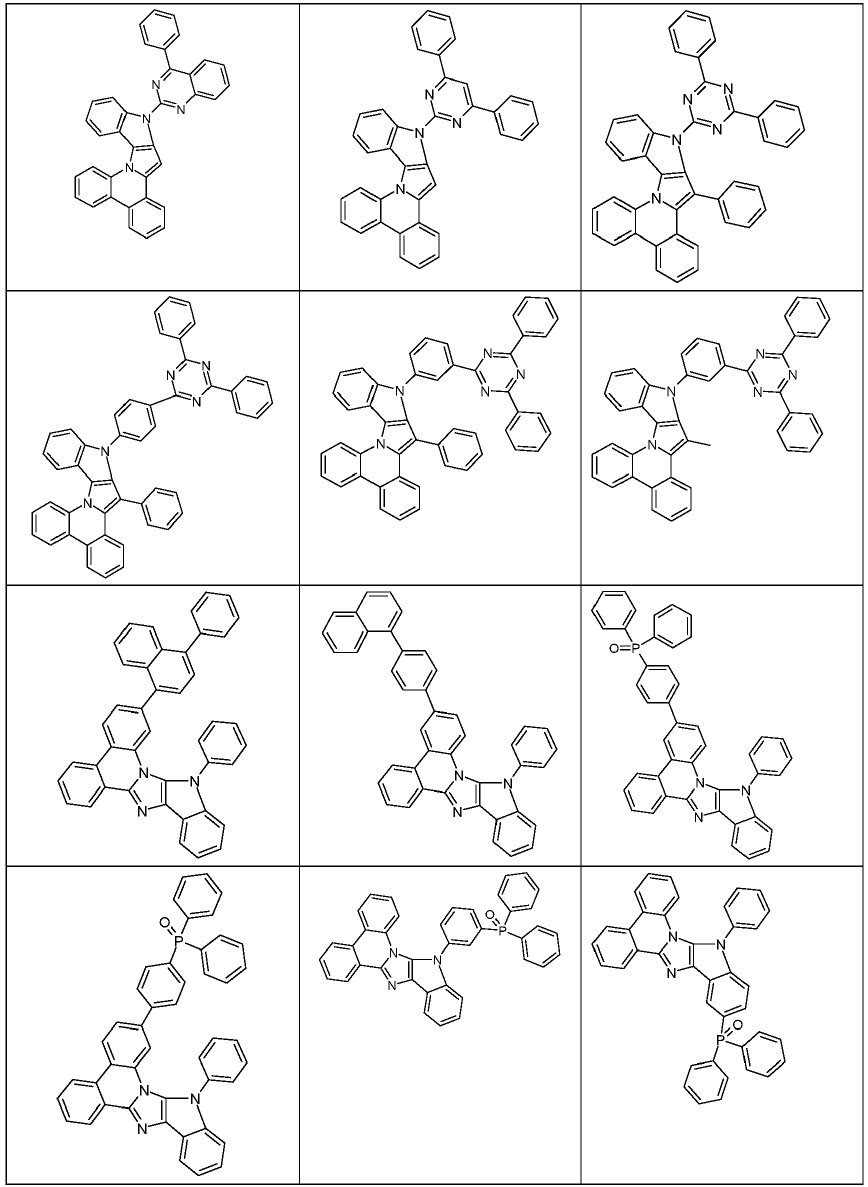

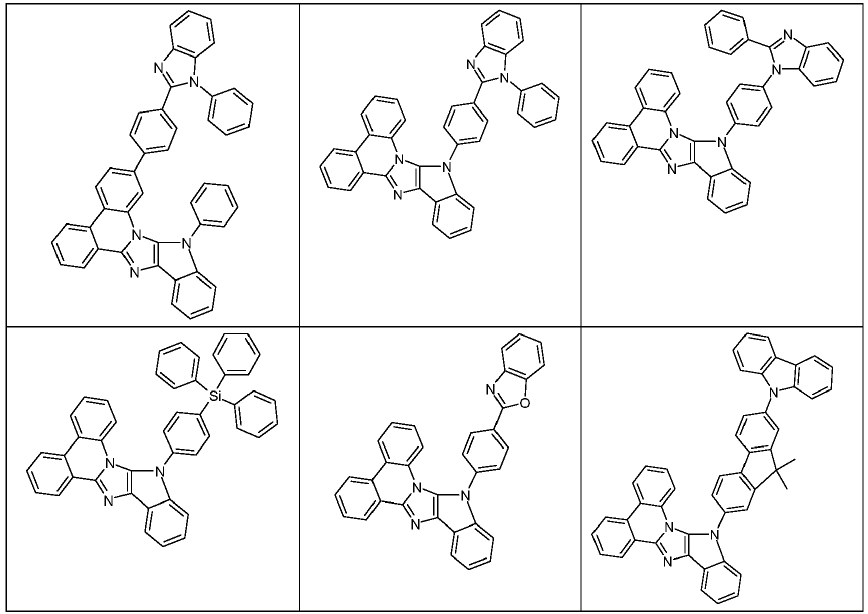

Die Grundstruktur der erfindungsgemäßen Verbindungen kann nach dem in Schema 1 bis 3 skizzierten Weg dargestellt werden. Dabei zeigt Schema 1 die Synthese von Brom-substituiertem Imidazo-phenanthridin. Dieses kann gemäß WO 2015/171627 ausgehend von optional substituiertem 2-Phenylimidazol durch Umsetzung mit Dibrombenzol, gefolgt von Bromierung synthetisiert werden. Alternativ kann die Synthese von Brom-substituiertem Imidazo-phenanthridin auch gemäß WO 2015/171627 ausgehend von optional substituiertem 2-Cyanobenzolboronsäure durch Umsetzung mit optional substituiertem 2-Chlor-6-amino-iodbenzol, gefolgt von Cyclisierung mit 2-Chloracetaldehyd, Suzuki-Kupplung und Bromierung erfolgen, wie in Schema 2 dargestellt. In Schema 3 ist die Umsetzung zur erfindungsgemäßen Verbindung gezeigt. Dazu wird die Bromgruppe durch Suzuki-Kupplung mit einer 2-Nitrobenzolboronsäure umgesetzt, gefolgt von Cyclisierung zum entsprechenden Indolderivat und Buchwald-Kupplung zur Einführung der Gruppe Ar am Stickstoffatom.

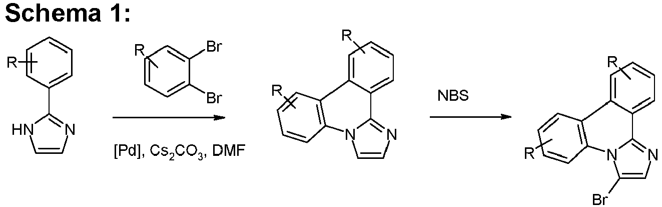

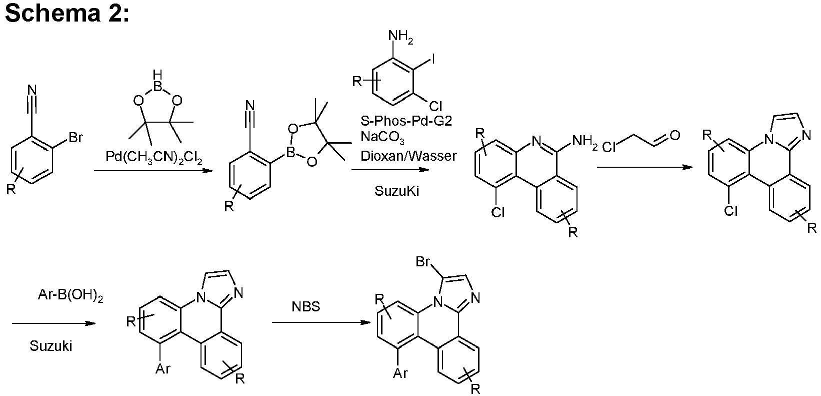

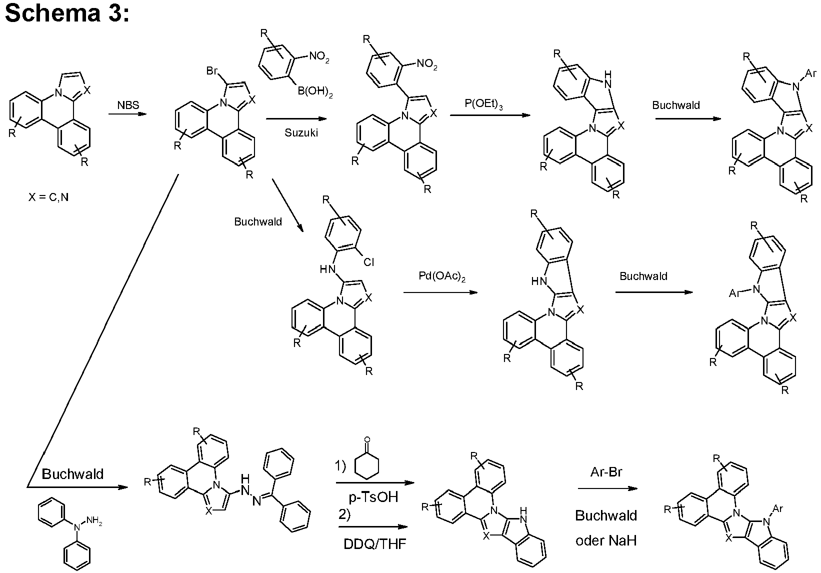

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und der noch nicht offen gelegten Anmeldung EP 16179378.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

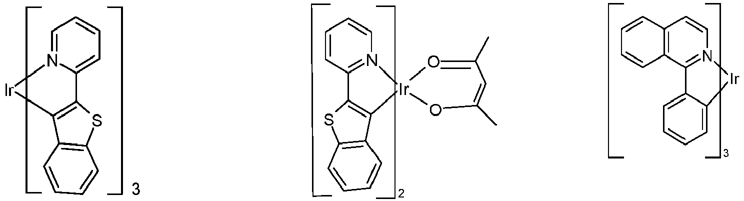

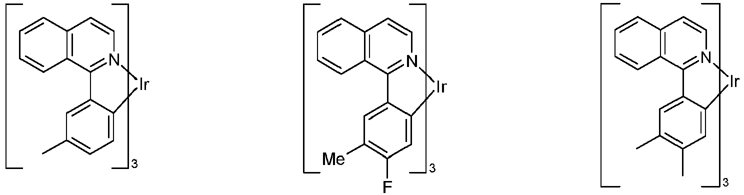

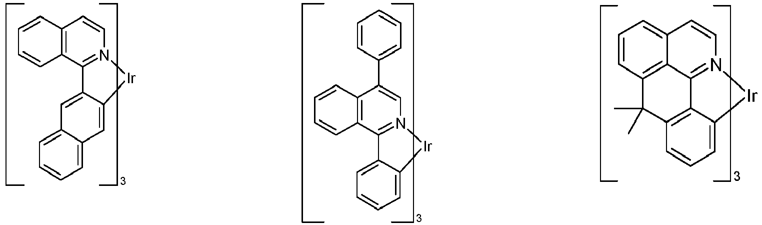

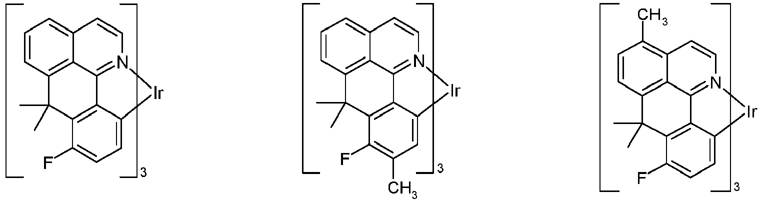

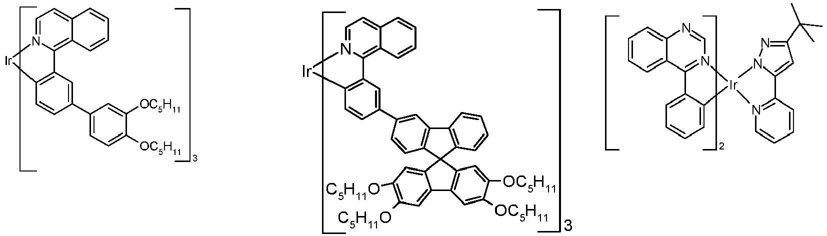

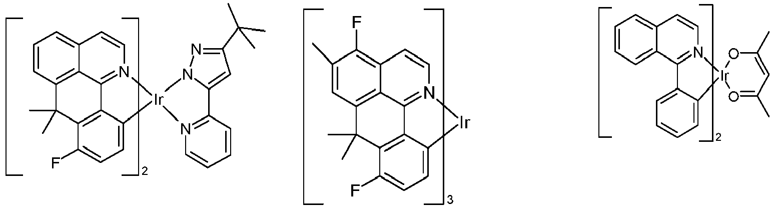

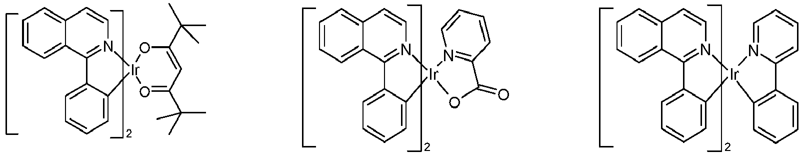

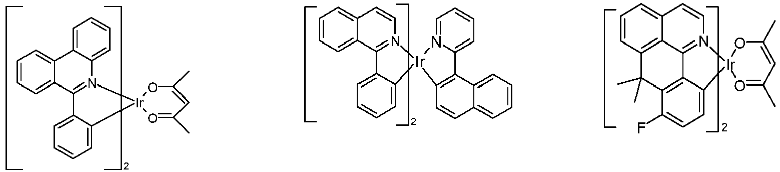

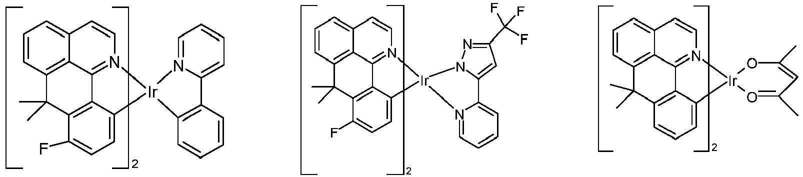

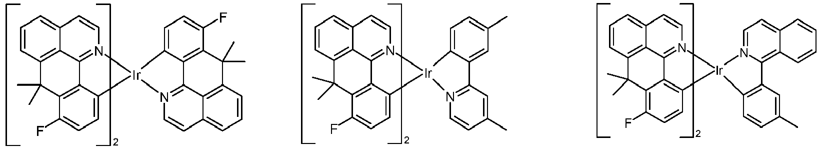

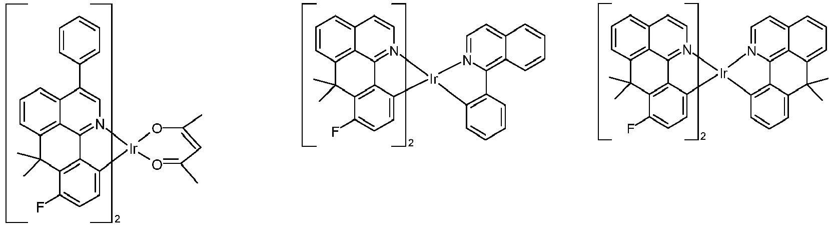

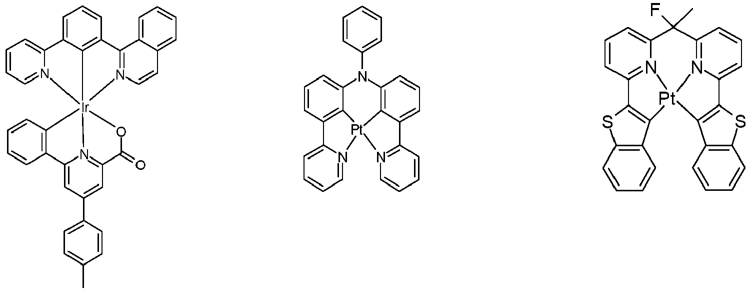

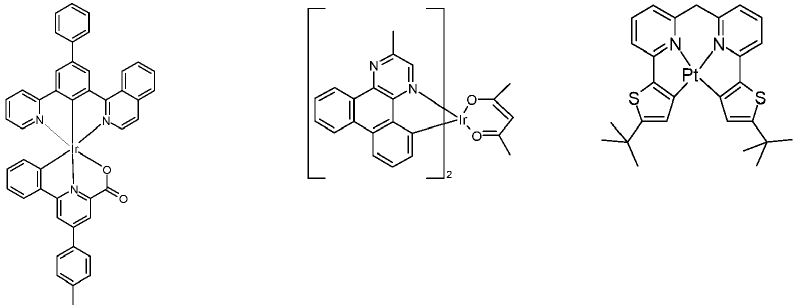

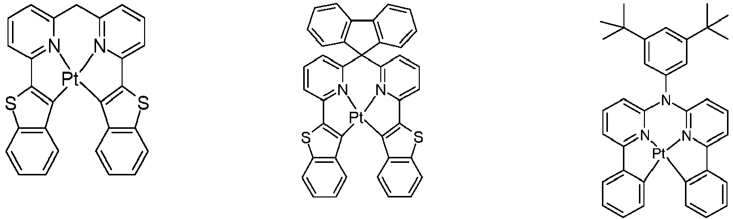

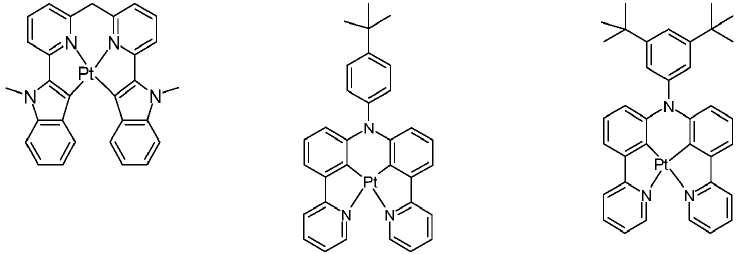

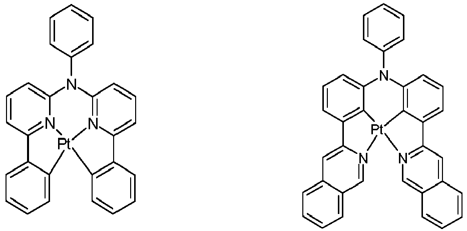

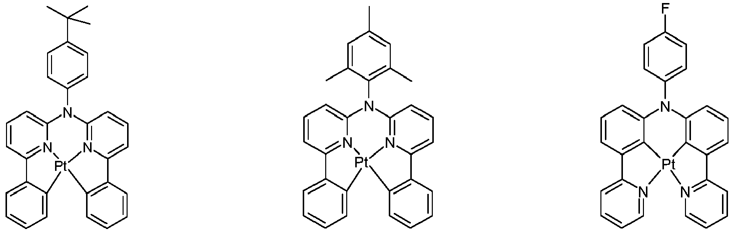

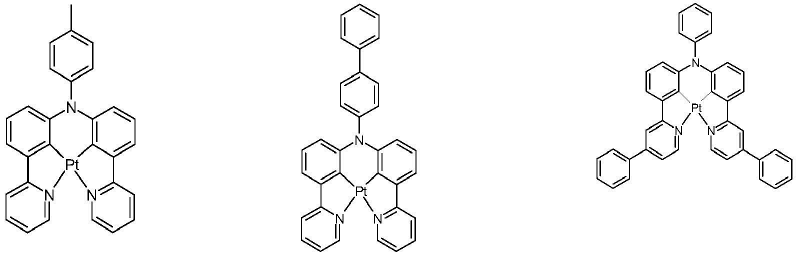

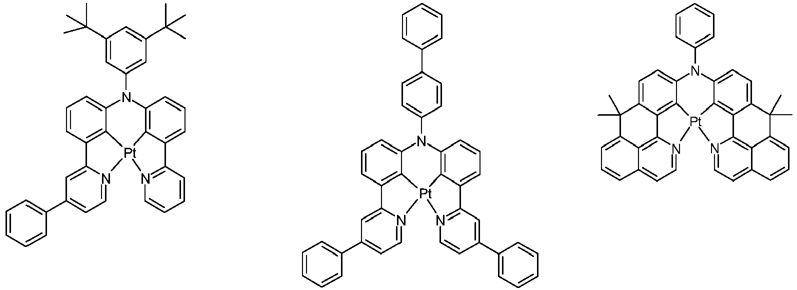

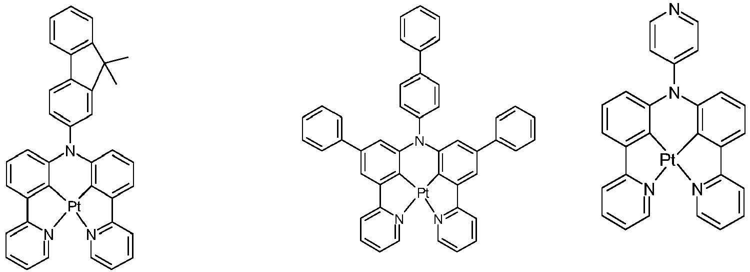

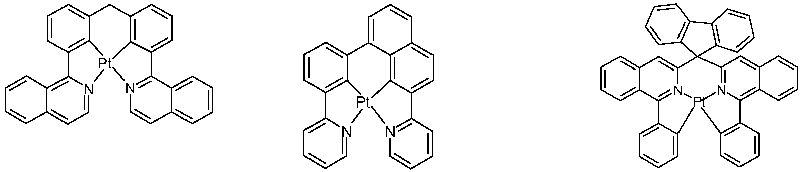

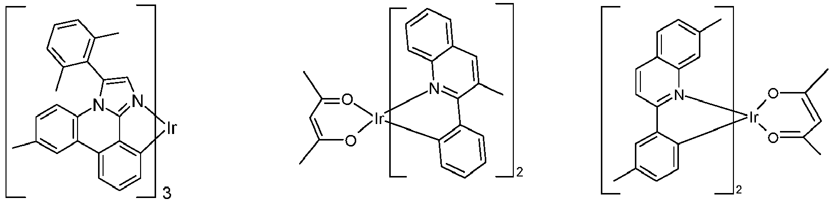

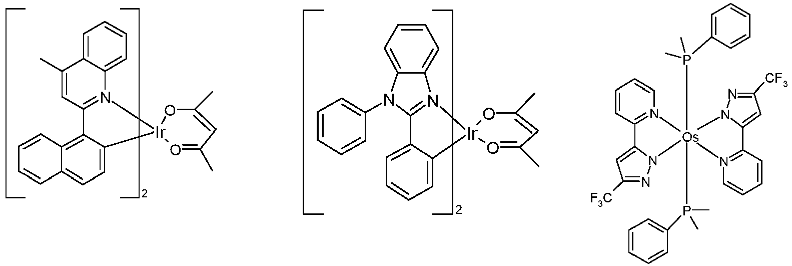

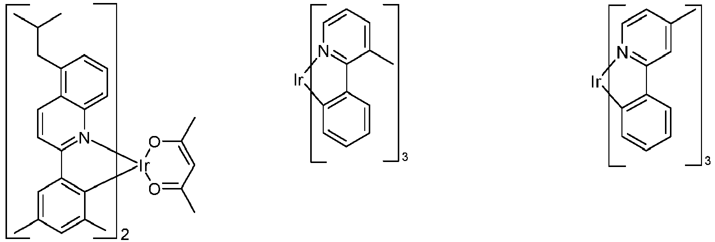

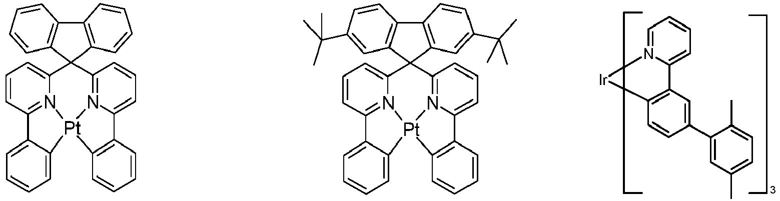

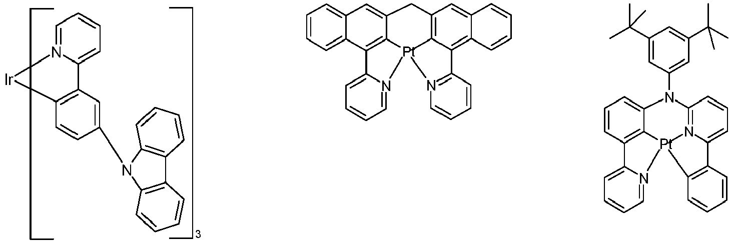

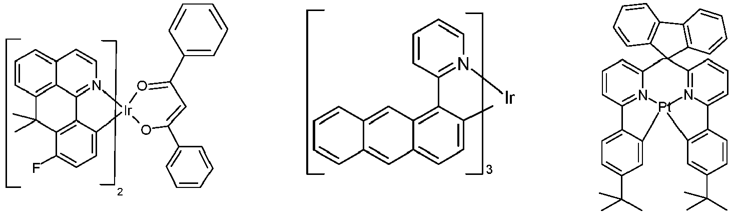

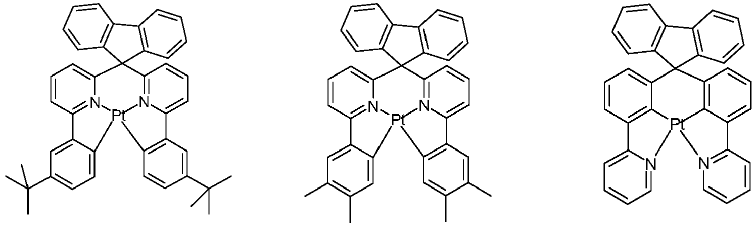

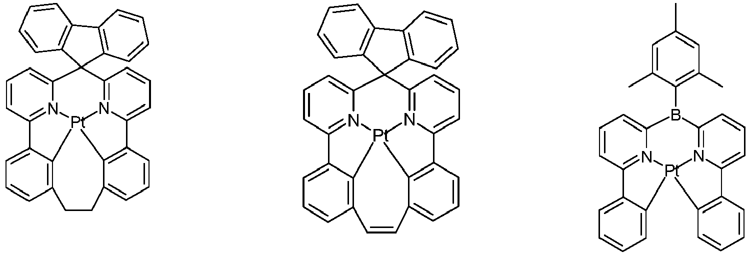

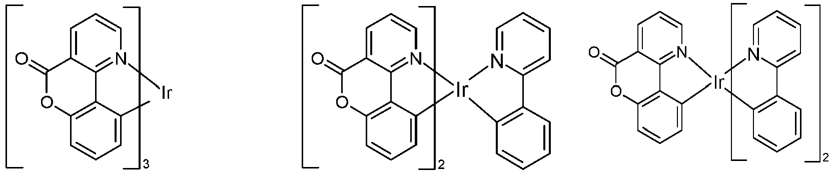

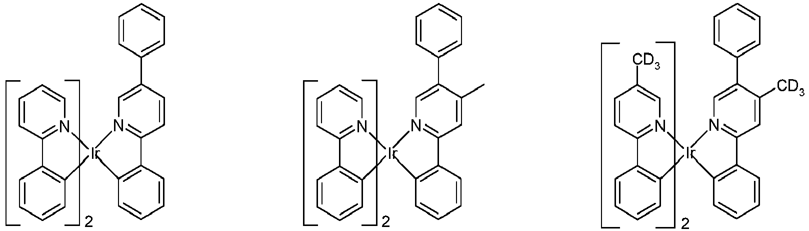

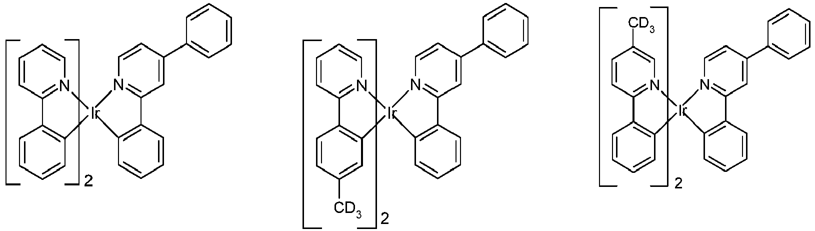

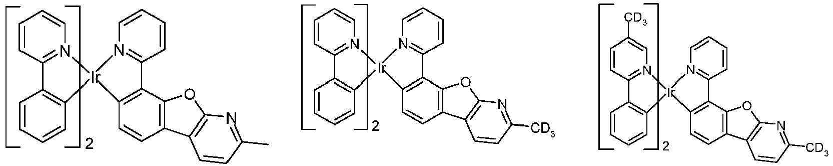

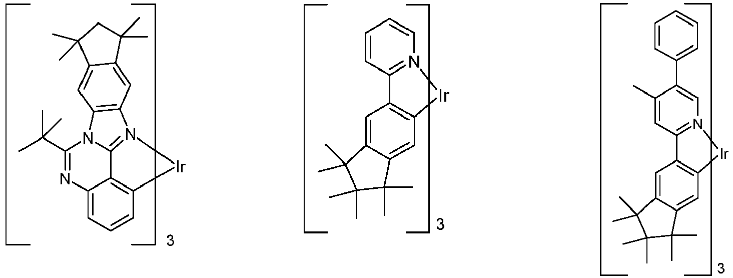

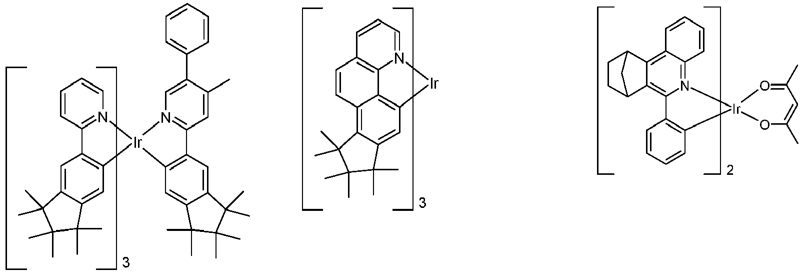

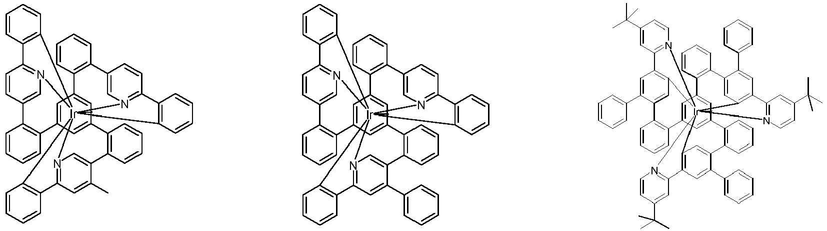

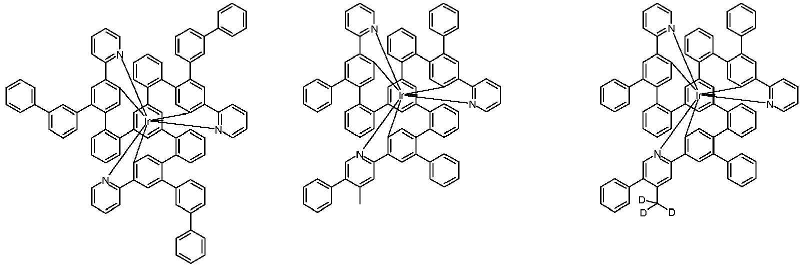

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) 7-Dibenzothiophen-1-yl-imidazo[1,2-f]phenanthridin

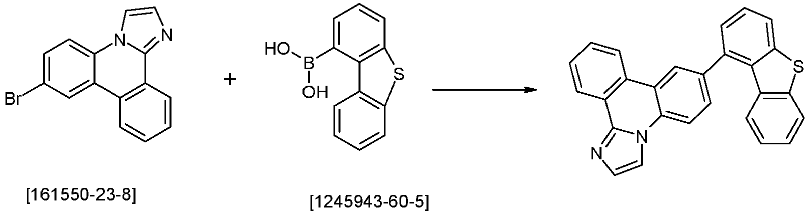

15.9 g (70 mmol) Dibenzothiophen-1-boronsäure, 20.8 g (70 mmol) 7-Brom-imidazo[1,2-f]phenanthridin und 14.7 g (139 mmol) Natriumcarbonat werden in 200 mL Toulol, 52 mL Ethanol und 100 mL Wasser suspendiert. Zu dieser Suspension werden 80 mg (0.69 mmol) Tetrakis-(triphenylphosphin)-palladium(0) gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Heptan / Dichlormethan umkristallisiert. Die Ausbeute beträgt 21.8 g (55 mmol), entsprechend 78 % der Theorie.

Analog können die folgenden Verbindungen erhalten werden:

| | **Edukt 1** | **Edukt 2** | **Produkt** | **Ausbeute** |
|---|---|---|---|---|
| 1a | 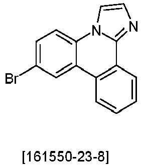 | 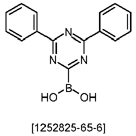 | 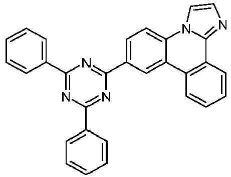 | 60% |
| 2a | 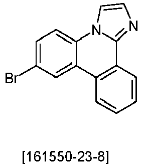 | 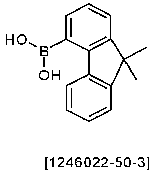 | 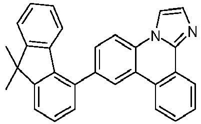 | 64% |
| 3a | 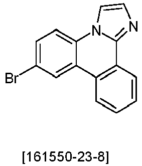 | 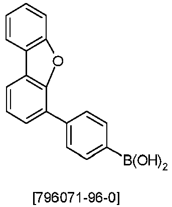 | 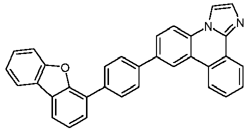 | 73% |
| 4a | 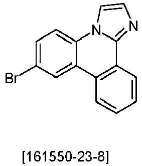 | 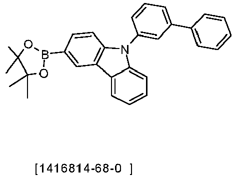 | 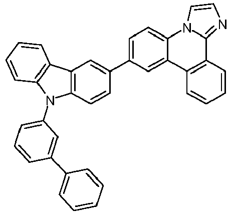 | 76% |
| 5a | 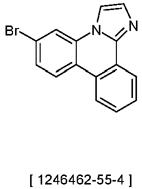 | 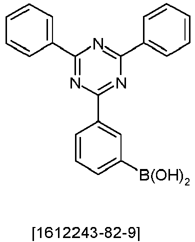 | 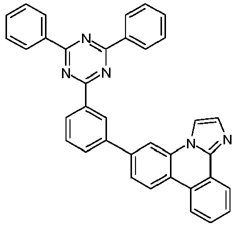 | 74% |
| 6a | 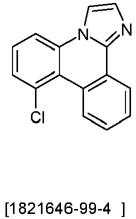 | 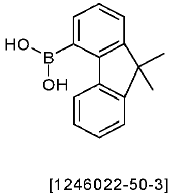 | 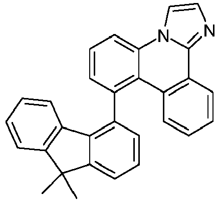 | 77% |
| 7a | 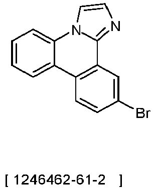 | 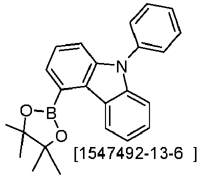 | 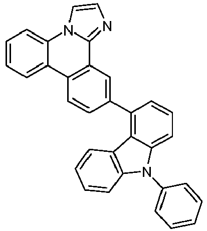 | 67% |
| 8a | 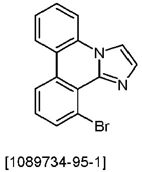 | 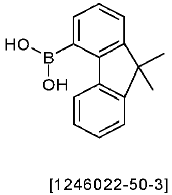 | 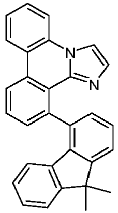 | 66% |

### b) 3-Brom-pyrrolo[1,2-f]phenanthridin

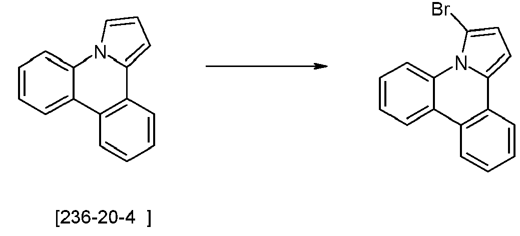

Eine Lösung von 33.4 g (154 mmol) Pyrrolo[1,2-f]phenanthridin in 1000 mL Chloroform wird bei 0 °C unter Lichtausschluss portionsweise mit 24.7 g (139 mmol) *N*-Bromsuccinimid versetzt und 2 h bei dieser Temperatur gerührt. Die Reaktion wird durch Zugabe von Natriumsulfit-Lösung beendet und weitere 30 min bei Raumtemperatur gerührt. Nach Phasentrennung wird die organische Phase mit Wasser gewaschen und die wässrige Phase mit Dichlormethan extrahiert. Die vereinigten organischen Phasen werden über Natriumsulfat getrocknet und im Vakuum eingeengt. Der Rückstand wird in Toluol gelöst und über Kieselgel filtriert. Anschließend wird das Rohprodukt aus Toluol/Heptan umkristallisiert. Ausbeute: 26 g (88 mmol), 58 % d. Th., farbloser Feststoff.

Analog können die folgenden Verbindungen erhalten werden:

| | **Edukt 1** | **Produkt** | **Ausbeute** |
|---|---|---|---|
| 1b | 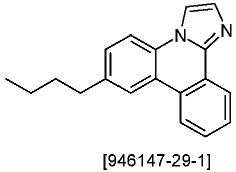 | 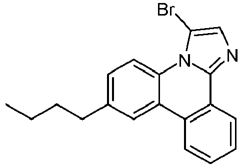 | 60% |
| 2b | 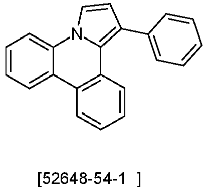 | 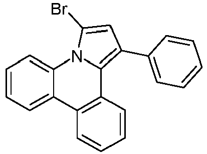 | 61% |
| 3b | 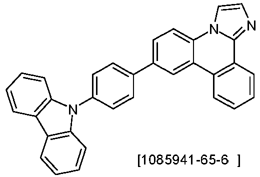 | 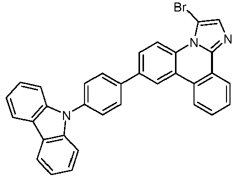 | 57% |
| 4b | 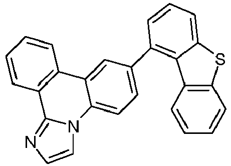 | 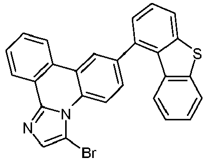 | 59% |
| 5b | 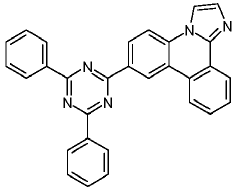 | 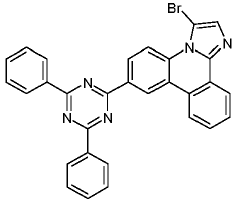 | 61% |
| 6b | 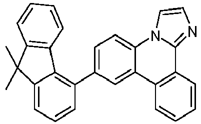 | 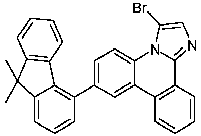 | 58% |
| 7b | 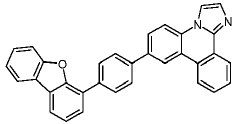 | 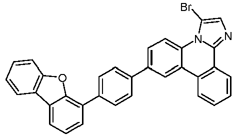 | 49% |
| 8b | 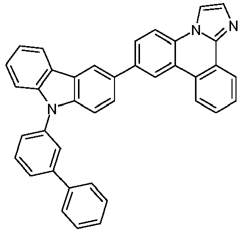 | 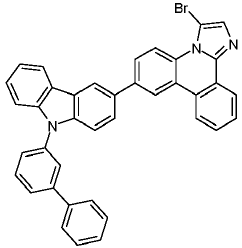 | 51% |
| 9b | 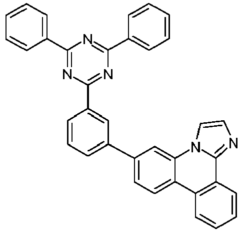 | 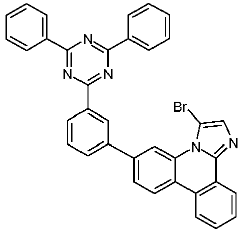 | 62% |
| 10b | 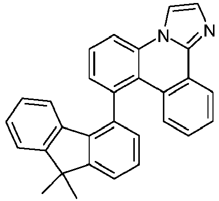 | 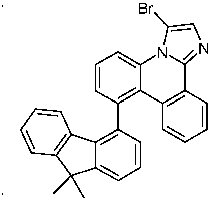 | 65% |
| 11b | 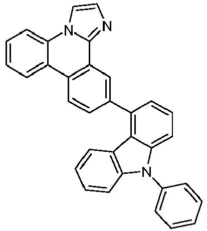 | 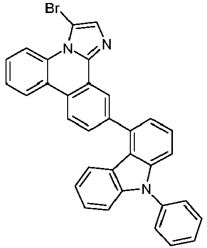 | 51% |
| 12b | 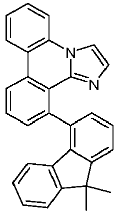 | 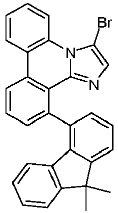 | 47% |

### c) (2-Chlorphenyl)-imidazo[1,2-f]phenanthridin-3-yl-amin

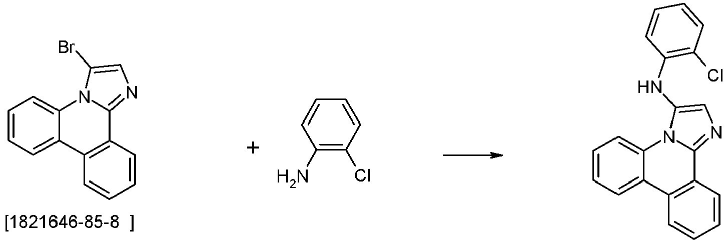

40.9 g (138 mmol) 3-Brom-imidazo[1,2-f]phenanthridin, 17.9 g (140 mmol) 2-Chloranilin, 68.2 g (710 mmol) Natrium-tert-butylat, 613 mg (3 mmol) Palladium(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 36 g (106 mmol), 79 % d.Th.

Analog können die folgenden Verbindungen erhalten werden:

| | **Edukt 1** | **Edukt 2** | **Produkt** | **Ausbeute** |
|---|---|---|---|---|
| 1c | 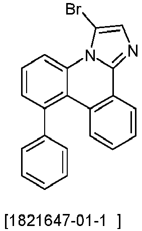 | 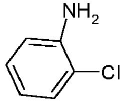 | 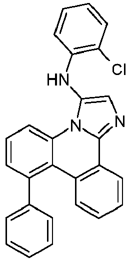 | 83% |
| 2c | 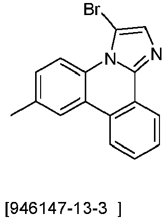 | 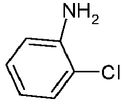 | 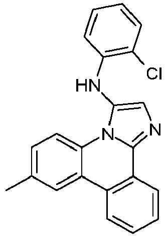 | 75% |
| 3c | 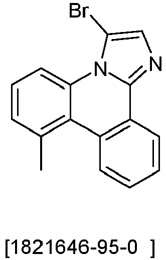 | 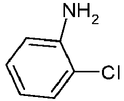 | 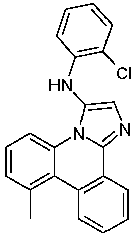 | 81% |
| 4c | 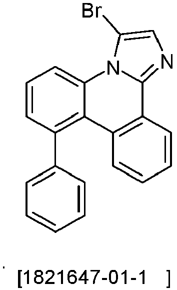 | 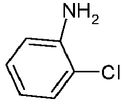 | 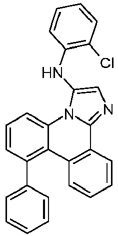 | 79% |
| 5c | 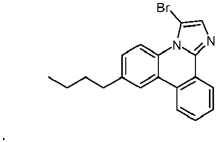 | 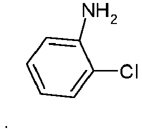 | 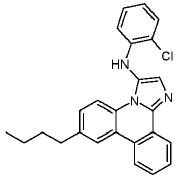 | 74% |
| 6c | 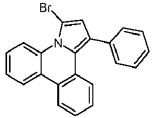 | 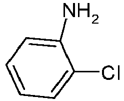 | 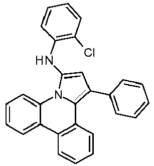 | 79% |
| 7c | 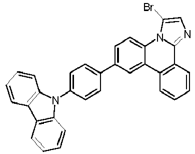 | 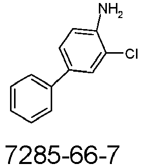 | 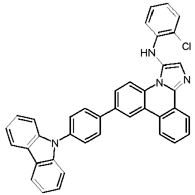 | 87% |
| 8c | 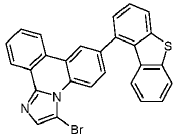 | 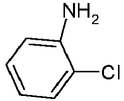 | 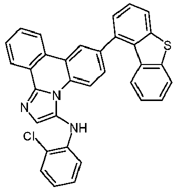 | 76% |
| 9c | 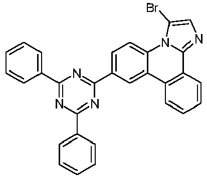 | 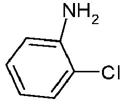 | 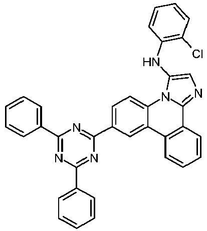 | 75% |
| 10c | 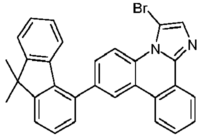 | 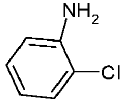 | 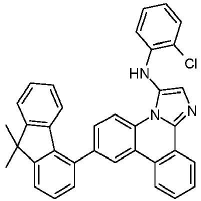 | 78% |
| 11c | 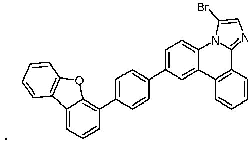 | 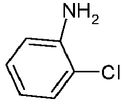 | 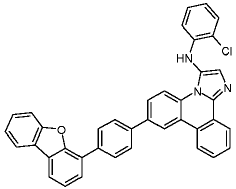 | 81% |
| 12c | 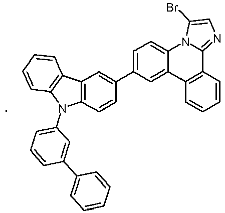 | 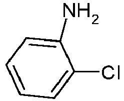 | 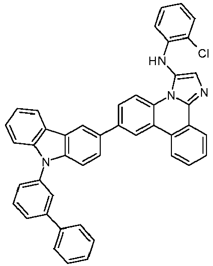 | 80% |
| 13c | 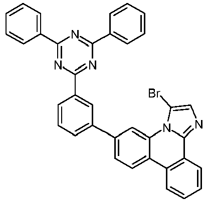 | 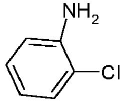 | 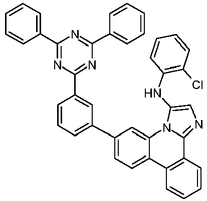 | 84% |
| 14c | 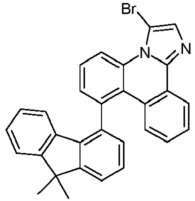 | 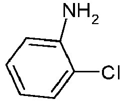 | 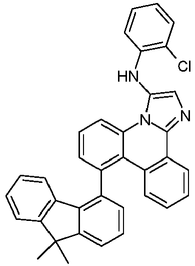 | 73% |
| 15c | 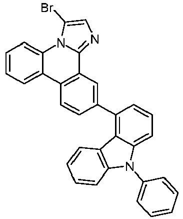 | 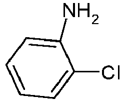 | 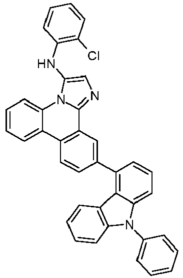 | 81% |
| 16c | 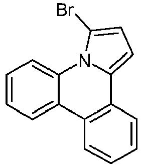 | 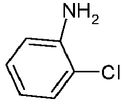 | 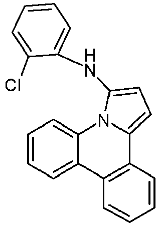 | 84% |
| 17c | 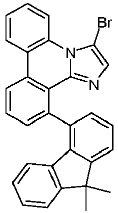 | 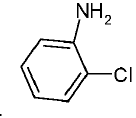 | 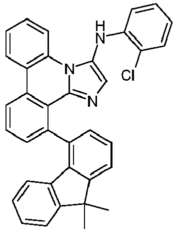 | 67% |

### d) Cyclisierung

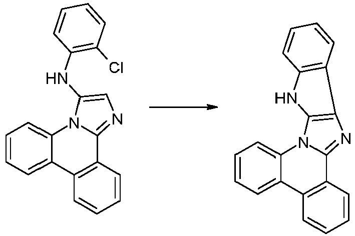

33 g (102 mmol) (2-Chlorphenyl)-imidazo[1,2-f]phenanthridin-3-yl-amin, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten wird die Reaktionmischung mit 300 ml Wasser und 400 mL Essigsäureethylester versetzt. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert (22 g, 71 mmol), 72 % d.Th..

Analog können die folgenden Verbindungen erhalten werden:

| | **Edukt** | **Produkt** | **Ausbeute** |
|---|---|---|---|
| 1d | 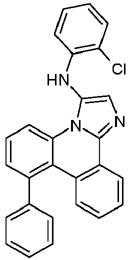 | 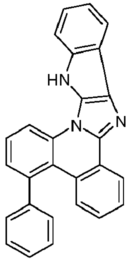 | 69% |
| 2d | 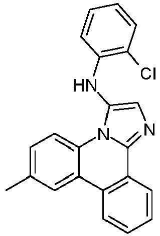 | 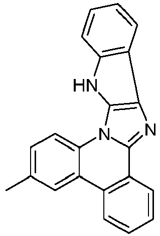 | 71% |
| 3d | 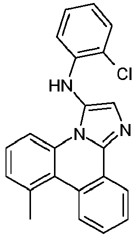 | 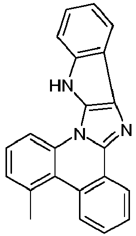 | 76% |
| 4d | 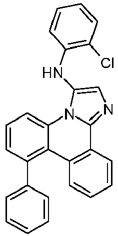 | 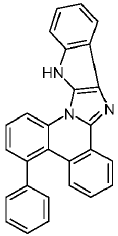 | 74% |
| 5d | 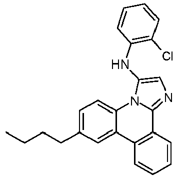 | 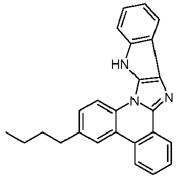 | 68% |
| 6d | 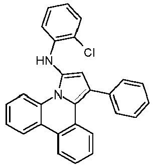 | 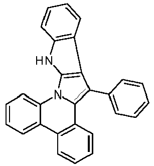 | 79% |
| 7d | 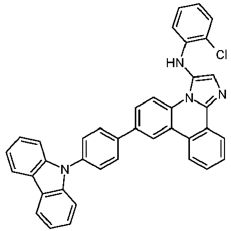 | 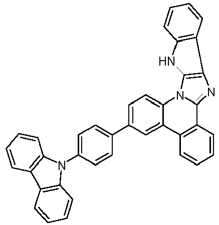 | 72% |
| 8d | 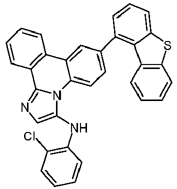 | 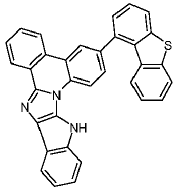 | 76% |
| 9d | 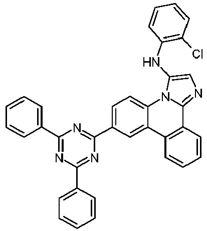 | 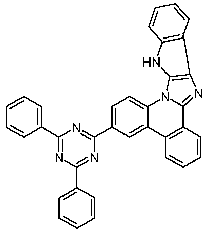 | 78% |
| 10d | 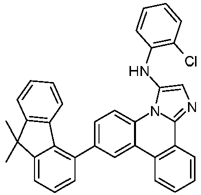 | 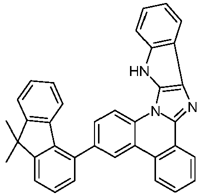 | 71% |
| 11d | 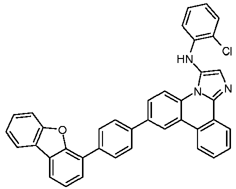 | 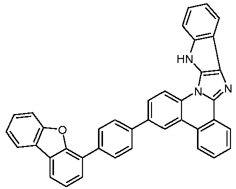 | 77% |
| 12d | 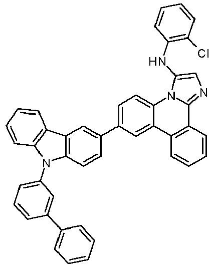 | 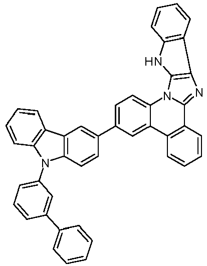 | 75% |
| 13d | 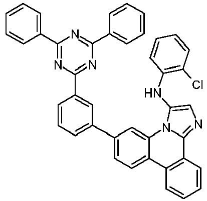 | 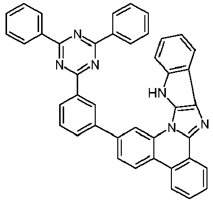 | 73% |
| 14d | 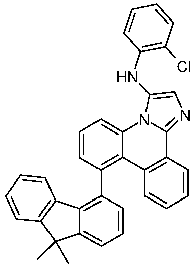 | 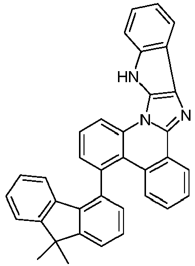 | 79% |
| 15d | 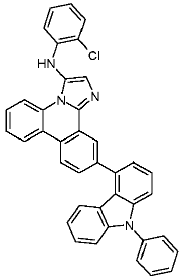 | 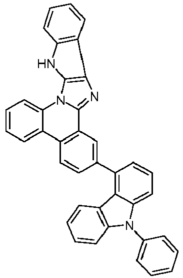 | 64% |
| 16d | 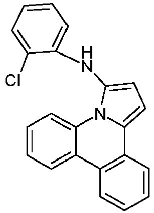 | 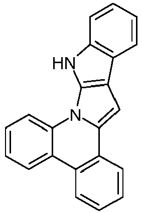 | 63% |
| 17d | 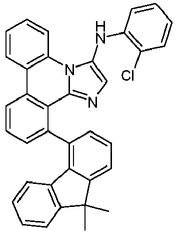 | 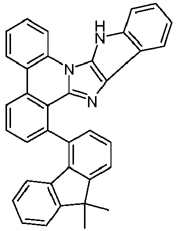 | 60% |

### e) 3-(2-Nitrophenyl)-imidazo[1,2-f]phenanthridin

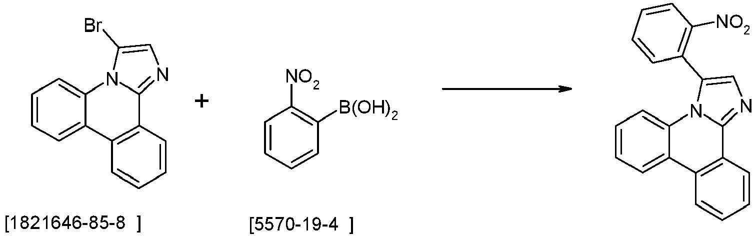

Eine gut gerührte, entgaste Suspension aus 30 g (183.8 mmol) 2-Nitrophenylboronsäure, 54 g (184 mmol) 3-Brom-imidazo[1,2-f]phenanthridin und 66.5 g (212.7 mmol) Kaliumcarbonat in einem Gemisch aus 250 ml Wasser und 250 ml THF wird mit 1.7 g (1.49 mmol) Pd(PPh₃)₄ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet. Ausbeute: 55 g (162 mmol), 90 % d. Th..

Analog können die folgenden Verbindungen erhalten werden:

| | **Edukt 1** | **Edukt 2** | **Produkt** | **Ausbeute** |
|---|---|---|---|---|
| 1e | 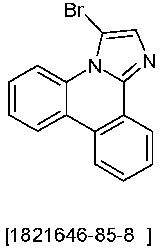 | 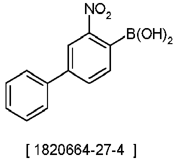 | 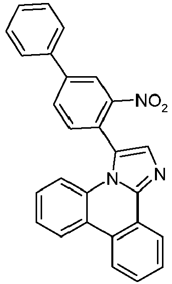 | 83% |
| 2e | 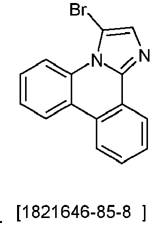 | 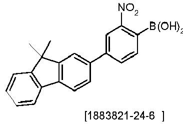 | 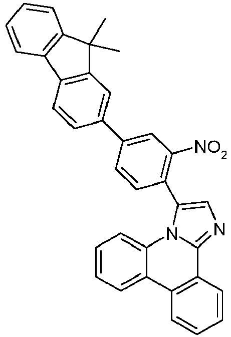 | 87% |
| 3e | 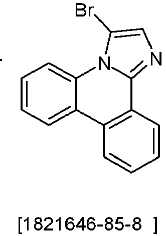 | 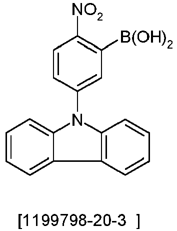 | 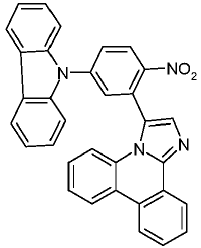 | 84% |
| 4e | 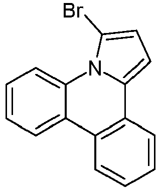 | 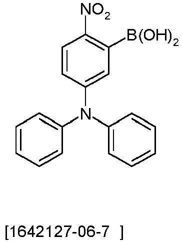 | 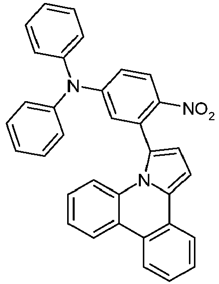 | 80% |

### f) Carbazolsynthese

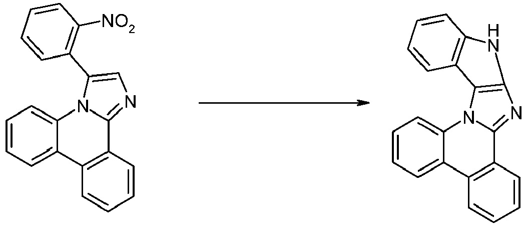

Eine Mischung aus 81.3 g (240 mmol) 3-(2-Nitrophenyl)-imidazo[1,2-f]-phenanthridin und 290.3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert. Ausbeute: 55 g (179 mmol), 75 % d. Th..

Analog können die folgenden Verbindungen erhalten werden:

| | **Edukt 1** | **Produkt** | **Ausbeute** |
|---|---|---|---|
| 1f | 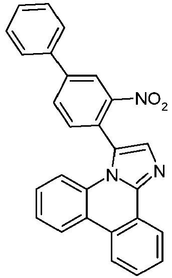 | 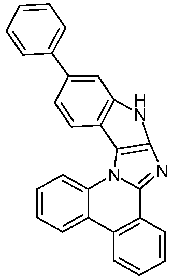 | 78% |
| 2f | 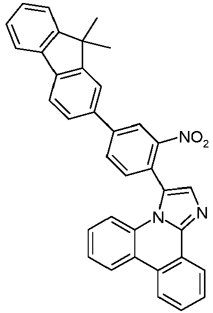 | 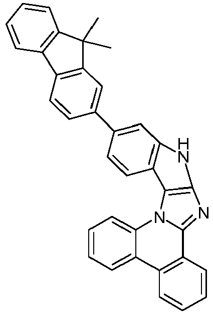 | 72% |
| 3f | 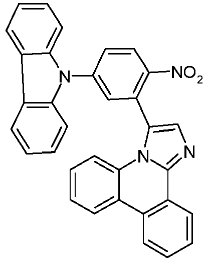 | 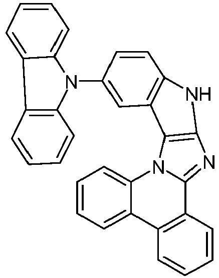 | 81% |
| 4f | 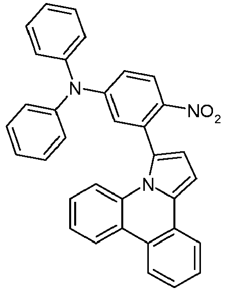 | 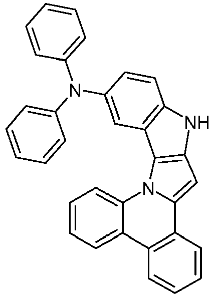 | 74% |

### g) Nukleophile Substitution

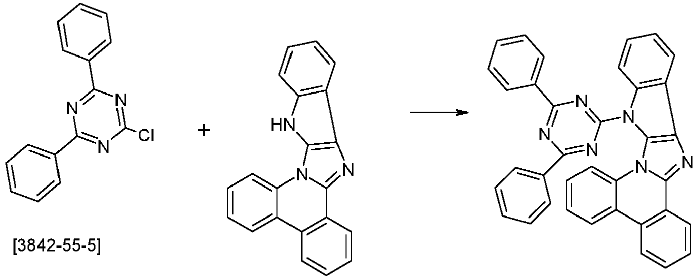

4.2 g (106 mmol) NaH, 60%ig in Mineralöl, werden in 300 mL Dimethylformamid unter Schutzatmosphäre gelöst. 32 g (106 mmol) der Verbindung d werden in 250 mL DMF gelöst und zu der Reaktionsmischung zugetropft. Nach 1 h bei Raumtemperatur wird eine Lösung von 2-Chlor-4,6-diphenyl-[1,3,5]triazin (34.5 g, 0.122 mol) in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol / n-Heptan umkristallisiert und abschließend im Hochvakuum sublimiert. Die Ausbeute beträgt 35 g (66 mmol), 64 % d. Th.; Reinheit 99.9 %.

Analog können die folgenden Verbindungen erhalten werden:

| | Edukt 1 | Edukt 2 | Produkt | Ausbeute |
|---|---|---|---|---|
| 1g | 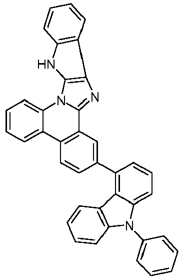 | 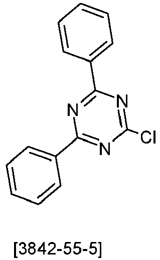 | 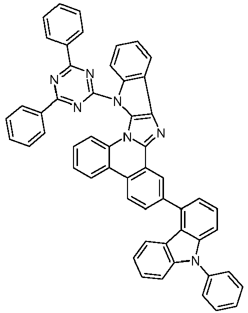 | 63% |
| 2g | 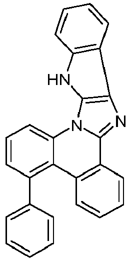 | 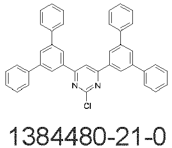 | 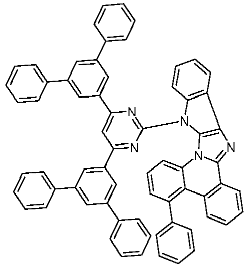 | 62% |
| 3g | 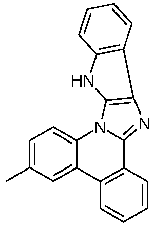 | 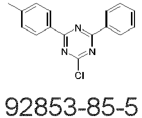 | 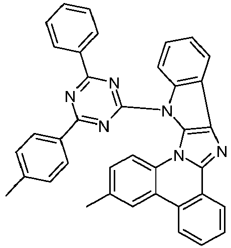 | 59% |
| 4g | 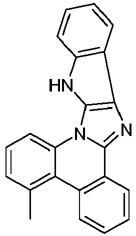 | 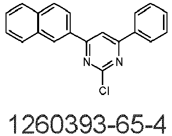 | 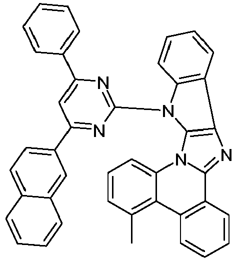 | 53% |
| 5g | 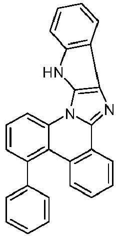 | 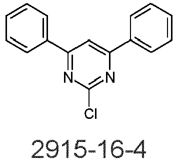 | 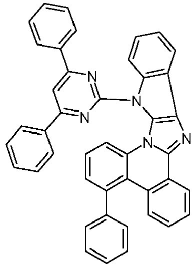 | 60% |
| 6g | 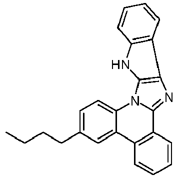 | 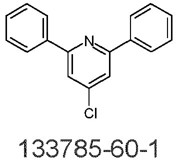 | 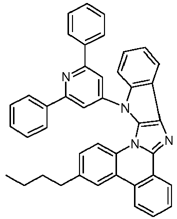 | 57% |
| 7g | 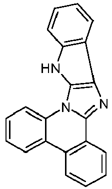 | 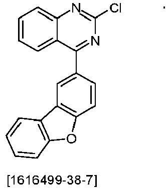 | 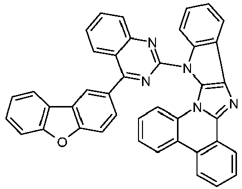 | 62% |
| 8g | 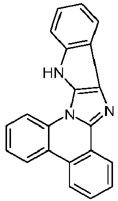 | 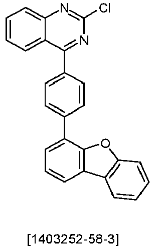 | 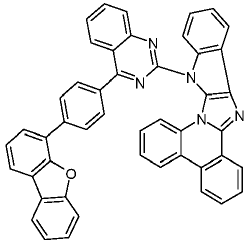 | 64% |
| 9g | 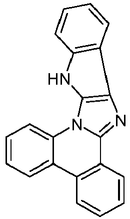 | 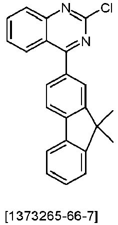 | 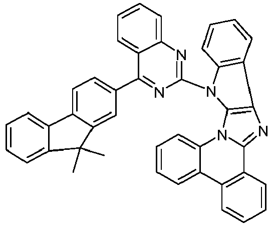 | 65% |
| 10g | 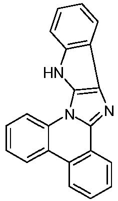 | 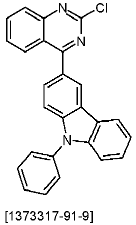 | 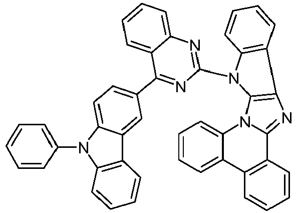 | 67% |
| 11g | 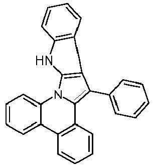 | 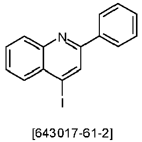 | 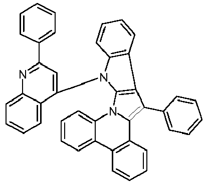 | 65% |
| 12g | 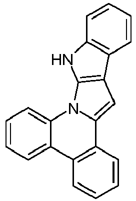 | 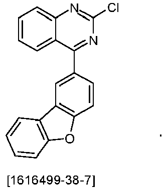 | 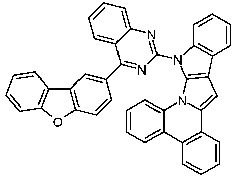 | |
| 13g | 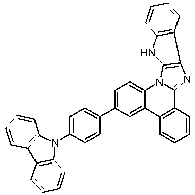 | 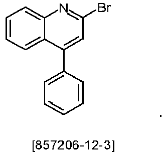 | 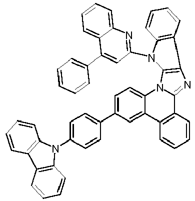 | 63% |
| 14g | 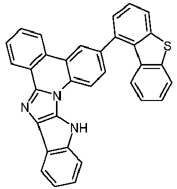 | 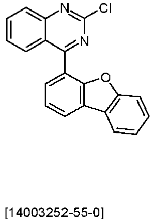 | 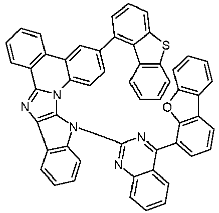 | 67% |
| 15g | 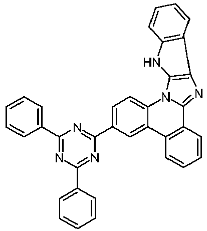 | 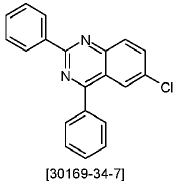 | 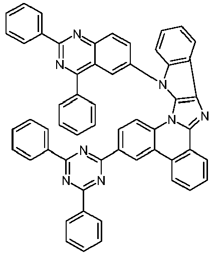 | 66% |
| 16g | 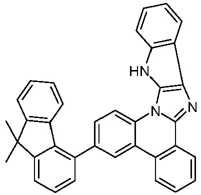 | 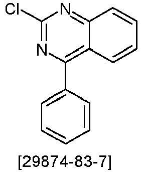 | 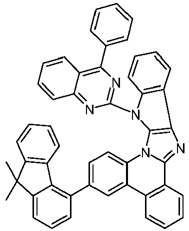 | 61% |
| 17g | 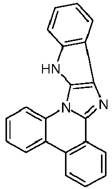 | 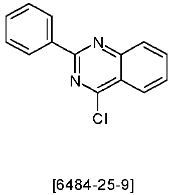 | 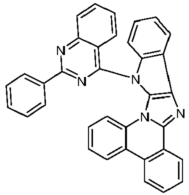 | 62% |
| 18e | 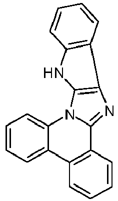 | 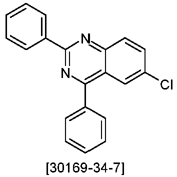 | 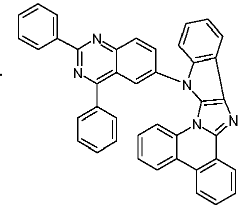 | 60% |
| 19g | 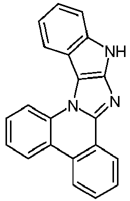 | 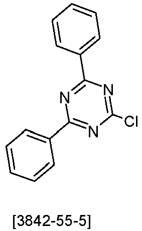 | 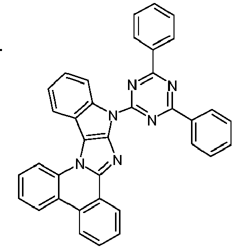 | 64% |
| 20g | 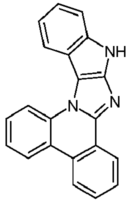 | 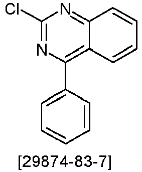 | 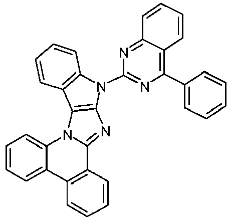 | 67% |
| 21g | 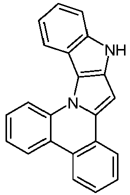 | 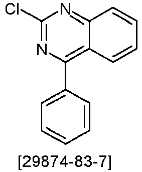 | 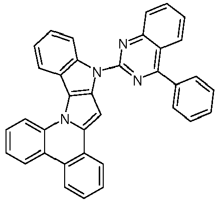 | 70% |
| 22g | 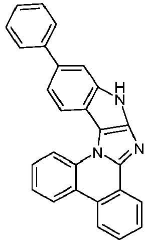 | 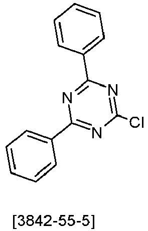 | 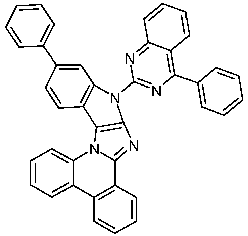 | 68% |
| 23g | 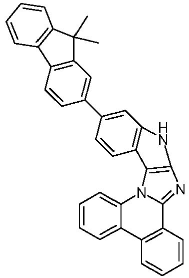 | 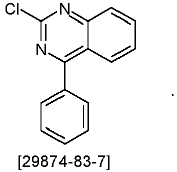 | 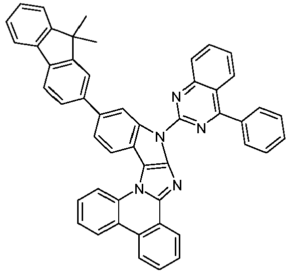 | 66% |
| 24g | 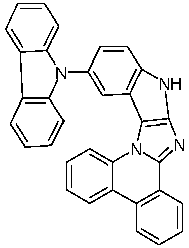 | 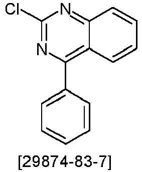 | 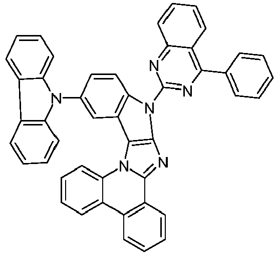 | 71% |
| 25g | 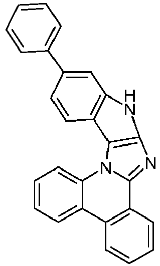 | 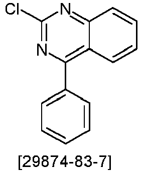 | 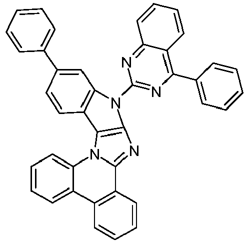 | 64% |
| 26g | 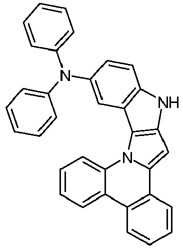 | 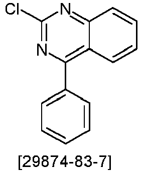 | 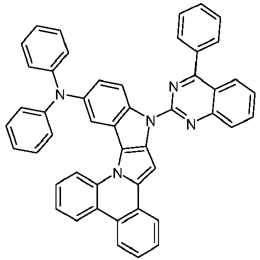 | 65% |
| 27g | 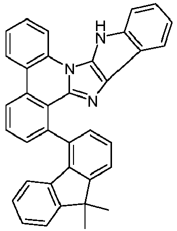 | 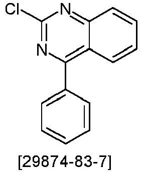 | 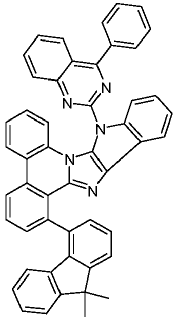 | 61% |

### h) Buchwald-Kupplung

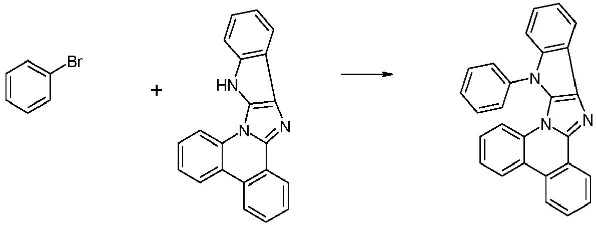

14.8 g (50 mmol) Verbindung d und 8.4 g (54 mmol) Brombenzol werden in 400 mL Toluol unter Argonatmosphäre gelöst. 1.0 g (5 mmol) Tri-tert-butylphosphin werden zugegeben und unter Argonatmosphäre gerührt. 0.6 g (2 mmol) Pd(OAc)₂ werden zugegeben und unter Argonatmosphäre gerührt, wonach 9.5 g (99 mmol) Natium-tert-butanolat zugegeben werden. Das Reaktionsgemisch wird 24 h unter Rückfluss gerührt. Nach dem Abkühlen wird die organische Phase abgetrennt, dreimal mit 200 ml Wasser gewaschen, über MgSO₄ getrocknet, filtriert und das Lösungsmittel im Vakuum entfernt. Der Rückstand wird durch Säulenchromatographie über Kieselgel (Eluent: DCM/Heptan (1:3)) gereinigt. Der Rückstand wird mit Toluol heiß extrahiert, aus Toluol / n-Heptan umkristallisiert und abschließend im Hochvakuum sublimiert. Die Ausbeute beträgt 19.4 g (50 mmol), 95 % der Theorie.

Analog können die folgenden Verbindungen erhalten werden:

| | **Edukt 1** | **Edukt 2** | **Produkt** | **Ausbeute** |
|---|---|---|---|---|
| 1h | 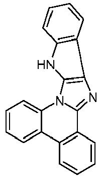 | 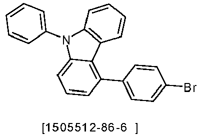 | 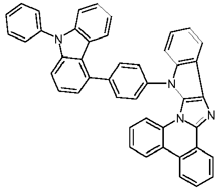 | 70% |
| 2h | 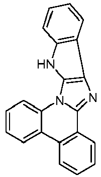 | 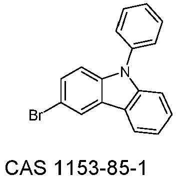 | 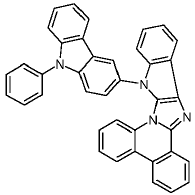 | 73% |
| 3h | 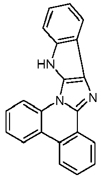 | 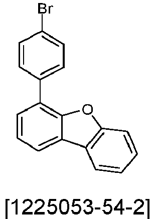 | 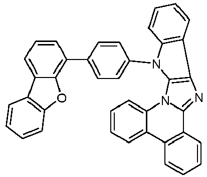 | 75% |
| 4h | 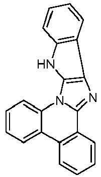 | 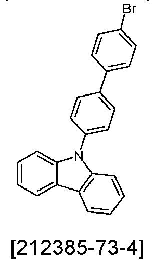 | 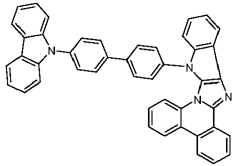 | 76% |
| 5h | 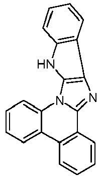 | 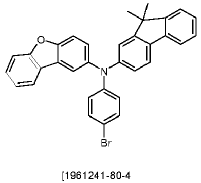 | 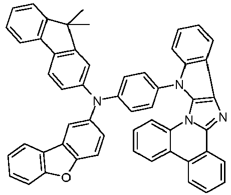 | 78% |
| 6h | 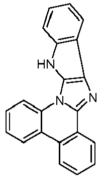 | 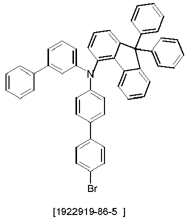 | 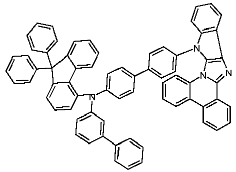 | 70% |
| 7h | 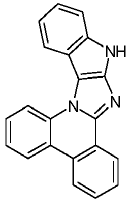 | 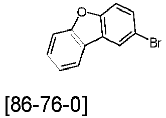 | 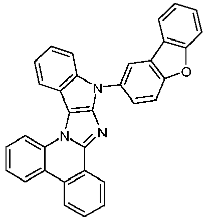 | 73% |
| 8h | 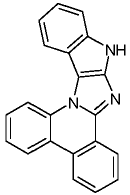 | 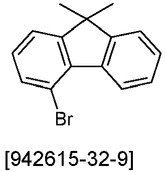 | 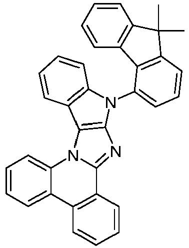 | 75% |
| 9h | 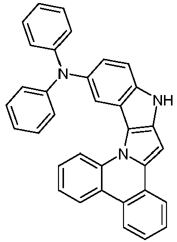 | 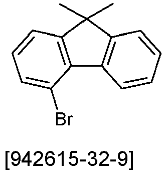 | 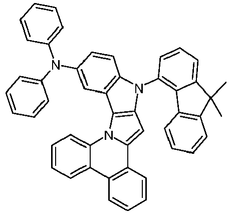 | 77% |

### Herstellung der OLEDs

In den folgenden Beispielen E1 bis E43 (siehe Tabelle 1) wird der Einsatz der erfindungsgemäßen Materialien in OLEDs vorgestellt.

**Vorbehandlung für die Beispiele E1-E43:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TER1 (50%:45%:5%) bedeutet hierbei, dass das Material IC1 in einem Volumenanteil von 50%, IC2 in einem Volumenanteil von 45% und TER1 in einem Volumenanteil von 5% in der Schicht vorliegt. Analog kann auch die Elektronentransportschicht aus einer Mischung von zwei Materialien bestehen.

Die OLEDs werden standardmäßig charakterisiert. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Mischungen in OLEDs

Die erfindungsgemäßen Materialien können in der Emissionsschicht in phosphoreszierenden OLEDs eingesetzt werden. Die erfindungsgemäßen Verbindungen EG1 bis EG33 werden in den Beispielen E1 bis E43 als Matrixmaterial für rot phosphoreszierende Emitter in der Emissionsschicht eingesetzt. Die Farbkoordinaten der Elektrolumineszenzspektren der OLEDs liegen bei CIEx=0.67 und CIEy= 0.33. Somit eignen sich die Materialien für den Einsatz in der Emissionsschicht von roten OLEDs. Des Weiteren lassen sich die erfindungsgemäßen Materialien in der Lochblockierschicht (HBL) oder Elektronenblockierschicht (EBL) erfolgreich einsetzen. Dies ist in den Beispielen E26 bzw. E35-E43 gezeigt. Auch hier liegen die Farbkoordinaten des Spektrums der OLED jeweils bei CIEx=0.67 und CIEy= 0.33.

**Tabelle 1: Aufbau der OLEDs**

| Bsp. | HIL Dicke | HTL Dicke | EBL Dicke | EML Dicke | HBL Dicke | ETL Dicke |
|---|---|---|---|---|---|---|
| E1 | HATCN 5nm | SpMA1 125nm | SpMA2 10nm | EG1:TER1 (95%:5%) 40nm | --- | ST1:LiQ (50%:50%) 35nm |
| E2 | HATCN 5nm | SpMA1 125nm | SpMA2 10nm | EG2:TER1 (95%:5%) 40nm | --- | ST1:LiQ (50%:50%) 35nm |
| E3 | HATCN 5nm | SpMA1 125nm | SpMA2 10nm | EG3:TER1 (95%:5%) 40nm | --- | ST1:LiQ (50%:50%) 35nm |
| E4 | HATCN 5nm | SpMA1 125nm | SpMA2 10nm | EG4:TER1 (95%:5%) 40nm | --- | ST1:LiQ (50%:50%) 35nm |
| E5 | HATCN 5nm | SpMA1 125nm | SpMA2 10nm | EG5:TER1 (95%:5%) 40nm | --- | ST1:LiQ (50%:50%) 35nm |
| E6 | HATCN 5nm | SpMA1 125nm | SpMA2 10nm | EG6:TER1 (95%:5%) 40nm | --- | ST1:LiQ (50%:50%) 35nm |
| E7 | HATCN 5nm | SpMA1 125nm | SpMA2 10nm | EG7:TER1 (95%:5%) 40nm | --- | ST1:LiQ (50%:50%) 35nm |
| E8 | HATCN 5nm | SpMA1 125nm | SpMA2 10nm | EG8:TER1 (95%:5%) 40nm | --- | ST1:LiQ (50%:50%) 35nm |
| E9 | HATCN 5nm | SpMA1 125nm | SpMA2 10nm | EG9:TER1 (95%:5%) 40nm | --- | ST1:LiQ (50%:50%) 30nm |
| E10 | HATCN 5nm | SpMA1 125nm | SpMA2 10nm | EG10:TER1 (95%:5%) 40nm | --- | ST1:LiQ (50%:50%) 35nm |
| E11 | HATCN 5nm | SpMA1 125nm | SpMA2 10nm | EG11:TER1 (95%:5%) 40nm | --- | ST1:LiQ (50%:50%) 35nm |
| E12 | HATCN 5nm | SpMA1 125nm | SpMA2 10nm | EG12:TER1 (95%:5%) 40nm | --- | ST1:LiQ (50%:50%) 35nm |
| E13 | HATCN 5nm | SpMA1 125nm | SpMA2 10nm | EG13:TER1 (95%:5%) 40nm | --- | ST1:LiQ (50%:50%) 35nm |
| E14 | HATCN 5nm | SpMA1 125nm | SpMA2 10nm | EG14:TER1 (95%:5%) 40nm | --- | ST1:LiQ (50%:50%) 35nm |
| E15 | HATCN 5nm | SpMA1 125nm | SpMA2 10nm | EG15:TER1 (95%:5%) 40nm | --- | ST1:LiQ (50%:50%) 35nm |
| E16 | HATCN 5nm | SpMA1 125nm | SpMA2 10nm | EG16:TER1 (95%:5%) 40nm | --- | ST1:LiQ (50%:50%) 35nm |
| E17 | HATCN 5nm | SpMA1 125nm | SpMA2 10nm | EG17:TER1 (95%:5%) 40nm | --- | ST1:LiQ (50%:50%) 35nm |
| E18 | HATCN 5nm | SpMA1 125nm | SpMA2 10nm | EG18:TER1 (95%:5%) 40nm | --- | ST1:LiQ (50%:50%) 35nm |
| E19 | HATCN 5nm | SpMA1 125nm | SpMA2 10nm | EG19:TER1 (95%:5%) 40nm | --- | ST1:LiQ (50%:50%) 35nm |
| E20 | HATCN 5nm | SpMA1 125nm | SpMA2 10nm | EG20:TER1 (95%:5%) 40nm | --- | ST1:LiQ (50%:50%) 35nm |
| E21 | HATCN 5nm | SpMA1 125nm | SpMA2 10nm | EG21:TER1 (95%:5%) 40nm | --- | ST1:LiQ (50%:50%) 35nm |
| E22 | HATCN 5nm | SpMA1 125nm | SpMA2 10nm | EG22:TER1 (95%:5%) 40nm | --- | ST1:LiQ (50%:50%) 35nm |
| E23 | HATCN 5nm | SpMA1 125nm | SpMA2 10nm | EG23:TER1 (95%:5%) 40nm | --- | ST1:LiQ (50%:50%) 35nm |
| E24 | HATCN 5nm | SpMA1 125nm | SpMA2 10nm | EG24:TER1 (95%:5%) 40nm | --- | ST1:LiQ (50%:50%) 35nm |
| E25 | HATCN 5nm | SpMA1 125nm | SpMA2 10nm | IC2:EG24:TER1 (50%:45%:5%) 40nm | --- | ST1:LiQ (50%:50%) 35nm |
| E26 | HATCN 5nm | SpMA1 125nm | SpMA2 10nm | EG24:TER1 (95%:5%) 40nm | EG24 5nm | ST1:LiQ (50%:50%) 35nm |
| E27 | HATCN 5nm | SpMA1 125nm | SpMA2 10nm | IC1:EG25:TER1 (50%:45%:5%) 40nm | --- | ST1:LiQ (50%:50%) 35nm |
| E28 | HATCN 5nm | SpMA1 125nm | SpMA2 10nm | IC1:EG26:TER1 (50%:45%:5%) 40nm | --- | ST1:LiQ (50%:50%) 35nm |
| E29 | HATCN 5nm | SpMA1 125nm | SpMA2 10nm | IC1:EG27:TER1 (50%:45%:5%) 40nm | --- | ST1:LiQ (50%:50%) 35nm |
| E30 | HATCN 5nm | SpMA1 125nm | SpMA2 10nm | IC1:EG28:TER1 (50%:45%:5%) 40nm | --- | ST1:LiQ (50%:50%) 35nm |
| E31 | HATCN 5nm | SpMA1 125nm | SpMA2 10nm | IC1:EG29:TER1 (50%:45%:5%) 40nm | --- | ST1:LiQ (50%:50%) 35nm |
| E32 | HATCN 5nm | SpMA1 125nm | SpMA2 10nm | IC1:EG30:TER1 (50%:45%:5%) 40nm | --- | ST1:LiQ (50%:50%) 35nm |
| E33 | HATCN 5nm | SpMA1 125nm | SpMA2 10nm | IC1:EG31:TER1 (50%:45%:5%) 40nm | --- | ST1:LiQ (50%:50%) 35nm |
| E34 | HATCN 5nm | SpMA1 125nm | SpMA2 10nm | IC1:EG32:TER1 (50%:45%:5%) 40nm | --- | ST1:LiQ (50%:50%) 35nm |
| E34 | HATCN 5nm | SpMA1 125nm | SpMA2 10nm | IC1:EG33:TER1 (50%:45%:5%) 40nm | --- | ST1:LiQ (50%:50%) 35nm |
| E35 | HATCN 5nm | SpMA1 125nm | EG26 10nm | IC1:EG26:TER1 (50%:45%:5%) 40nm | --- | ST1:LiQ (50%:50%) 35nm |
| E36 | HATCN 5nm | SpMA1 125nm | EG27 10nm | IC1:EG27:TER1 (50%:45%:5%) 40nm | --- | ST1:LiQ (50%:50%) 35nm |
| E37 | HATCN 5nm | SpMA1 125nm | EG28 10nm | IC1:EG28:TER1 (50%:45%:5%) 40nm | --- | ST1:LiQ (50%:50%) 35nm |
| E38 | HATCN 5nm | SpMA1 125nm | EG29 10nm | IC1:EG29:TER1 (50%:45%:5%) 40nm | --- | ST1:LiQ (50%:50%) 35nm |
| E39 | HATCN 5nm | SpMA1 125nm | EG30 10nm | IC1:EG30:TER1 (50%:45%:5%) 40nm | --- | ST1:LiQ (50%:50%) 35nm |
| E40 | HATCN 5nm | SpMA1 125nm | EG31 10nm | IC1:EG31:TER1 (50%:45%:5%) 40nm | --- | ST1:LiQ (50%:50%) 35nm |
| E42 | HATCN 5nm | SpMA1 125nm | EG32 10nm | IC1:EG32:TER1 (50%:45%:5%) 40nm | --- | ST1:LiQ (50%:50%) 35nm |
| E43 | HATCN 5nm | SpMA1 125nm | EG33 10nm | IC1:EG33:TER1 (50%:45%:5%) 40nm | --- | ST1:LiQ (50%:50%) 35nm |

**Tabelle 2: Strukturformeln der Materialien für die OLEDs**

| | |
|---|---|
| 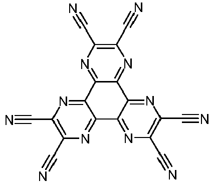 | 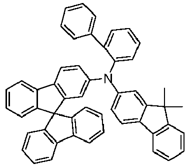 |
| HATCN | SpMA1 |
| 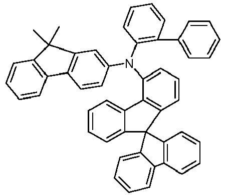 | 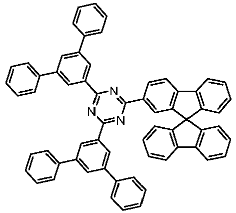 |
| SpMA2 | ST1 |
| 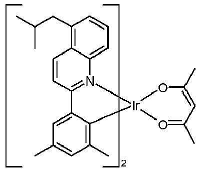 | 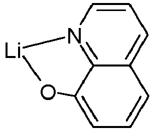 |
| TER1 | LiQ |
| 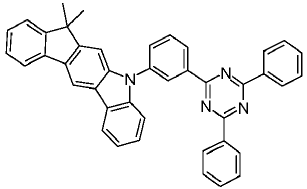 | 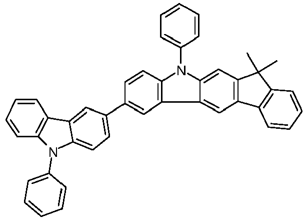 |
| IC1 | IC2 |
| 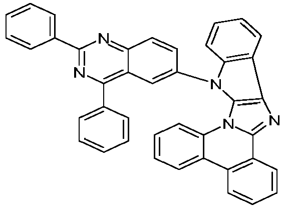 | 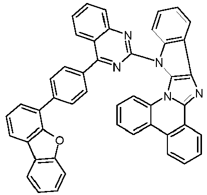 |
| EG1 | EG2 |
| 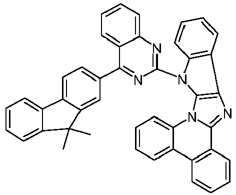 | 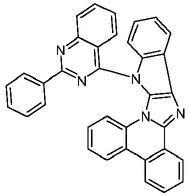 |
| EG3 | EG4 |
| 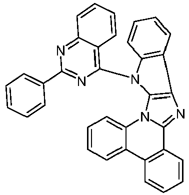 | 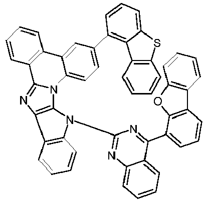 |
| EG5 | EG6 |
| 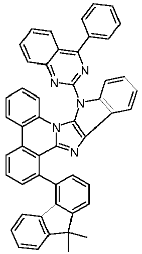 | 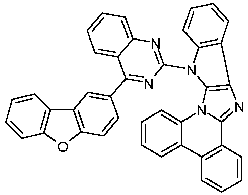 |
| EG7 | EG8 |
| 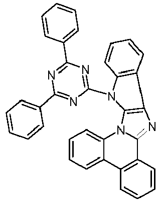 | 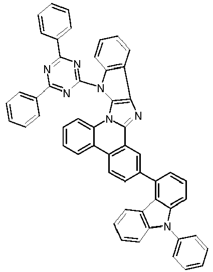 |
| EG9 | EG10 |
| 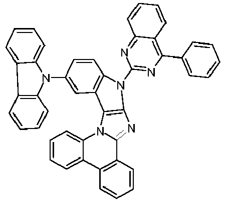 | 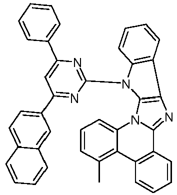 |
| EG11 | EG12 |
| 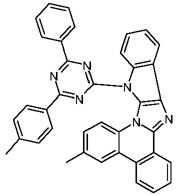 | 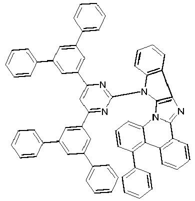 |
| EG13 | EG14 |
| 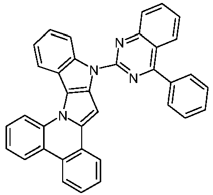 | 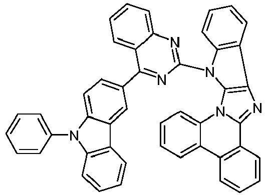 |
| EG15 | EG16 |
| 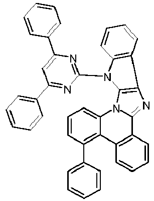 | 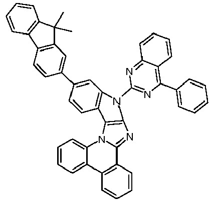 |
| EG17 | EG18 |
| 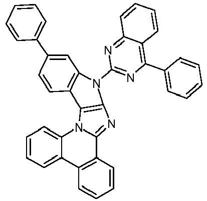 | 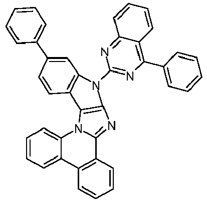 |
| EG19 | EG20 |
| 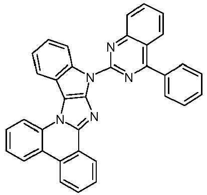 | 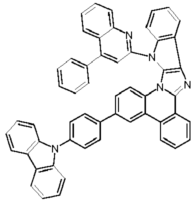 |
| EG21 | EG22 |
| 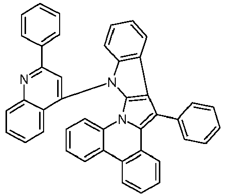 | 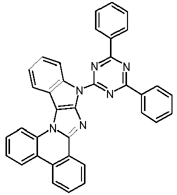 |
| EG23 | EG24 |
| 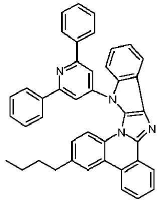 | 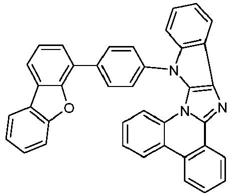 |
| EG25 | EG26 |
| 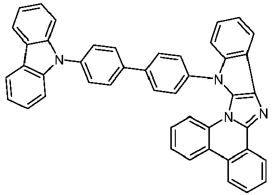 | 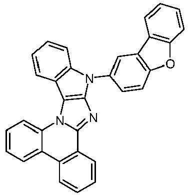 |
| EG27 | EG28 |
| 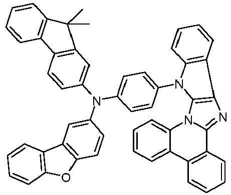 | 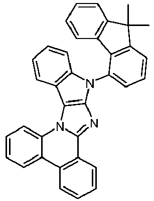 |
| EG29 | EG30 |
| 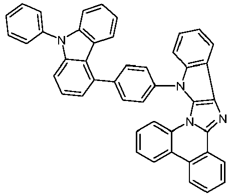 | 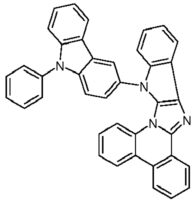 |
| EG31 | EG32 |
| 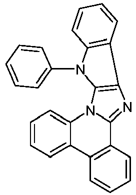 | |
| EG33 | |

## Patentansprüche

1. Verbindung gemäß Formel (1), 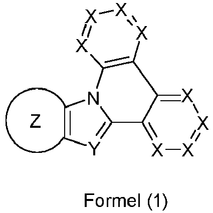 wobei für die verwendeten Symbole gilt:
Z ist eine Gruppe der Formel (2), 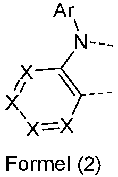 wobei die gestrichelten Bindungen die Verknüpfung dieser Gruppe mit den beiden explizit einzeichneten Kohlenstoffatomen in Formel (1) kennzeichnen;
X ist bei jedem Auftreten gleich oder verschieden CR oder N;
Y ist CR oder N;
Ar ist ein aromatisches oder heteroaromatisches Ringsystem mit 5 bis 40 aromatischen Ringatomen, welches mit einem oder mehreren Resten R substituiert sein kann;
R ist bei jedem Auftreten gleich oder verschieden H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann;
Ar' ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

2. Verbindung nach Anspruch 1, **dadurch gekennzeichnet, dass** Z eine Gruppe der Formel (2a) ist, 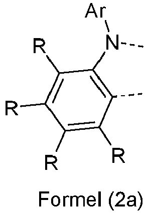 wobei die Symbole die in Anspruch 1 genannten Bedeutungen aufweisen.

3. Verbindung nach Anspruch 1 oder 2, **dadurch gekennzeichnet, dass** maximal ein Symbol X pro Cyclus für N steht und die anderen Symbole X für CR stehen.

4. Verbindung nach einem oder mehreren der Ansprüche 1 bis 3 gemäß Formel (3a-1), (4a-1), (3a-2) oder (4a-2), 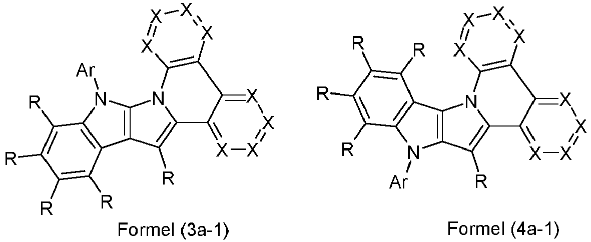 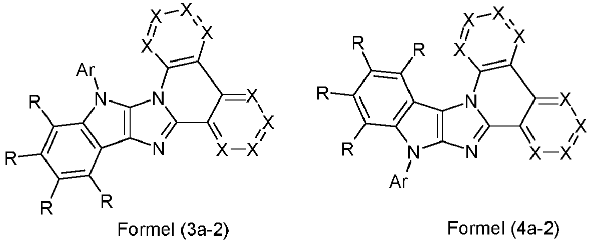 wobei die Symbole die in Anspruch 1 genannten Bedeutungen aufweisen und maximal ein Symbol X für N steht.

5. Verbindung nach einem oder mehreren der Ansprüche 1 bis 4 gemäß Formel (3b-1), (4b-1), (3b-2) oder (4b-2), 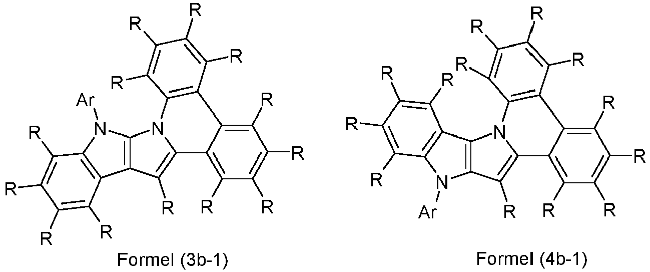 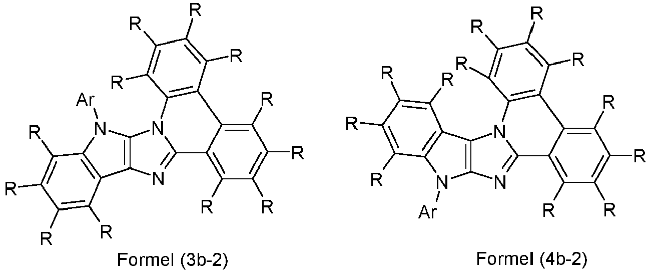 wobei die Symbole die in Anspruch 1 genannten Bedeutungen aufweisen.

6. Verbindung nach einem oder mehreren der Ansprüche 1 bis 5, **dadurch gekennzeichnet, dass** maximal zwei Reste R für eine Gruppe ungleich Wasserstoff stehen.

7. Verbindung nach einem oder mehreren der Ansprüche 1 bis 6, ausgewählt aus den Verbindungen der Formeln (3c-1), (4c-1), (3c-2) und (4c-2), 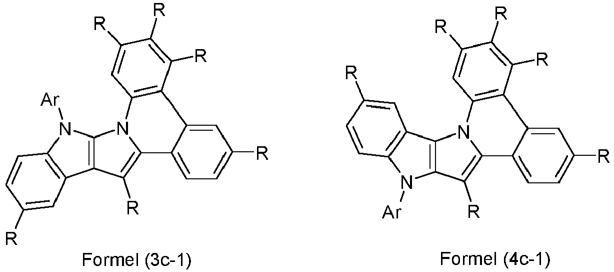 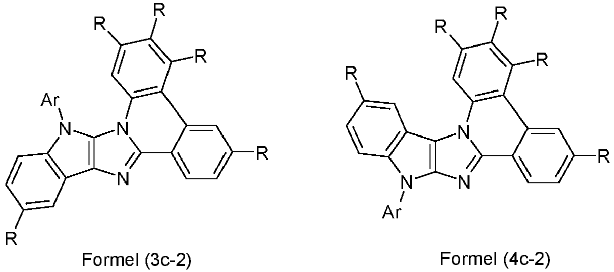 wobei die Symbole die in Anspruch 1 genannten Bedeutungen aufweisen und maximal zwei Gruppen R ungleich Wasserstoff sind.

8. Verbindung nach einem oder mehreren der Ansprüche 1 bis 7, **dadurch gekennzeichnet, dass** Ar ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R substituiert sein können.

9. Verbindung nach einem oder mehreren der Ansprüche 1 bis 8, **dadurch gekennzeichnet, dass** R bei jedem Auftreten gleich oder verschieden ausgewählt ist aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann.

10. Formulierung, enthaltend mindestens eine Verbindung nach einem oder mehreren der Ansprüche 1 bis 9 und mindestens eine weitere Verbindung, insbesondere mindestens ein Lösemittel.

11. Verwendung einer Verbindung nach einem oder mehreren der Ansprüche 1 bis 9 in einer elektronischen Vorrichtung, insbesondere in einer organischen Elektrolumineszenzvorrichtung.

12. Elektronische Vorrichtung enthaltend mindestens eine Verbindung nach einem oder mehreren der Ansprüche 1 bis 9.

13. Elektronische Vorrichtung nach Anspruch 12, wobei es sich um eine organische Elektrolumineszenzvorrichtung handelt, **dadurch gekennzeichnet, dass** die Verbindung nach einem oder mehreren der Ansprüche 1 bis 9 in einer emittierenden Schicht, insbesondere als Matrixmaterial, und/oder in einer Elektronentransportschicht und/oder in einer Lochblockierschicht und/oder in einer Lochtransportschicht und/oder in einer Exzitonenblockierschicht eingesetzt wird.

## Claims

1. Compound of the formula (1), 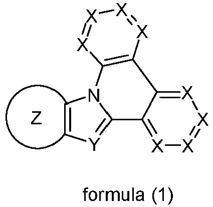 where the following applies to the symbols used:
Z is a group of the formula (2), 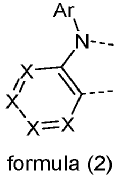 where the dashed bonds denote the linking of this group to the two carbon atoms explicitly drawn in in formula (1);
X is on each occurrence, identically or differently, CR or N;
Y is CR or N;
Ar is an aromatic or heteroaromatic ring system having 5 to 40 aromatic ring atoms, which may be substituted by one or more radicals R;
R is on each occurrence, identically or differently, H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by Si(R¹)₂, C=O, NR¹, O, S or CONR¹, or an aromatic or heteroaromatic ring system having 5 to 60 aromatic ring atoms, preferably having 5 to 40 aromatic ring atoms, which may in each case be substituted by one or more radicals R¹;
Ar' is on each occurrence, identically or differently, an aromatic or heteroaromatic ring system having 5 to 40 aromatic ring atoms, which may be substituted by one or more radicals R¹;
R¹ is on each occurrence, identically or differently, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R², where one or more non-adjacent CH₂ groups may be replaced by Si(R²)₂, C=O, NR², O, S or CONR², or an aromatic or heteroaromatic ring system having 5 to 40 aromatic ring atoms, which may in each case be substituted by one or more radicals R²; two or more radicals R¹ may form a ring system with one another;
R² is on each occurrence, identically or differently, H, D, F or an aliphatic, aromatic or heteroaromatic organic radical, in particular a hydrocarbon radical, having 1 to 20 C atoms, in which, in addition, one or more H atoms may be replaced by F.

2. Compound according to Claim 1, **characterised in that** Z is a group of the formula (2a), 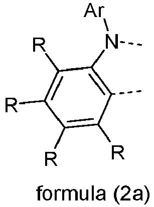 where the symbols have the meanings given in Claim 1.

3. Compound according to Claim 1 or 2, **characterised in that** a maximum of one symbol X per ring stands for N and the other symbols X stand for CR.

4. Compound according to one or more of Claims 1 to 3 of the formula (3a-1), (4a-1), (3a-2) or (4a-2), 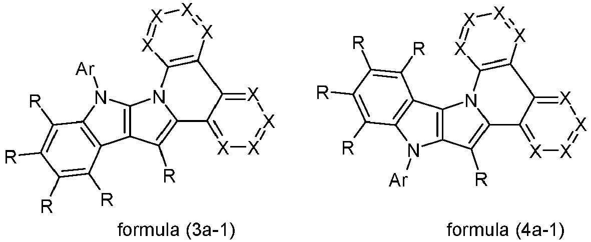 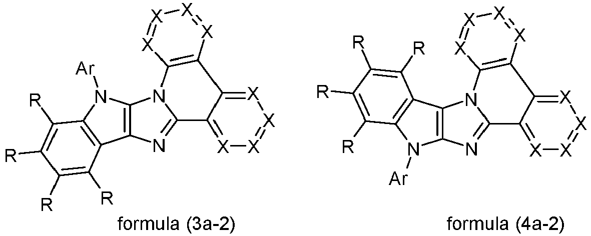 where the symbols have the meanings given in Claim 1 and a maximum of one symbol X stands for N.

5. Compound according to one or more of Claims 1 to 4 of the formula (3b-1), (4b-1), (3b-2) or (4b-2), 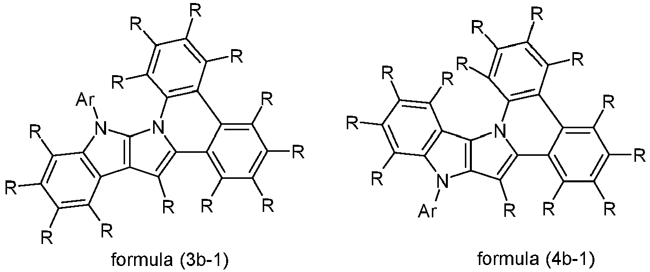 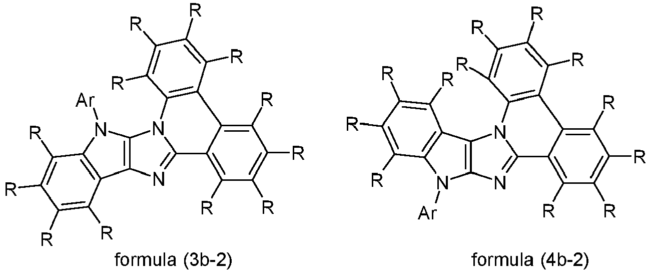 where the symbols have the meanings given in Claim 1.

6. Compound according to one or more of Claims 1 to 5, **characterised in that** a maximum of two radicals R stand for a group other than hydrogen.

7. Compound according to one or more of Claims 1 to 6, selected from the compounds of the formulae (3c-1), (4c-1), (3c-2) and (4c-2), 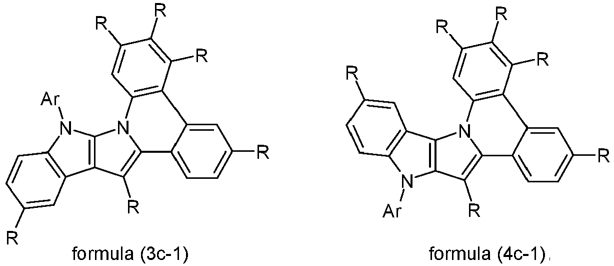 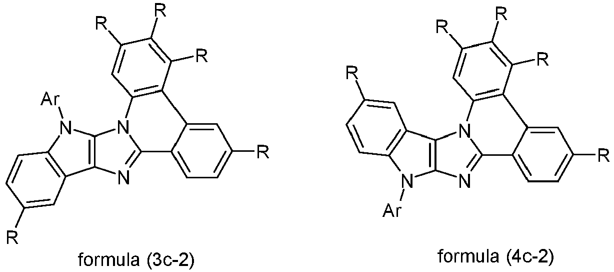 where the symbols have the meanings given in Claim 1 and a maximum of two groups R are not equal to hydrogen.

8. Compound according to one or more of Claims 1 to 7, **characterised in that** Ar is selected from phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, quinoline, quinazoline, benzimidazole, phenanthrene, triphenylene or a combination of two or three of these groups, which may in each case be substituted by one or more radicals R.

9. Compound according to one or more of Claims 1 to 8, **characterised in that** R is selected on each occurrence, identically or differently, from the group consisting of H, D, F, N(Ar')₂, CN, OR¹, a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R¹, but is preferably unsubstituted, and where one or more non-adjacent CH₂ groups may be replaced by O, or an aromatic or heteroaromatic ring system having 6 to 30 aromatic ring atoms, which may in each case be substituted by one or more radicals R¹.

10. Formulation comprising at least one compound according to one or more of Claims 1 to 9 and at least one further compound, in particular at least one solvent.

11. Use of a compound according to one or more of Claims 1 to 9 in an electronic device, in particular in an organic electroluminescent device.

12. Electronic device containing at least one compound according to one or more of Claims 1 to 9.

13. Electronic device according to Claim 12, which is an organic electroluminescent device, **characterised in that** the compound according to one or more of Claims 1 to 9 is employed in an emitting layer, in particular as matrix material, and/or in an electron-transport layer and/or in a hole-blocking layer and/or in a hole-transport layer and/or in an exciton-blocking layer.

## Revendications

1. Composé de formule (1), 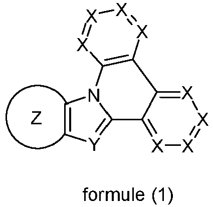 dans lequel ce qui suit s'applique aux symboles utilisés :
Z est un groupement de formule (2), 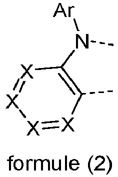 où les lignes en pointillés désignent la liaison de ce groupement aux deux atomes de carbone explicitement dessinés dans la formule (1) ;
X est à chaque occurrence, de manière identique ou différente, CR ou N;
Y est CR ou N ;
Ar est un noyau aromatique ou hétéroaromatique ayant de 5 à 40 atomes de cycle aromatique, pouvant être substitué par un ou plusieurs radicaux R ;
R est à chaque occurrence, de manière identique ou différente, H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut être dans chaque cas substitué par un ou plusieurs radicaux R¹, où un ou plusieurs groupements CH₂ non adjacents peuvent être remplacés par Si(R¹)₂, C=O, NR¹, O, S ou CONR¹, ou un noyau aromatique ou hétéroaromatique ayant de 5 à 60 atomes de cycle aromatique, préférablement ayant de 5 à 40 atomes de cycle aromatique, pouvant être dans chaque cas substitué par un ou plusieurs radicaux R¹ ;
Ar' est à chaque occurrence, de manière identique ou différente, un noyau aromatique ou hétéroaromatique ayant de 5 à 40 atomes de cycle aromatique, pouvant être substitué par un ou plusieurs radicaux R¹ ;
R¹ est à chaque occurrence, de manière identique ou différente, H, D, F, Cl, Br, I, N(R²)2, CN, NO₂, OR², SR², Si(R²)₃, B(OR²)₂, C(=O)R², P(=O)(R²)₂, S(=O)R², S(=O)₂R², OSO₂R², un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut être dans chaque cas substitué par un ou plusieurs radicaux R², où un ou plusieurs groupements CH₂ non adjacents peuvent être remplacés par Si(R²)₂, C=O, NR², O, S ou CONR², ou un noyau aromatique ou hétéroaromatique ayant de 5 à 40 atomes de cycle aromatique, pouvant être dans chaque cas substitué par un ou plusieurs radicaux R² ; deux, ou plus, radicaux R¹ peuvent former un noyau l'un avec l'autre ;
R² est à chaque occurrence, de manière identique ou différente, H, D, F ou un radical organique aliphatique, aromatique ou hétéroaromatique, en particulier un radical hydrocarboné, ayant de 1 à 20 atomes de C, dans lequel, de plus, un ou plusieurs atomes de H peuvent être remplacés par F.

2. Composé selon la revendication 1, **caractérisé en ce que** Z est un groupement de formule (2a), 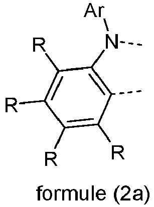 où les symboles revêtent les significations données selon la revendication 1.

3. Composé selon la revendication 1 ou 2, **caractérisé en ce qu'**un maximum d'un symbole X par cycle représente N et les autres symboles X représentent CR.

4. Composé selon l'une ou plusieurs parmi les revendications 1 à 3, de formule (3a-1), (4a-1), (3a-2) ou (4a-2), 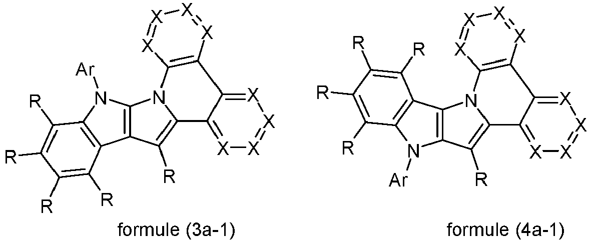 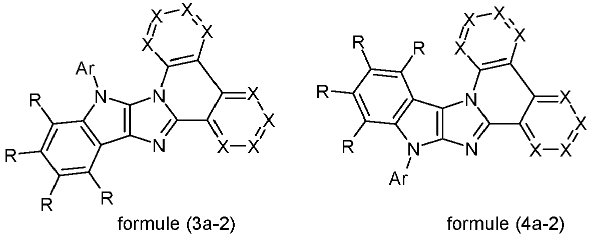 où les symboles revêtent les significations données selon la revendication 1 et un maximum d'un symbole X représente N.

5. Composé selon l'une ou plusieurs parmi les revendications 1 à 4, de formule (3b-1), (4b-1), (3b-2) ou (4b-2), 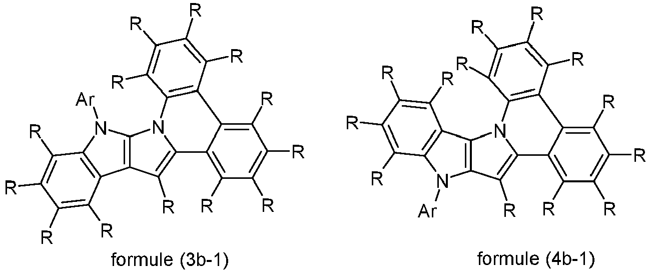 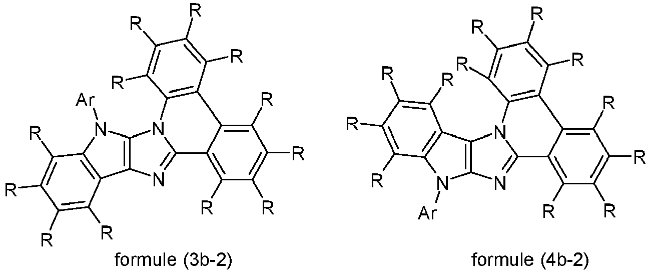 où les symboles revêtent les significations données selon la revendication 1.

6. Composé selon l'une ou plusieurs parmi les revendications 1 à 5, **caractérisé en ce qu'**un maximum de deux radicaux R représentent un groupement autre qu'hydrogène.

7. Composé selon l'une ou plusieurs parmi les revendications 1 à 6, choisi parmi les composés de formules (3c-1), (4c-1), (3c-2) et (4c-2), 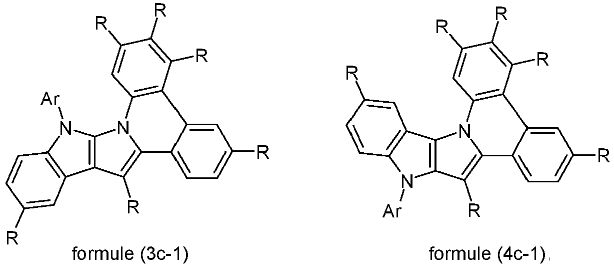 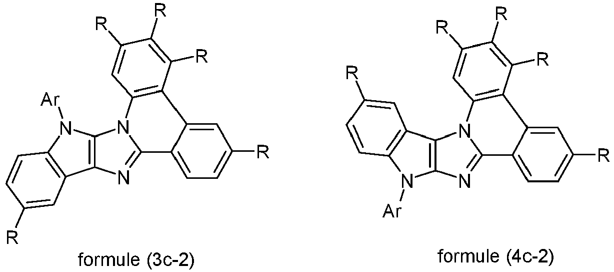 où les symboles revêtent les significations données selon la revendication 1 et un maximum de deux groupements R ne sont pas équivalents à hydrogène.

8. Composé selon l'une ou plusieurs parmi les revendications 1 à 7, **caractérisé en ce que** Ar est choisi parmi phényle, biphényle, terphényle, quaterphényle, fluorène, spirobifluorène, naphtalène, indole, benzofurane, benzothiophène, carbazole, dibenzofurane, dibenzothiophène, indénocarbazole, indolocarbazole, pyridine, pyrimidine, pyrazine, pyridazine, triazine, quinoléine, quinazoline, benzimidazole, phénanthrène, triphénylène ou une combinaison de deux ou trois parmi ces groupements, qui peuvent être dans chaque cas substitués par un ou plusieurs radicaux R.

9. Composé selon l'une ou plusieurs parmi les revendications 1 à 8, **caractérisé en ce que** R est choisi à chaque occurrence, de manière identique ou différente, dans le groupe constitué par H, D, F, N(Ar')₂, CN, OR¹, un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut être dans chaque cas substitué par un ou plusieurs radicaux R¹, mais est préférablement non substitué, et où un ou plusieurs groupements CH₂ non adjacents peuvent être remplacés par O, ou un noyau aromatique ou hétéroaromatique ayant de 6 à 30 atomes de cycle aromatique, pouvant être dans chaque cas substitué par un ou plusieurs radicaux R¹.

10. Formulation comprenant au moins un composé selon l'une ou plusieurs parmi les revendications 1 à 9 et au moins un autre composé, en particulier au moins un solvant.

11. Utilisation d'un composé selon l'une ou plusieurs parmi les revendications 1 à 9 dans un dispositif électronique, en particulier dans un dispositif électroluminescent organique.

12. Dispositif électronique contenant au moins un composé selon l'une ou plusieurs parmi les revendications 1 à 9.

13. Dispositif électronique selon la revendication 12, qui est un dispositif électroluminescent organique, **caractérisé en ce que** le composé selon l'une ou plusieurs parmi les revendications 1 à 9 est employé dans une couche émettrice, en particulier comme matériau de matrice, et/ou dans une couche de transport d'électrons et/ou dans une couche de blocage de trous et/ou dans une couche de transport de trous et/ou dans une couche de blocage d'excitons.
